# EUROPEAN PATENT SPECIFICATION

(11) **EP 2 495 229 B1**
(45) Date of publication and mention of the grant of the patent: **01.06.2016**
(21) Application number: 10826830.1
(22) Date of filing: 28.10.2010
(51) Int. Cl.: C07C 13/605, C08G 61/12, C08G 73/02, C09K 11/06, H01L 51/42, H01L 51/50, C07C 211/45, C07C 13/44

(54) **LOW-MOLECULAR COMPOUND, POLYMER, MATERIAL FOR ELECTRONIC DEVICES, COMPOSITION FOR ELECTRONIC DEVICES, ORGANIC ELECTROLUMINESCENT ELEMENT, ORGANIC SOLAR CELL ELEMENT, DISPLAY AND LIGHTING EQUIPMENT**
VERBINDUNG VON NIEDRIGEM MOLEKULARGEWICHT, POLYMER, MATERIAL FÜR ELEKTRONISCHE VORRICHTUNGEN, ZUSAMMENSETZUNG FÜR ELEKTRONISCHE VORRICHTUNGEN, ORGANISCHES ELEKTROLUMINESZENZELEMENT, ORGANISCHES SOLARZELLENELEMENT, ANZEIGE UND BELEUCHTUNGSVORRICHTUNG
COMPOSÉ DE FAIBLE POIDS MOLÉCULAIRE, POLYMÈRE, MATÉRIAU POUR DISPOSITIFS ÉLECTRONIQUES, COMPOSITION POUR DISPOSITIFS ÉLECTRONIQUES, ÉLÉMENT ORGANIQUE ÉLECTROLUMINESCENT, ÉLÉMENT DE CELLULE SOLAIRE ORGANIQUE, ÉQUIPEMENT D'AFFICHAGE ET D'ÉCLAIRAGE

(30) Priority: 30.10.2009 JP 2009251096
(43) Date of publication of application: 05.09.2012
(73) Proprietor: Mitsubishi Chemical Corporation, Chiyoda-ku Tokyo 100-8251 (JP)
(72) Inventor: URANO Toshiyuki, Kanagawa 227-8502 (JP); NAKAI Toshimitsu, Kanagawa 227-8502 (JP); IIDA Koichiro, Kanagawa 227-8502 (JP); LI Yanjun, Kanagawa 227-8502 (JP); BABA Tatsushi, Kanagawa 227-8502 (JP)
(74) Representative: Vossius & Partner Patentanwälte Rechtsanwälte mbB
(86) International application number: PCT/JP2010/069220
(87) International publication number: WO 2011/052698

(56) References cited:
- EP-A1- 1 847 525
- WO-A1-2004/014985
- WO-A1-2009/102027
- WO-A1-2009/102027
- GB-A- 2 415 960
- JP-A- 2004 362 930
- ALBERT PADWA ET AL.: 'Synthesis and Ring-Opening Reactions in the 1,3'-Bicyclopropenyl Series' THE JOURNAL OF ORGANIC CHEMISTRY vol. 49, no. 5, 1984, pages 856 - 862
- RYO SHINTANI ET AL.: 'Rhodium-Catalyzed Asymmetric Arylation of N-Tosyl Ketimines' JOURNAL OF THE AMERICAN CHEMICAL SOCIETY vol. 132, 01 September 2010, pages 13168 - 13169
- YUNFEI LUO ET AL.: 'Chemoenzymatic Synthesis and Application of Bicyclo[2.2.2]octadiene Ligands: Increased Efficiency in Rhodium-Catalyzed Asymmetric Conjugate Additions by Electronic Tuning' ANGEWANDTE CHEMIE INTERNATIONAL EDITION vol. 49, no. 15, 12 March 2010, pages 2750 - 2754

## Description

### Technical Field

The present invention relates to a low-molecular compound, a polymer, electronic-device material, a composition for electronic devices, an organic electroluminescent element, an organic solar cell element, a display, and a lighting.

### Background Art

Electroluminescent elements employing an organic thin film (organic electroluminescent elements) are being developed in recent years. Examples of methods for forming an organic thin film for use in an organic electroluminescent element include a vacuum deposition method and a wet film formation method. The vacuum deposition method has an advantage that since superposition of layers by this method is easy, it is easy to improve charge injection from the anode and/or cathode or to confine excitons in the photosensitive layer. On the other hand, the wet film formation method has advantages, for example, that no vacuum process is necessary and film formation in a larger area is easy, and that it is easy to incorporate a plurality of materials having various functions into one layer (coating fluid).

However, superposition of layers by the wet film formation method is difficult, and elements obtained by the method have lower driving stability than elements produced by the vacuum deposition method and have not reached a practical level at present except some of these. When organic thin films are superposed by a wet film formation method, it is required to impart solubility in organic solvents to the material for organic electroluminescent elements by, for example, introducing a long-chain alkyl group thereinto. Furthermore, in the case where after the formation of an organic thin film, another organic thin film (upper layer) is formed thereon by a wet film formation method, the material for organic electroluminescent elements which is contained in the lower layer is required to be insoluble in the coating solution for forming the upper layer. In a method which has hitherto been employed for such superposition of layers, a crosslinkable group or the like is introduced into the material for organic electroluminescent elements which is to be incorporated into the lower layer and, after formation of the lower layer, the crosslinkable group is crosslinked by a heat treatment, irradiation with energy, etc. to render the material insoluble in the coating solution for upper-layer formation. For example, non-patent document 1 and non-patent document 2 have proposed the following compounds having crosslinkable groups, and have proposed a method for superposing organic thin films in which an organic thin film is rendered insoluble in organic solvents by reacting the crosslinkable groups.

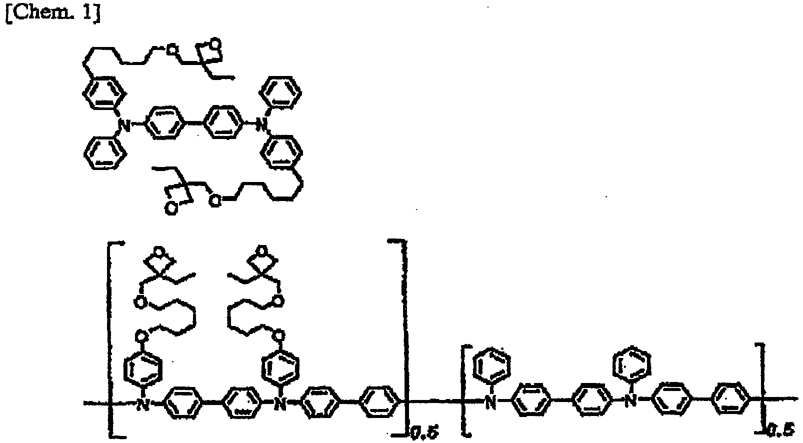

Furthermore, non-patent document 3, for example, has proposed the following compound, which has a elimination group. A method in which the elimination group is reacted to thereby render the compound insoluble in organic solvents has been proposed therein as another technique.
Patent document 1 discloses a conjugated polymer consisting of a specific repeating unit, wherein the conjugated polymer contains an insolubilizing group as a substituent, and has a weight average molecular weight (Mw) of 20,000 or more and a dispersity (Mw/Mn, wherein Mn indicates a number average molecular weight) of 2.40 or less. >

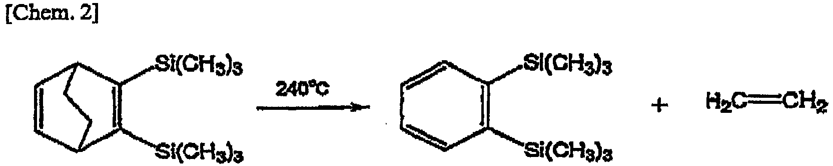

### Prior-Art Documents

### Non-Patent Documents

Non-Patent Document 1: Applied Physics Letters, Vol.86, p.221102, 2005
Non-Patent Document 2: Macromolecules, Vol.39, p.8911, 2006
Non-Patent Document 3: Collect. Czech. Chem. Commun., Vol.54, p.3090, 1989

### Patent Document

Patent Document 1: WO 2009/102027

### Summary of the Invention

### Problems that the Invention is to Solve

However, in the method described in non-patent documents 1 and 2, long-chain alkyl groups and unreacted crosslinkable groups remain in the organic thin film. There is a problem that such long-chain alkyl groups and unreacted crosslinkable groups are causative of a decrease in charge transport efficiency, a decrease in the luminescent efficiency of the organic electroluminescent element, a decrease in the driving stability thereof, etc.

The elimination group proposed in non-patent document 3 undergoes an elimination reaction at a high temperature and, hence, there is a possibility that the characteristics of the element to be obtained might be reduced by the heat treatment conducted after film formation.

Furthermore, there also has been the following problem. In the case where a material for organic electroluminescent elements which is a low-molecular compound is used, the molecules of the material for organic electroluminescent elements become readily movable as a result of a thermal drying treatment conducted after film formation or by the influence of high temperatures which result during driving of the luminescent element, because the material has a low glass transition temperature. Consequently, the molecular orientation in the organic layer is disordered, and this is apt to result in a decrease in luminescent efficiency or charge transport efficiency.

Meanwhile, plastic substrates for use in flexible displays or the like have low heat resistance, and there is a possibility that high-temperature heating after film formation might affect the working life of the elements.

It has hence been desired to provide a compound which can be formed, by a wet process, into a film that is capable of being insolubilized by a heat treatment at a relatively low temperature and of being coated with other layers by a wet film formation method, and which is usable as a material for electronic devices that decrease little in charge transport efficiency or luminescent efficiency and have excellent driving stability.

### Means for Solving the Problems

As a result of investigations diligently made by the present inventors, it was found that a compound having a specific structure is suitable for wet film formation because of the solubility thereof in organic solvents during wet film formation and can be insolubilized by heating at a low temperature after the wet film formation. The invention has been thus achieved.

Essential points of the invention reside in the following items 1 to 12.
1. A compound which is represented by the following general formula (V) and has a molecular weight of 400 or higher: 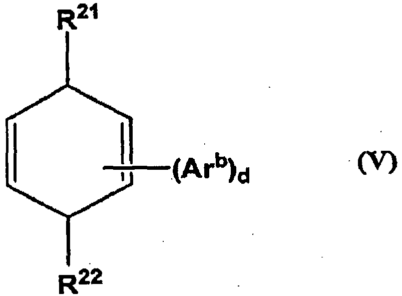 wherein
   R²¹ and R²² are bonded to each other to represent a group represented by -CH=CH- or -CH₂-CH₂-;
   Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
   d represents an integer of 1 to 4;
   when a plurality of Ar^{b}s are contained in the molecule, the Ar^{b}s may be the same or different; and
   with the proviso that the compound which is represented by the following general formula (V) is not 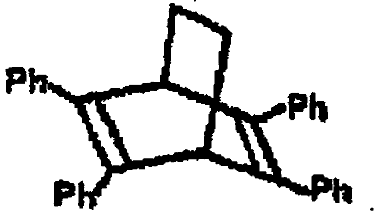
2. The compound according to item 1, wherein d in the general formula (V) is an integer of 1 to 3.
3. An electronic-device material which comprises a compound which is represented by the following general formula (V) and has a molecular weight of 400 or higher: 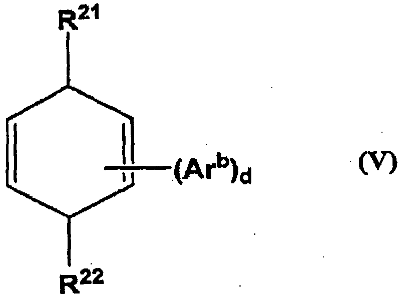 wherein
   R²¹ and R²² are bonded to each other to represent a group represented by -CH=CH- or -CH₂-CH₂-;
   Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
   d represents an integer of 1 to 4 or 1 to 3;
   when a plurality of Ar^{b}s are contained in the molecule, the Ar^{b}s may be the same or different.
4. A polymer which comprises a monovalent or divalent group represented by the following general formula (III): 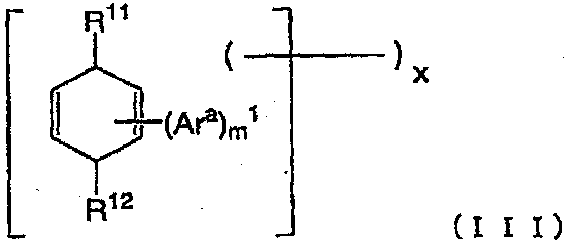 wherein
   R¹¹ and R¹² are bonded to each other to represent a group represented by -CH=CH- or -CH₂-CH₂-;
   Ar^{a} represents an aromatic hydrocarbon ring group which may have a substituent, wherein the substituent of Ar^{a} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
   m¹ represents an integer of 1 to 4;
   x represents 1 or 2;
   when a plurality of Ar^{a}s are contained in the molecule, the Ar^{a}s may be the same or different.
5. The polymer according to item 4, which comprises a repeating unit represented by the following formula (IV): 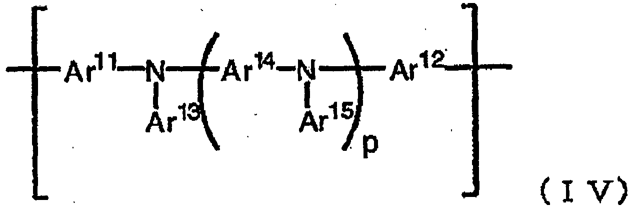 wherein
   p represents an integer of 0 to 3,
   Ar¹¹ and Ar¹² each independently represent a direct bond or an aromatic ring group which may have a substituent, and
   Ar¹³ to Ar¹⁵ each independently represent an aromatic ring group which may have a substituent,
   both of Ar¹¹ and Ar¹² being not a direct bond.
6. An electronic-device material which comprises the polymer according to item 4 or 5.
7. A composition for electronic device which comprises the electronic-device material according to item 3 or 6 and a solvent.
8. An organic electroluminescent element which comprises an anode, a cathode, and an organic layer interposed between the anode and the cathode, wherein the organic layer comprises a compound which is represented by the following general formula (V) and has a molecular weight of 400 or higher: 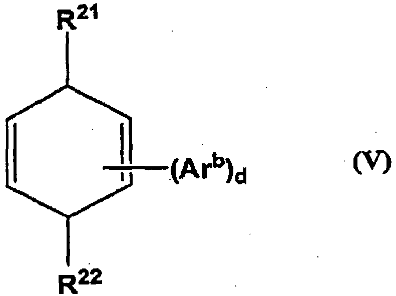 wherein
   R²¹ and R²² are bonded to each other to represent a group represented by -CH=CH- or -CH₂-CH₂-;
   Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
   d represents an integer of 1 to 4 or 1 to 3;
   when a plurality of Ar^{b}s are contained in the molecule, the Ar^{b}s may be the same or different;, or the polymer according to item 4 or 5 and wherein the organic layer is a layer formed by a wet film formation method using the composition for electronic devices according to item 7.
9. The organic electroluminescent element according to item 8, wherein the organic layer is a luminescent layer.
10. An organic solar cell element which comprises an anode, a cathode, and an organic layer interposed between the anode and the cathode, wherein the organic layer comprises a compound
   which is represented by the following general formula (V) and has a molecular weight of 400 or higher: 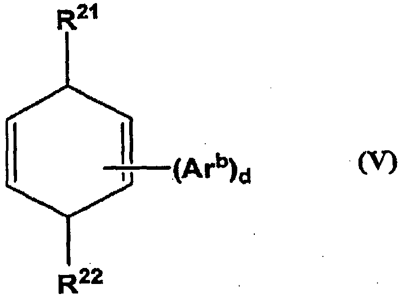 wherein
   R²¹ and R²² are bonded to each other to represent a group represented by -CH=CH- or -CH₂-CH₂-;
   Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
   d represents an integer of 1 to 4 or 1 to 3;
   when a plurality of Ar^{b}s are contained in the molecule, the Ar^{b}s may be the same or different;, or the polymer according to item 4 or 5 and wherein the organic layer is a layer formed by a wet film formation method, using the composition for electronic devices according to item 7.
11. A display which comprises the organic electroluminescent element according to item 8 or 9.
12. A lighting which comprises the organic electroluminescent element according to item 8 or 9.

### Effects of the Invention

Since the organic compounds of the invention have a structure having a elimination group, the compounds are highly soluble in the organic solvent during wet film formation. Furthermore, since the elimination group is eliminated by heating after the film formation, the solubility of the film in organic solvents can be considerably reduced.

It is, therefore, possible to superpose another layer by, for example, a wet film formation method on the organic layer formed from either of the organic compounds of the invention. For example, in the case of a material for layer formation for organic electroluminescent elements which exhibits satisfactory performance when formed into a film by a vapor deposition method but has too low solubility to be used in a wet film formation method, this material can be rendered applicable to wet film formation by incorporating the material into a compound represented by general formula (V).

It is less necessary for the organic compounds of the invention to contain in the molecule a long-chain alkyl group for improving solubility in organic solvents or a crosslinkable group for reducing the solubility of the film in other solvents after film formation.

Consequently, the organic electroluminescent element formed using either of the organic compounds of the invention has a high luminescent efficiency, low operating voltage, and long working life. Namely, the organic electroluminescent element formed using the organic compound of the invention has a low operating voltage and hence produces the effect of energy saving.

Furthermore, since the organic compounds of the invention have a low elimination temperature for the elimination group, a low temperature suffices for the heat treatment to be conducted after film formation. As a result, the heating after film formation exerts little influence on the substrate and the other layers. Consequently, the element obtained has a long working life, and the organic compounds of the invention can be used in a wide range of applications including flexible displays employing lowly heat-resistance plastic substrates or the like.

The organic compounds of the invention, the electronic-device materials including the organic compounds, and the composition for electronic devices which contains either of the electronic-device materials have excellent film-forming properties, charge-transporting properties, luminescent properties, and heat resistance, and are hence applicable to the formation of an organic layer such as a hole injection layer, hole transport layer, luminescent layer, electron injection layer, or electron transport layer according to the layer configuration of the element. It is more preferred that the organic compounds, the electronic-device materials, and the composition should be used for a luminescent layer.

The organic electroluminescent element obtained using any of the organic compounds of the invention, the electronic-device materials including the organic compounds, and the composition for electronic devices which contains either of the electronic-device materials can be used in displays and lightings. Specifically, the organic electroluminescent element is thought to be applied to flat panel displays (e.g., displays for OA computers and wall-mounted TV receivers), vehicle-mounted display elements, cell phone displays, light sources taking advantage of the feature of a surface light emitter (e.g., the light source of a copier and the backlights of a liquid-crystal display and instrument), display panels, and marker lights, and has a high technical value.

### Brief Description of the Drawings

[Fig. 1] Fig. 1 is a diagrammatic sectional view illustrating one embodiment of the organic electroluminescent element of the invention.
[Fig. 2] Fig. 2 is a diagrammatic sectional view illustrating one embodiment of the organic solar cell element of the invention.

### Modes for Carrying Out the Invention

The matter, methods, and the like shown below as examples are embodiments (representative embodiments) of the invention, and the invention should not be construed as being limited to the embodiments unless the invention departs from the spirit thereof.

The group represented by -CH=CH- or -CH₂-CH₂- formed by the R²¹ and R²² in general formula (V) which have been bonded to each other, or by the R¹ and R² in general formula (II) given later which have been bonded to each other, or by the R¹¹ and R¹² in general formula (III) which have been bonded to each other is often referred to as "elimination group" in the invention.

The term "organic compounds of the invention" is a general term for the "compound of the invention" represented by general formula (V) and the "polymer of the invention" represented by general formula (III). In the invention, the term "polymer" means a compound which includes a plurality of divalent repeating units in the structure and has a molecular weight distribution.

Furthermore, "aromatic ring" is a general term for an "aromatic hydrocarbon ring" and an "aromatic heterocycle".

Moreover, "% by mass", "mass ppm", and "parts by mass" have the same meanings as "% by weight", "weight ppm", and "parts by weight", respectively. The mere expression "ppm" means "weight ppm".

### <Explanation of the Elimination group>

One example of reactions of the elimination group (elimination reactions) in the organic compounds of the invention is shown below.

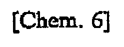

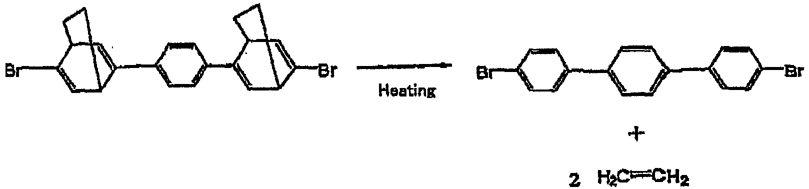

In the case of the reaction scheme shown above, the elimination groups each are the portion of the following structure which is surrounded by the circle.

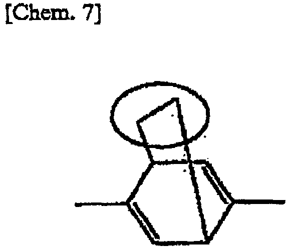

Before a heat treatment, such elimination groups serve to prevent stacking of the molecules and thereby improve the solubility of the organic compound in organic solvents. The elimination groups are eliminated from the organic compound by a heat treatment and, as a result, the compound which has undergone the elimination can have considerably reduced solubility in organic solvents. In the invention, the treatment for considerably reducing the solubility of the organic compounds of the invention by an elimination reaction of the elimination group is referred to as "insolubilization".

As a result, it becomes easy to superpose an organic thin film by a wet film formation method on the organic layer formed from the organic compounds of the invention.

Furthermore, it is less necessary that a group such as a long-chain alkyl group for enhancing solubility in organic solvents or a crosslinkable group for reducing the solubility of the organic layer in organic solvents should be introduced into the molecule as in the compounds which have been used in conventional multilayer coating operations. Consequently, unlike the compounds which have been used hitherto, the organic compounds of the invention give a film in which a long-chain alkyl group or unreacted crosslinkable group, which is apt to affect the operating voltage and working life of the organic electroluminescent element, does not remain. Namely, the organic electroluminescent element having an organic layer formed from the organic compounds of the invention is presumed to have a low operating voltage and a long working life.

Another feature is that the cyclohexadiene ring to which the elimination group has been bonded is not part of a fused ring and, hence, the elimination temperature is relatively low.

### <Compound Represented by General Formula (V) (hereinafter often referred to as "compound of the invention")>

The compound of the invention is characterized by being represented by the following general formula (V) and having a molecular weight of 400 or higher. 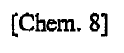 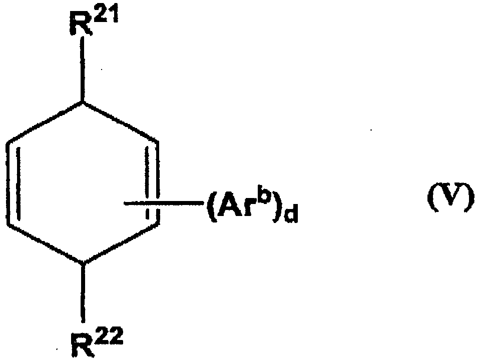
(In general formula (V), R²¹ and R²² have been bonded to each other to represent the group represented by -CH=CH- or -CH₂-CH₂-; Ar^{b} represents an aromatic hydrocarbon ring group which may have a substituent as defined in claim 1, and d represents an integer of 1 to 4.
When a plurality of Ar^{b}s are contained in the molecule, the Ar^{b}s may be the same or different.
In disclosed compounds the cyclohexadiene ring in general formula (V) may have one or more substituents besides R²¹, R²², and Ar^{b}, and none of the substituents forms a cyclic structure fused to the cyclohexadiene ring.)

In general formula (V), R²¹ and R²² have been bonded to each other to constitute the group represented by -CH=CH- or -CH₂-CH₂.

Since R²¹ and R²² have been bonded to each other to constitute the group represented by -CH=CH- or -CH₂-CH₂-, the compound of the invention has excellent electrical stability. Furthermore, since the elimination group portion has a low molecular weight, the component which has been eliminated is less apt to remain in the residual layer. Especially when the compound is used for forming a luminescent layer, not only the luminescent material can be prevented from deteriorating but also charges within the luminescent layer can be moved more efficiently.

Ar^{b} in general formula (V) is an aromatic hydrocarbon ring group which may have a substituent.

Examples of the aromatic hydrocarbon ring group include mono- or divalent groups derived from 5- or 6-membered monocycles or di- to pentacyclic fused rings, such as a benzocyclohexadiene ring, naphthalene ring, anthracene ring, phenanthrene ring, perylene ring, tetracene ring, pyrene ring, benzpyrene ring, chrysene ring, triphenylene ring, fluorene ring, and fluoranthene ring. Preferred from the standpoint of electrical stability are a benzocyclohexadiene ring, naphthalene ring, anthracene ring, perylene ring, tetracene ring, pyrene ring, chrysene ring, triphenylene ring, and fluorene ring.

Since Ar^{b} in general formula (V) is an aromatic hydrocarbon ring group which may have a substituent, the compound of the invention has a strong tendency to form a covalent bond. The temperature at which the elimination group portion is eliminated from the cyclohexadiene ring is not too low, and a relatively stable structure can be maintained. On the other hand, the compound formed by the elimination of the elimination group from that compound has low polarity because Ar^{b} is an aromatic hydrocarbon ring group which may have a substituent, and the layer containing this compound has far lower solubility in organic solvents than the original compound which has not undergone the elimination. Thus, the effect of the invention described above can be produced.

The substituent of Ar^{b} is as defined in claim 1.

Examples of the substituent which may be possessed by the aromatic hydrocarbon ring group represented by Ar^{b} include some of the substituents Z which will be described later. More preferred examples thereof include the following functional groups.
Disclosed examples thereof include
linear or branched alkyl groups which may have a substituent (preferably, linear or branched alkyl groups having 1-8 carbon atoms, examples of which include methyl, ethyl, n-propyl, 2-propyl, n-butyl, isobutyl, and tert-butyl);
aralkyl groups (preferably, aralkyl groups having 7-15 carbon atoms, examples of which include benzyl);
alkoxy groups (preferably, alkoxy groups having 1-8 carbon atoms, examples of which include methoxy, ethoxy, and butoxy);
aryloxy groups (preferably, aryloxy groups having an aromatic hydrocarbon ring group with 6-12 carbon atoms, examples of which include phenyloxy, 1-naphthyloxy, and 2-naphthyloxy);
acyl groups (preferably, acyl groups having 2-10 carbon atoms, examples of which include formyl, acetyl, and benzoyl);
alkenyl groups (preferably, alkenyl groups having 2-9 carbon atoms, examples of which include vinyl, allyl, and 1-butenyl);
alkynyl groups (preferably, alkynyl groups having 2-9 carbon atoms, examples of which include ethynyl and propargyl);
heteroaryloxy groups (preferably, heteroaryloxy groups having a 5- or 6-membered aromatic heterocyclic group; examples thereof include pyridyloxy and thienyloxy);
alkylcarbonyloxy groups (preferably, alkylcarbonyloxy groups having 2-10 carbon atoms, examples of which include acetoxy);
halogen atoms (in particular, a fluorine atom or a chlorine atom);
silyl groups (examples thereof include trimethylsilyl and triphenylsilyl); According to the present invention are aromatic hydrocarbon ring groups (examples thereof include monovalent groups derived from 5- or 6-membered monocycles or di- to pentacyclic fused rings, such as a benzocyclohexadiene ring, naphthalene ring, anthracene ring, phenanthrene ring, perylene ring, tetracene ring, pyrene ring, benzpyrene ring, chrysene ring, triphenylene ring, and fluoranthene ring);
aromatic heterocyclic groups (examples thereof include monovalent groups derived from 5- or 6-membered monocycles or di- to tetracyclic fused rings, such as a furan ring, benzofuran ring, thiophene ring, benzothiophene ring, pyrrole ring, pyrazole ring, imidazole ring, oxadiazole ring, indole ring, carbazole ring, pyrroloimidazole ring, pyrrolopyrazole ring, pyrrolopyrrole ring, thienopyrrole ring, thienothiophene ring, furopyrrole ring, furofuan ring, thienofuran ring, benzisoxazole ring, benzisothiazole ring, benzimidazole ring, pyridine ring, pyrazine ring, pyridazine ring, pyrimidine ring, triazine ring, quinoline ring, isoquinoline ring, cinnoline ring, quinoxaline ring, benzimidazole ring, perimidine ring, and quinazoline ring ; and
arylamino groups having an aromatic hydrocarbon ring group with 6-12 carbon atoms (examples thereof include phenylamino, diphenylamino, and ditolylamino).

Because of influences on the operating voltage and life of the organic electroluminescent element are taken into account, it is preferred that the hydrocarbon chain portions such as the alkyl, aralkyl, alkoxy, alkenyl, and alkynyl groups should be short (specifically, should have about 1-3 carbon atoms).

Preferred of those, from the standpoints of the electrical stability, heat resistance, etc. of the compound itself, are arylamino and heteroarylamino groups which may have a substituent and which have a group derived from a ring selected from the group consisting of a benzocyclohexadiene ring, naphthalene ring, anthracene ring, dibenzofuran ring, pyrene ring, and fluorene ring. Examples of the substituents which may be possessed by the arylamino groups and heteroarylamino groups include the substituents Z which will be described later.

A mono- or divalent group derived from a compound represented by general formula (V) may be possessed as the substituent which can be possessed by the aromatic hydrocarbon ring group represented by Ar^{b} (according to the invention) or as a part of the groups which can be possessed by the cyclohexadiene ring besides R²¹, R²², and Ar^{b} (disclosed embodiment).

Examples of the substituents which may be possessed besides R²¹, R²², and Ar^{b} include hydroxy, linear or branched alkyl groups which may have a substituent, aralkyl groups which may have a substituent, alkoxy groups which may have a substituent, aryloxy groups which may have a substituent, acyl groups which may have a substituent, alkenyl groups which may have a substituent, alkynyl groups which may have a substituent, acyloxy groups which may have a substituent, or acylamino groups which may have a substituent Preferred from the standpoints of electrical stability, etc. are linear or branched alkyl groups which may have a substituent, aralkyl groups which may have a substituent, alkenyl groups which may have a substituent, alkynyl groups which may have a substituent, and the like.

Examples of the substituent which may be further possessed by the alkyl, aralkyl, alkoxy, aryloxy, acyl, alkenyl, alkynyl, acyloxy, or acylamino groups include the substituents Z shown below. When influences on the operating voltage and life of the organic electroluminescent element are taken into account, it is preferred that the hydrocarbon chain portions such as the alkyl, aralkyl, alkoxy, alkenyl, and alkynyl groups in [Substituents Z] should be short (specifically, should have about 1-3 carbon atoms).

### [Substituents Z]

### Examples thereof include

linear or branched alkyl groups (preferably, linear or branched alkyl groups having 1-8 carbon atoms, examples of which include methyl, ethyl, n-propyl, 2-propyl, n-butyl, isobutyl, and tert-butyl);
aralkyl groups (preferably, aralkyl groups having 7-15 carbon atoms, examples of which include benzyl);
alkoxy groups (preferably, alkoxy groups having 1-8 carbon atoms, examples of which include methoxy, ethoxy, and butoxy);
aryloxy groups (preferably, aryloxy groups having an aromatic hydrocarbon ring group with 6-12 carbon atoms, examples of which include phenyloxy, 1-naphthyloxy, and 2-naphthyloxy);
acyl groups (preferably, acyl groups having 2-10 carbon atoms, examples of which include formyl, acetyl, and benzoyl);
alkenyl groups (preferably, alkenyl groups having 2-9 carbon atoms, examples of which include vinyl, allyl, and 1-butenyl);
alkynyl groups (preferably, alkynyl groups having 2-9 carbon atoms, examples of which include ethynyl and propargyl);
heteroaryloxy groups (preferably, heteroaryloxy groups having a 5- or 6-membered aromatic heterocyclic group; examples thereof include pyridyloxy and thienyloxy);
alkoxycarbonyl groups (preferably, alkoxycarbonyl groups having 2-10 carbon atoms, examples of which include methoxycarbonyl and ethoxycarbonyl);
aryloxycarbonyl groups (preferably, aryloxycarbonyl groups having 7-13 carbon atoms, examples of which include phenoxycarbonyl);
alkylcarbonyloxy groups (preferably, alkylcarbonyloxy groups having 2-10 carbon atoms, examples of which include acetoxy);
halogen atoms (in particular, a fluorine atom or a chlorine atom);
carboxy;
cyano;
hydroxy;
mercapto;
alkylthio groups (preferably, alkylthio groups having 1-8 carbon atoms, examples of which include methylthio and ethylthio);
arylthio groups (preferably, arylthio groups having 6-12 carbon atoms, examples of which include phenylthio and 1-naphthylthio);
sulfonyl groups (examples thereof include mesyl and tosyl);
silyl groups (examples thereof include trimethylsilyl and triphenylsilyl);
boryl groups (examples thereof include dimesitylboryl);
phosphino groups (examples thereof include diphenylphosphino);
aromatic hydrocarbon ring groups (examples thereof include monovalent groups derived from 5- or 6-membered monocycles or di- to pentacyclic fused rings, such as a benzocyclohexadiene ring, naphthalene ring, anthracene ring, phenanthrene ring, perylene ring, tetracene ring, pyrene ring, benzpyrene ring, chrysene ring, triphenylene ring, and fluoranthene ring);
aromatic heterocyclic groups (examples thereof include monovalent groups derived from 5- or 6-membered monocycles or di- to tetracyclic fused rings, such as a furan ring, benzofuran ring, thiophene ring, benzothiophene ring, pyrrole ring, pyrazole ring, imidazole ring, oxadiazole ring, indole ring, carbazole ring, pyrroloimidazole ring, pyrrolopyrazole ring, pyrrolopyrrole ring, thienopyrrole ring, thienothiophene ring, furopyrrole ring, furofuran ring, thienofuran ring, benzisoxazole ring, benzisothiazole ring, benzimidazole ring, pyridine ring, pyrazine ring, pyridazine ring, pyrimidine ring, triazine ring, quinoline ring, isoquinoline ring, cinnoline ring, quinoxaline ring, benzimidazole ring, perimidine ring, and quinazoline ring);
amino groups (preferably, alkylamino groups having one or more alkyl groups with 1-8 carbon atoms, examples of which include methylamino, dimethylamino, diethylamino, and dibenzylamino); and
arylamino groups having an aromatic hydrocarbon ring group with 6-12 carbon atoms (examples thereof include phenylamino, diphenylamino, and ditolylamino).

In the case where those groups further have a substituent, examples of this substituent include the substituents shown above in the section [Substituents Z].

The number of substituents which may be possessed besides R²¹, R²², and Ar^{b} is not limited unless the desired effects are lessened, and the compound may have substituents of two or more different kinds.

None of the substituents which may be possessed by the cyclohexadiene ring besides R²¹, R²², and Ar^{b} in general formula (V) forms a cyclic structure fused to the cyclohexadiene ring. Because of this, the elimination reaction which will be described later occurs at a relatively low temperature of 70°C-200°C and the desired effets can be produced. Although details of the reason therefor are unclear, the reason can be presumed to be as follows.

In case where there is a cyclic structure fused to the cyclohexadiene ring, this cyclic structure is conjugated with the cyclohexadiene ring and this conjugation stabilizes the cyclohexadiene ring. As a result, the reactivity of the elimination group decreases.

Symbol d represents an integer of 1 to 4. From the standpoint of tendency to lower the elimination temperature, it is preferred that d should be 1. Meanwhile, from the standpoint of ease of obtaining a large difference in solubility in organic solvents through the elimination, it is also preferred that d should be 2 or 3.

In the case where a plurality of Ar^{b}s are contained in the molecule of the compound of the invention, the multiple Ar^{b}s may have the same structure of different structures.

In the invention, the molecular weight of the compound is 400 or higher, preferably 500 or higher, more preferably 600 or higher, and is generally 1,000,000 or lower, preferably 500.000 or lower, more preferably 200,000 or lower.

By regulating the molecular weight thereof so as to be within that range, the compound can be easily purified to a high degree. In particular, in case where the compound has a molecular weight lower than 400, this compound has enhanced crystallinity and reduced film-forming properties. It is therefore essential in the invention that the molecular weight of the compound is 400 or higher.

Disclosed herein is a compound represented by the following general formula (II), from the standpoints of ease of obtaining a large difference in solubility in organic solvents through an elimination reaction and of ease of inhibiting crystallization of the compound in a coating film and thereby forming an even film.
These compounds are according to the present invention in as far as the requirements of claim 1 are met 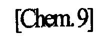 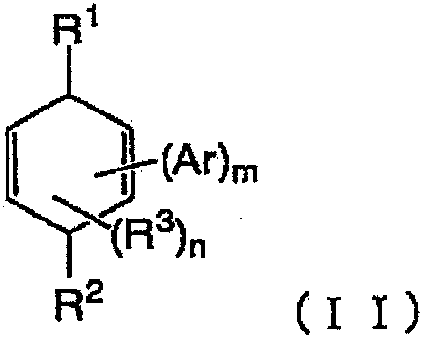
(In general formula (II), Ar, m, R¹, and R² respectively have the same meanings as the Ar^{b}, d, R²¹, and R²² contained in general formula (V).
R³ represents a hydroxy group, an alkyl group which may have a substituent, an aralkyl group which may have a substituent, an alkoxy goup which may have a substituent, an aryloxy group which may have a substituent, an acyl group which may have a substituent, an alkenyl group which may have a substituent, an alkynyl group which may have a substituent, an acyloxy group which may have a substituent, or an acylamino group which may have a substituent.
Symbol n represents an integer of 0 to 3, and 1≤m+n≤4.
When a plurality of Ar groups and R³ groups are contained in the molecule, the groups may be the same or different.
Although the cyclohexadiene ring in general formula (II) may have one or more substituents besides R¹, R², and Ar, none of the substituents forms a cyclic structure fused to the cyclohexadiene ring.)

Ar, m, R¹, and R² in general formula (II) respectively have the same meanings as the Ar^{b}, d, R²¹, and R²² contained in general formula (V), and examples and preferred examples of Ar, m, R¹, and R² also are the same as those of the Ar^{b}, d, Ar²¹, and R²² contained in general formula (V).

R³ is a substituent which can be possessed, besides Ar, R¹, and R², by the cyclohexadiene ring contained in general formula (II). Like the substituents which can be possessed, besides Ar^{b}, R²¹, and R²², by the cyclohexadiene ring contained in general formula (V), R³ represents a hydroxy group, an alkyl group which may have a substituent, an aralkyl group which may have a substituent, an alkoxy group which may have a substituent, an aryloxy group which may have a substituent, an acyl group which may have a substituent, an alkenyl group which may have a substituent, an alkynyl group which may have a substituent, an acyloxy group which may have a substituent, or an acylamino group which may have a substituent. Examples and preferred examples of R³ are the same as those of the substituents which can be possessed, besides Ar^{b}, R²¹, and R²², by the cyclohexadiene ring contained in general formula (V).

Symbol n in general formula (II) represents an integer of O to 3, and 1≤m+n≤4.

It is preferred that n should be 1 to 3 from the standpoints of high solubility in organic solvents and satisfactory film-forming properties. From the standpoints of high charge-transporting ability and high glass transition temperature, it is preferred that n should be 0 to 2.

It is more preferred that the compound represented by general formula (II) should be a compound represented by any of the following general formulae (VI) to (IX), from the standpoint of obtaining a large difference in solubility in organic solvents through an elimination reaction of the elimination group portion.
These compounds are according to the present invention in as far as the requirements of claim 1 are met.

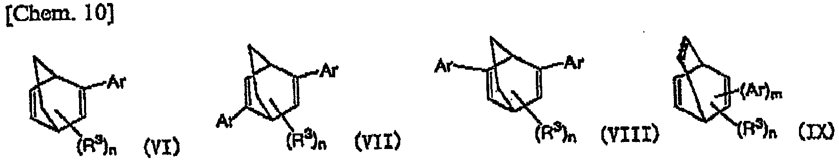

(In general formulae (VI) to (IX), Ar, R³, m, and n have the same meanings as in general formula (II).)

Of these, compounds represented by general formula (VIII) are especially preferred for the following reason.

In each of the compounds represented by general formula (VIII), one Ar on the cyclohexadiene ring is located in a position meta to the other Ar. It is thought that the compound hence has satisfactory charge-transporting ability. In addition, the m-position bonding exerts a relatively great influence based on steric hindrance and, hence, the compound which has undergone an elimination reaction is less apt to crystallize. Thus, evenness of the film formed from such a compound can be ensured.

Furthermore, the compounds represented by general formula (VIII) have a elimination group relatively at the center of the molecular structure and are hence apt to be affected by a change in molecular structure which occurs through an elimination reaction. Namely, it is presumed that the compounds are apt to have a large difference in solubility in organic solvents through an elimination reaction.

From the standpoint of enhancing hole-transporting ability, it is more preferred that the compounds should contain in the molecule a mono- or divalent group represented by the following general formula (X), unless the desired effects are lessened. 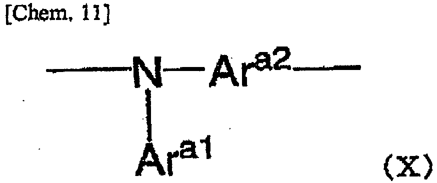
(In general formula (X), Ar^{a1} and Ar^{a2} each independently represent an aromatic hydrocarbon ring group which may have a substituent or an aromatic heterocyclic group which may have a substituent.
However, when the Ar^{a2} is present at an end, one of the linking portions is replaced with a hydrogen atom.)

In general formula (X), Ar^{a1} and Ar^{a2} may be the same or different. Ar^{a1} and Ar^{a2} each independently represent an aromatic hydrocarbon ring group which may have a substituent or an aromatic heterocyclic group which may have a substituent. When the Ar^{a2} is present at an end, a hydrogen atom is bonded to the linking portion not bonded to the nitrogen atom.

Examples of the optionally substituted aromatic hydrocarbon ring groups and optionally substituted aromatic heterocyclic groups which are represented by Ar^{a1} and Ar^{a2} and examples of the substituents thereof are the same as those of the Ar¹¹ to Ar¹⁵ contained in formula (IV) which will be described later.

Examples of the groups which may be further possessed by the compound of the invention are shown as the following structural formulae C.

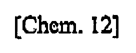

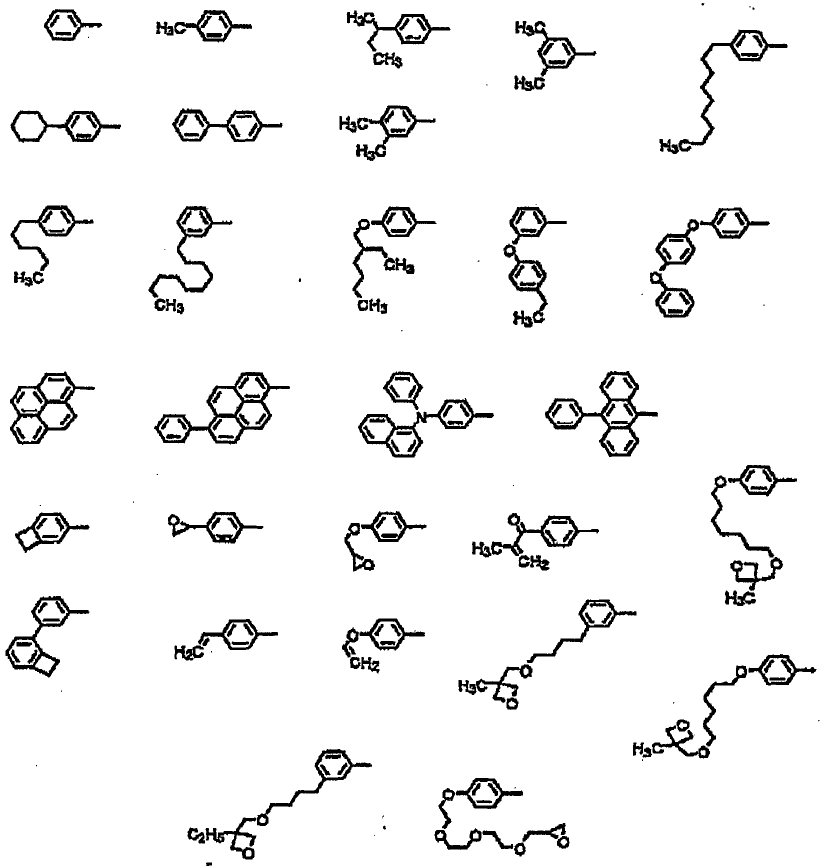

The elimination temperature of the elimination group portion in the compound of the invention is generally 200°C or lower.

Namely, in the compound of the invention, the elimination group is eliminated from the cyclohexadiene ring of the compound generally at 200°C or lower. However, the elimination temperature is more preferably 190°C or lower, even more preferably 180°C or lower. Meanwhile, the elimination group is eliminated from the cyclohexadiene ring generally at 70°C or higher. However, the elimination temperature is more preferably 100°C or higher, even more preferably 110°C or higher.

By regulating the temperature at which the elimination group is eliminated from the cyclohexadiene ring of the compound to a value within that range, film formation at a low temperature is rendered possible. As a result, the heat treatment to be conducted after film formation exerts little influence on the substrate and the other layers, and an organic electroluminescent element having a long working life can be provided.

### <Examples of the Compound of the Invention>

Preferred examples of the compound of the invention are shown below in a far as these compounds are within the scope of present claim 1.

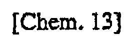

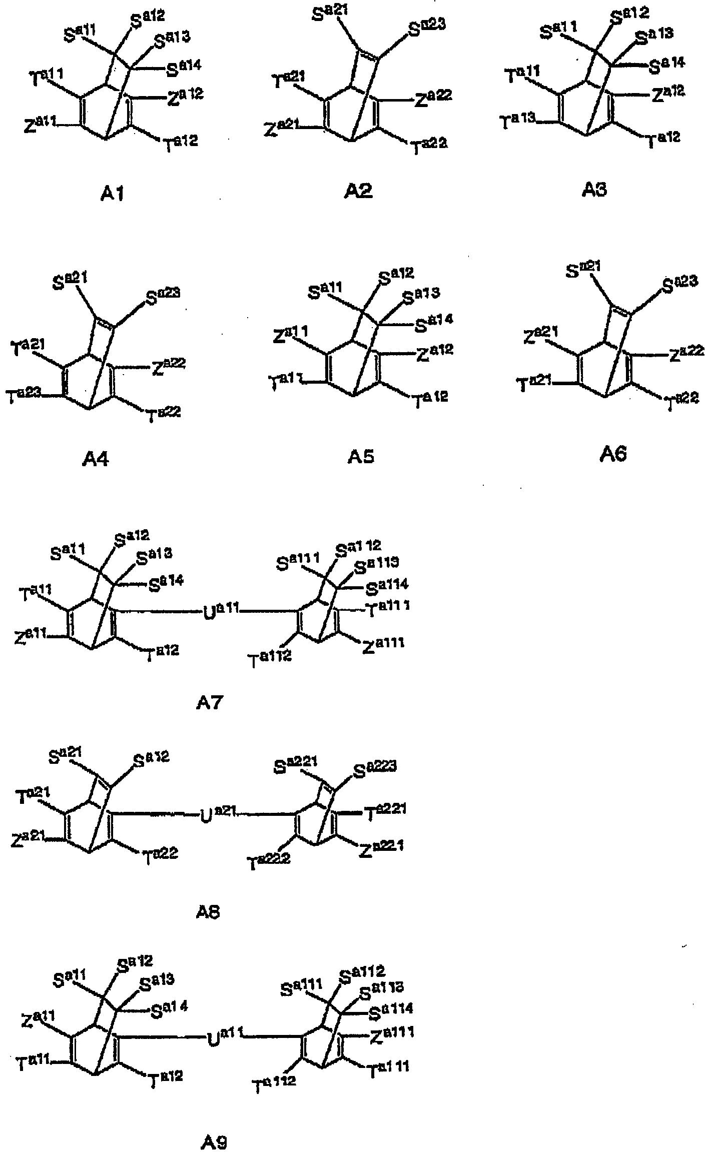

(Combinations of Z^{a11}, Z^{a12}, Z^{a21}, Z^{a22}, Z^{a111}, Z^{a221}, S^{a11} to S^{a14}, S^{a111} to S^{a114}, S^{a21} to S^{a23}, S^{a221} to S^{a223}, T^{a11} to T^{a13}, T^{a21} to T^{a23}, T^{a111}, T^{a112}, T^{a221}, T^{a222}, U^{a11}, and U^{a21} in general formulae (A1) to (A9) are shown in the following Tables 1 to 3).

### [A1 to A6]

Combinations of Z^{a11} and Z^{a12}, Z^{a21} and Z^{a22}, S^{a11} to S^{a14}**,** S^{a21} to S^{a23}, T^{a11} to T^{a13}, and T^{a21} to T^{a23} in general formulae (A1) to (A6) are shown in Table 1.

**[Table 1]**

| | No. 1 | No. 2 | No. 3 | No. 4 | No. 5 | No. 6 | No. 7 | No. 8 |
|---|---|---|---|---|---|---|---|---|
| Z^{a11}, Z^{a21} | 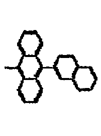 | 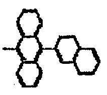 | 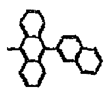 | 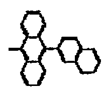 | 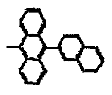 | -H | 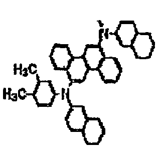 | 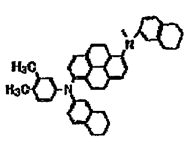 |
| Z^{a12}, Z^{a22} | | 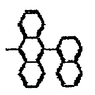 | 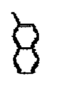 | 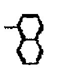 | 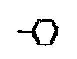 | 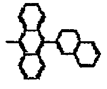 | | |
| T^{a11}∼T^{a13} | -H | -H | -H | -H | -H | 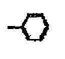 | -H | -H |
| T^{a21}∼T^{a23} | | | | | | | | |
| S^{a11}∼S^{a14} | -H | -H | -H | -H | -H | -H | -H | -H |
| S^{a21}∼S^{a23} | | | | | | | | |

### [A3]

With respect to general formula (A3), other combinations of Z^{a12}, S^{a11} to S^{a14}, and T^{a11} to T^{a13} are shown in Table 2.

**[Table 2]**

| | No. 9 | No. 10 | No.11 | No. 12 | No. 13 | No. 14 |
|---|---|---|---|---|---|---|
| Z^{a12} | 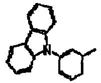 | 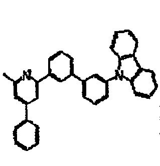 | 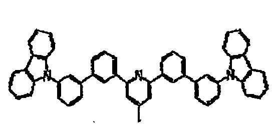 | 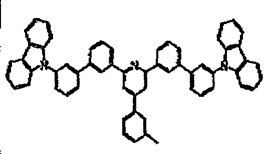 | 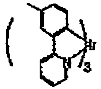 | 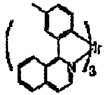 |
| T^{a11}∼T^{a13} | -H | -H | -H | -H | -H | -H |
| S^{a11}∼S^{a14} | | | | | | |

### [A7 to A9]

With respect to general formulae (A7) to (A9), combinations of Z^{a11} and Z^{a111}, Z^{a21} and Z^{a221}, S^{a11} to S^{a14}, S^{a21}, S^{a111} to S^{a114}, S^{a221} to S^{a223}, T^{a11} and T^{a12}, T^{a21} and T^{a22}, T^{a111} and T^{a112}, and T^{a221} and T^{a222} are shown in Table 3.

**[Table 3]**

| | No. 15 | No. 16 | No. 17 | No. 18 | No. 19 | No. 20 | No. 21 | No. 22 | No. 23 | No. 24 | No. 25 | No. 26 (A7AB) |
|---|---|---|---|---|---|---|---|---|---|---|---|---|
| Z^{a11}, Z^{a21} | 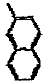 | 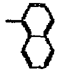 | 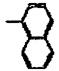 | 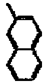 | 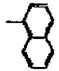 | 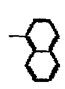 | 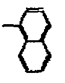 | 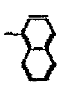 | 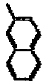 | 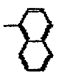 | 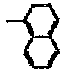 | 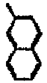 |
| Z^{a111}, Z^{a221} | | 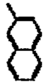 | | | 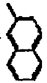 | | -H | -H | -H | -H | -H | -H |
| T^{a11}∼T^{a12} | | | | | | | 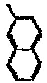 | 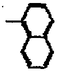 | 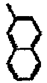 | 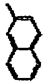 | 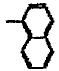 | 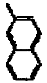 |
| T^{a111}, T^{a112} | -H | -H | -H | -H | -H | -H | | | | | | |
| T^{a21}∼T^{a22} | | | | | | | | | | | | |
| T^{a221}, T^{a222} | | | | | | | | | | | | |
| U^{a11}, U^{a21} | 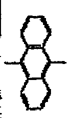 | 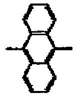 | 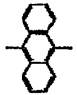 | 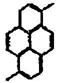 | 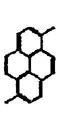 | 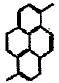 | 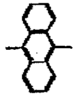 | 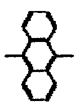 | 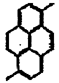 | 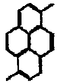 | 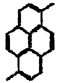 | 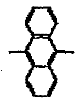 |
| S^{a11}∼S^{a14} | | | | | | | | | | | | |
| S^{a111}∼S^{a114} | -H | -H | -H | -H | -H | -H | -H | -H | -H | -H | -H | -H |
| S^{a21} | | | | | | | | | | | | |
| S^{a221}, S^{a223} | | | | | | | | | | | | |

### <Methods for Synthesizing the Compound of the Invention>

The compound of the invention can be synthesized, for example, by the following methods from starting materials selected according to the structure of the target compound.

### Examples of synthesis methods include

(1) a method in which a diene compound is caused to add chloroacrylonitrile to synthesize an organic compound having a elimination group (synthesis schemes 1 to 3),
(2) a method in which benzoquinone is reacted with maleic anhydride to synthesize an organic compound having a elimination group (synthesis scheme 4),
(3) a method in which a diene compound is reacted with methylsilylacetylene to synthesize an organic compound having a elimination group (synthesis scheme 5),
(4) a method in which a diene compound is reacted with an acetylenedicarboxylic acid ester to synthesize an organic compound having a elimination group (synthesis scheme 6),
(5) a method in which cyclohexen-1-one is subjected to addition reaction with a Meldrum's acid to synthesize an organic compound having a elimination group (synthesis scheme 7),
(6) a method in which dimethyl 2,5-dioxo-1,4-cyclohexanedicarboxylate is subjected to addition reaction with 1,4-dibromoethane to synthesize an organic compound having a elimination group (synthesis scheme 8), and
(7) a method in which a cyclohexadiene ring is reacted with benzyl acrylate to synthesize an organic compound having a elimination group (synthesis scheme 9).

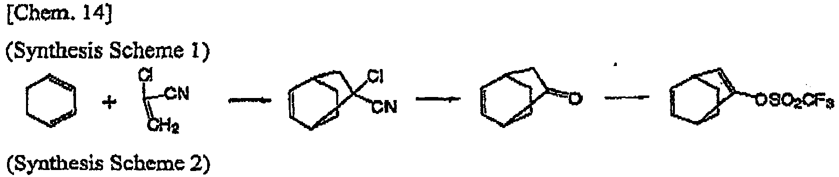

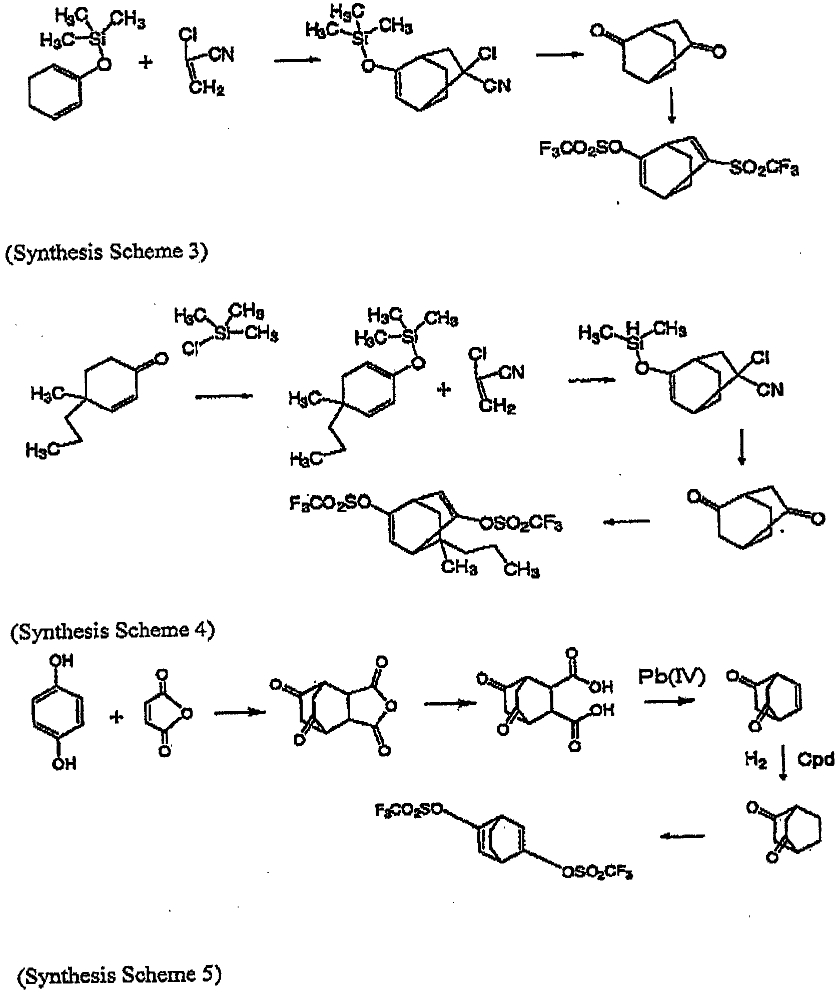

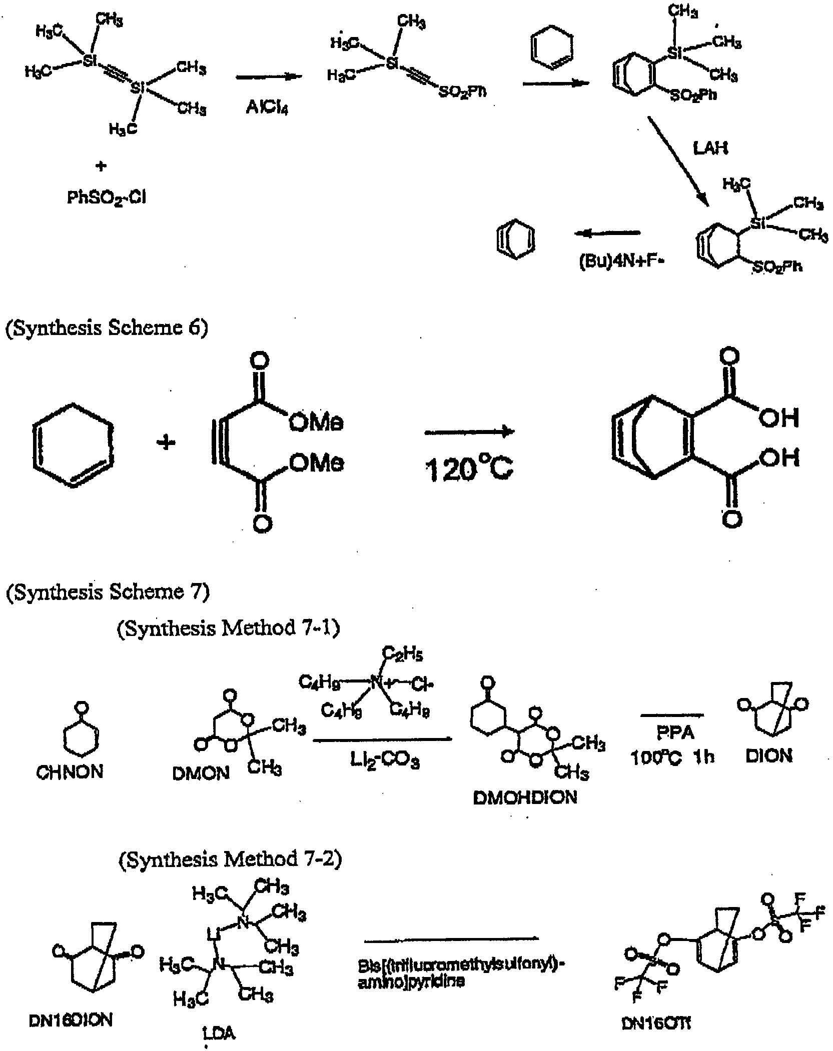

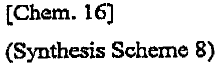

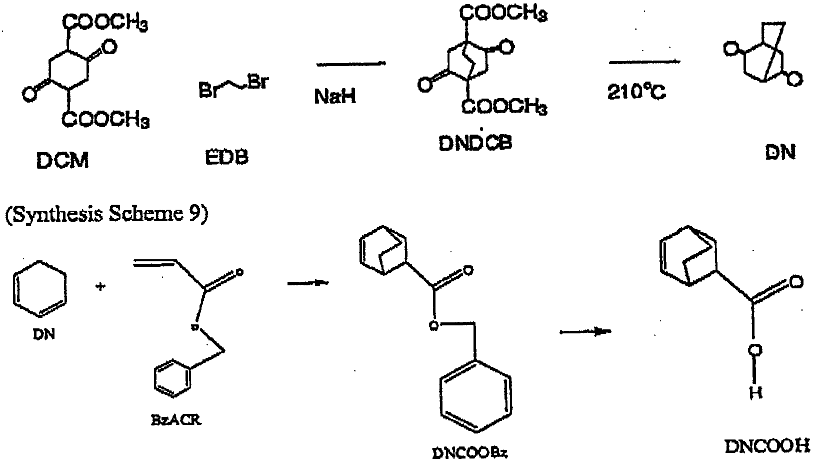

Of the synthesis methods shown above, synthesis methods (1), (2), and (5) are preferred from the standpoint that in these methods, the amount of by-products resulting from the addition reaction is small and the target compound is easy to obtain. Synthesis methods (1) and (5) are more preferred especially from the standpoint of a high yield of bicyclooctadiene.

A dibromo form of a compound having a elimination group is obtained through the reactions shown by the following synthesis schemes A1 to A3.

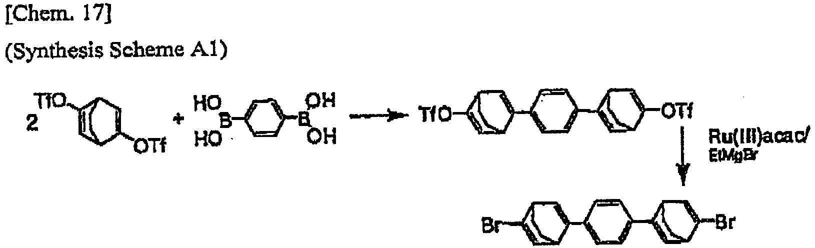

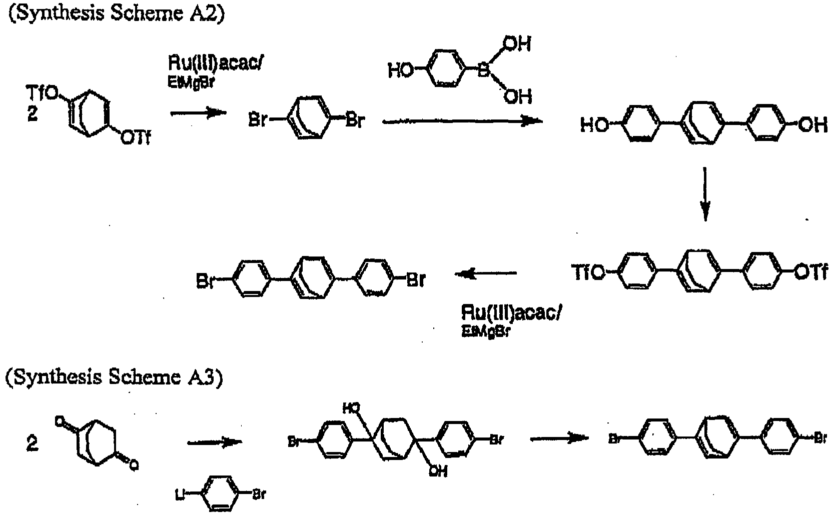

Meanwhile, a diamino form of a compound having a elimination group is synthesized by introducing p-amidophenyl groups into a dihydroxy form of a compound having a elimination group and aminating the amide groups with potassium hydroxide, as shown by synthesis scheme A4.

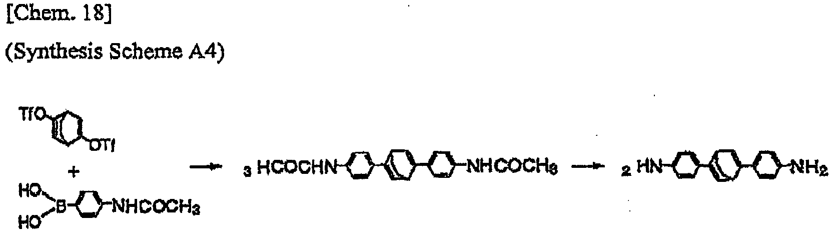

With respect to low-molecular compounds also, known coupling techniques such as the reaction for forming an N-Ar bond and reaction for forming an Ar-Ar bond which will be shown later in the section [Methods for Synthesizing the Polymer] can be used to synthesize the compounds.

Methods for synthesizing a compound having a single molecular weight using a reaction for forming an N-Ar bond or a redaction for forming an Ar-Ar bond are shown below.

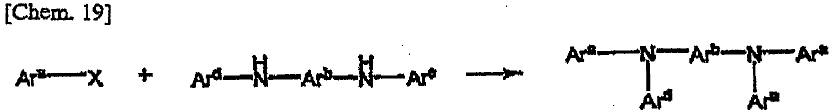

(In the formulae, X represents a halogen atom or a sulfonic ester group, e.g., a CF₃SO₂O- group. Ar^{a}, Ar^{d}, and Ar^{c} each independently represent an aromatic hydrocarbon ring group which may have a substituent or an aromatic heterocyclic group which may have a substituent, and Ar^{b} represents a divalent aromatic hydrocarbon ring group which may have a substituent or a divalent aromatic heterocyclic group which may have a substituent At least one of Ar^{a}, Ar^{b}, Ar^{d}, and Ar^{c} has a partial structure including a elimination group bonded thereto.) 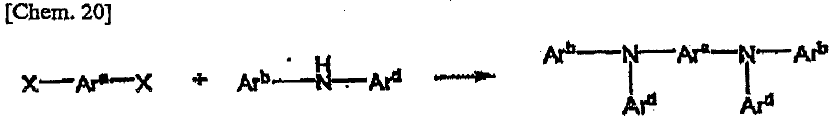
(In the formulae, X represents a halogen atom or a sulfonic ester group, e.g., a CF₃SO₂O- group. Ar^{b} and Ar^{d} each independently represent an aromatic hydrocarbon ring group which may have a substituent or an aromatic heterocyclic group which may have a substituent, and Ar^{a} represents a divalent aromatic hydrocarbon ring group which may have a substituent or a divalent aromatic heterocyclic group which may have a substituent
At least one of Ar^{a}, Ar^{b}, and Ar^{d} has a partial structure including a elimination group bonded thereto.)

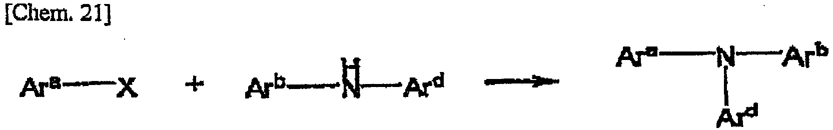

(In the formulae, X represents a halogen atom or a sulfonic ester group, e.g., a CF₃SO₂O- group. Ar^{a}, Ar^{b}, and Ar^{d} each independently represent an aromatic hydrocarbon ring group which may have a substituent or an aromatic heterocyclic group which may have a substituent.
At least one of Ar^{a}, Ar^{b}, and Ar^{d} has a partial structure including a elimination group bonded thereto.)

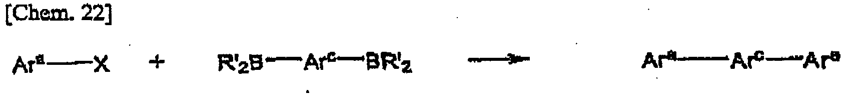

(In the formulae, X represents a halogen atom or a sulfonic ester group, e.g., a CF₃SO₂O- group, and R' represents a hydroxy group or the R's represent alkoxy groups which may be bonded to each other to form a ring. Ar^{a} represents an aromatic hydrocarbon ring group which may have a substituent or an aromatic heterocyclic group which may have a substituent, and Ar^{c} represents a divalent aromatic hydrocarbon ring group which may have a substituent or a divalent aromatic heterocyclic group which may have a substituent. Ar^{a} and/or Ar^{c} has a partial structure including a elimination group bonded thereto.)

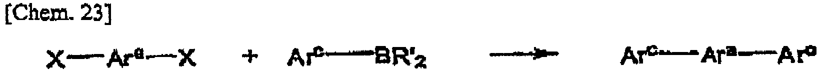

(In the formulae, X represents a halogen atom or a sulfonic ester group, e.g., a CF₃SO₂O- group, and R' represents a hydroxy group or the R's represent alkoxy groups which may be bonded to each other to form a ring. Ar^{c} represents an aromatic hydrocarbon ring group which may have a substituent or an aromatic heterocyclic group which may have a substituent, and Ar^{a} represents a divalent aromatic hydrocarbon ring group which may have a substituent or a divalent aromatic heterocyclic group which may have a substituent. Ar^{a} and/or Ar^{c} has a partial structure including a elimination group bonded thereto, and may have a crosslinkable group.)

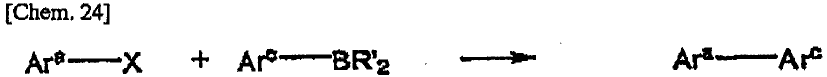

(In the formulae, X represents a halogen atom or a sulfonic ester group, e.g., a CF₃SO₂O- group, and R' represents a hydroxy group or the R's represent alkoxy groups which may be bonded to each other to form a ring. Ar^{a} and Ar^{c} each independently represent an aromatic hydrocarbon ring group which may have a substituent or an aromatic heterocyclic group which may have a substituent Ar^{a} and/or Ar^{c} has a partial structure including a elimination group bonded thereto.)

### <Polymer of the Invention>

The polymer of the invention is characterized by having a monovalent or divalent group represented by the following general formula (III). 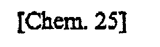 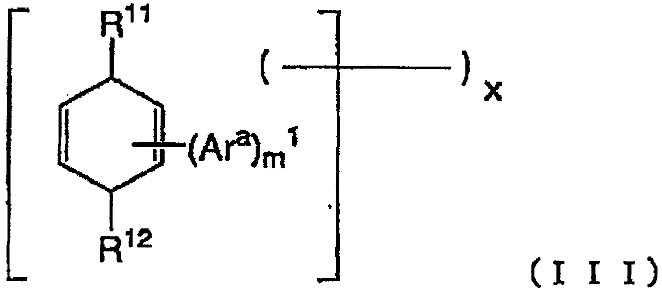
(In general formula (III), R¹¹ and R¹² have been bonded to each other to represent the group represented by -CH=CH- or -CH₂-CH₂-;
Ar^{a} represents an aromatic hydrocarbon ring group which may have a substituent as defined in claim 1, and m¹ represents an integer of 1-4.
Symbol x represents 1 or 2.
When a plurality of Ar^{a}s are contained in the molecule, the Ar^{a}s may be the same or different
Disclosed herein are compounds wherein the cylclohexadiene ring in general formula (III) has one or more substituents besides R¹¹, R¹², and Arⁿ, and none of the substituents forms a cyclic structure fused to the cyclohexadiene ring.)

Examples of Ar^{a} are the same as the examples of the Ar^{b} contained in general formula (V), and preferred examples thereof also are the same.

Symbol m¹ represents an integer of I to 4, and x represents 1 or 2.

Symbol m¹ is the same as the d contained in general formula (V), and a preferred range thereof also is the same.

Furthermore, disclosed herein are compounds of general formula (III) in which the cyclohexadiene ring may have one or more substituents besides R¹¹, R¹², and Ar^{a}. The substituents possessed by the cyclohexadiene ring besides R¹¹, R¹², and Ar^{a} are the same as the substituents possessed, besides R²¹, R²², and Ar^{b}, by the cyclohexadiene ring contained in general formula (V), and preferred examples thereof also are the same.

For the reason described above, the polymer of the invention satisfies that none of the substituents possessed besides R¹¹, R¹², and Ar^{a} by the cyclohexadiene ring contained in general formula (III) forms a cyclic structure fused to the cyclohexadiene ring.

It is also preferred that the organic compounds of the invention should be polymers, from the following standpoints.

In the case of a polymer, the number of elimination groups contained per molecule is easy to regulate. Consequently, it is easy to obtain a large difference in solubility in organic solvents through an elimination reaction.

Furthermore, the polymer of the invention is relatively less apt to crystallize and, hence, has excellent film-forming properties. Consequently, when the polymer of the invention is used to obtain an organic electroluminescent element, current concentration does not occur and short-circuiting or the like is hence less apt to occur. It is therefore easy to obtain an organic electroluminescent element having a long working life.

The weight-average molecular weight of the polymer of the invention is generally 3,000,000 or lower, preferably 1,000,000 or lower, more preferably 500,000 or lower, and is generally 1,000 or higher, preferably 2,500 or higher, more preferably 5,000 or higher.

On the other hand, the number-average molecular weight thereof is generally 2,500,000 or lower, preferably 750,000 or lower, more preferably 400,000 or lower, and is generally 500 or higher, preferably 1,500 or higher, more preferably 3,000 or higher.

Usually, the weight-average molecular weights are determined by SEC (size exclusion chromatography) analysis. In SEC analysis, components having higher molecular weights are eluted in shorter time periods, while components having lower molecular weights necessitate longer elution times. Elution times for a sample are converted to molecular weights using a calibration curve calculated from elution times for polystyrene having known molecular weights (reference samples), and a weight-average molecular weight and a number-average molecular weight can be thus calculated.

In the invention, by regulating the molecular weights of the polymer so as to be within those ranges, purification of the polymer is facilitated. It is also possible to obtain the polymer which has satisfactory film-forming properties, a high glass transition temperature, a high melting point, a high vaporization temperature, and excellent heat resistance.

The polymer of the invention has a solubility in toluene at ordinary temperature (25°C) and ordinary pressure (100 kPa) of generally 0.1% by weight or more, preferably 0.5% by weight or more, more preferably 1% by weight or more.

The elimination groups in the polymer of the invention may be contained in parts other than the repeating units of the polymer (i.e., in the molecular ends). The number of elimination groups contained per molecule of the polymer is preferably 5 or more on average, more preferably 10 or more on average, even more preferably 50 or more on average. When the number thereof is less than the lower limit, there are cases where the polymer which has not undergone insolubilization has low solubility in organic solvents and there is a possibility that this compound might undergo an insufficient difference in solubility in organic solvents through insolubilization.

It is preferred that the polymer of the invention should be a polymer which further contains a repeating unit represented by the following general formula (IV). 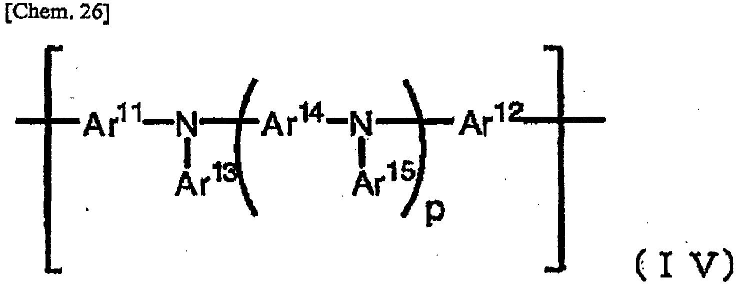
(In general formula (IV), p represents an integer of 0-3, and Ar¹¹ and Ar¹² each independently represent a direct bond or an aromatic ring group which may have a substituent.
Ar¹³ to Ar¹⁵ each independently represent an aromatic ring group which may have a substituent.
However, at least Ar¹¹ or Ar¹² is not a direct bond.)

In general formula (IV), Ar¹¹ and Ar¹² each independently represent a direct bond or an aromatic ring group which may have a substituent, and Ar¹³ to Ar¹⁵ each independently represent an aromatic ring group which may have a substituent.

Examples of the aromatic hydrocarbon ring group which may have a substituent include mono- or divalent groups derived from 5- or 6-membered monocycles or di- to pentacyclic fused rings, such as a benzocyclohexadiene ring, naphthalene ring, anthracene ring, phenanthrene ring, perylene ring, tetracene ring, pyrene ring, benzpyrene ring, chrysene ring, triphenylene ring, fluorene ring, and fluoranthene ring.

Examples of the aromatic heterocyclic group which may have a substituent include mono- or divalent groups derived from 5- or 6-membered monocycles or di- to tetracyclic fused rings, such as a furan ring, benzofuran ring, dibenzofuran ring, thiophene ring, benzothiophene ring, pyrrole ring, pyrazole ring, imidazole ring, oxadiazole ring, indole ring, carbazole ring, pyrroloimidazole ring, pyrrolopyrazole ring, pyrrolopyrrole ring, thienopyrrole ring, thienothiophene ring, furopyrrole ring, furofuran ring, thienofuran ring, benzisoxazole ring, benzisothiazole ring, benzimidazole ring, pyridine ring, pyrazine ring, pyridazine ring, perimidine ring, triazine ring, quinoline ring, isoquinoline ring, cinnoline ring, quinoxaline ring, perimidine ring, and quinazoline ring.

From the standpoint of the electrochemical stability of the polymer to be obtained, it is preferred that Ar¹¹ to Ar¹⁵ each should independently be a mono- or divalent group derived from a ring selected from the group consisting of a benzocyclohexadiene ring, naphthalene ring, anthracene ring, phenanthrene ring, pyrene ring, and fluorene ring. It is also preferred that Ar¹¹ to Ar¹⁵ each should be a divalent group composed of rings of one or more kinds selected from that group and bonded to each other through a direct bond. More preferred are a biphenylene group, a terphenylene group, and the like.

Examples of the substituents which may be possessed by the aromatic hydrocarbon ring groups and aromatic heterocyclic groups represented by Ar¹¹ to Ar¹⁵ include the substituents given above in the section [Substituents Z] and groups represented by general formula (III). The number of these substituents is not limited, and those aromatic groups each may have, for example, one or more substituents of one kind or substituents of two or more kinds in any desired combination.

Symbol p represents an integer of 0 to 3.

It is preferred that p should be 0, from the standpoint of enhancing the solubility in organic solvents and the film-forming properties of the polymer. From the standpoint of improving the hole-transporting ability of the polymer, it is preferred that p should be 1 to 3.

Besides the repeating unit represented by general formula (IV), the polymer may contain a repeating unit which is a divalent group represented by general formula (III) and further contain other repeating units. Two or more kinds of repeating units represented by general formula (IV) and two or more kinds of repeating units which are divalent groups represented by general formula (III) may be present in the molecule.

The content of repeating units which are divalent groups represented by general formula (III) in the polymer chain is generally 5% by mole or higher, preferably 10% by mole or higher.

It is preferred that the polymer of the invention should be one in which at least one of Ar¹¹, Ar¹², and Ar¹⁴ in general formula (IV) contains a group represented by general formula (III). More specifically, it is preferred that at least one of Ar¹¹, Ar¹², and Ar¹⁴ in formula (IV) should be a group represented by the following formula (IV-1) or (IV-2). 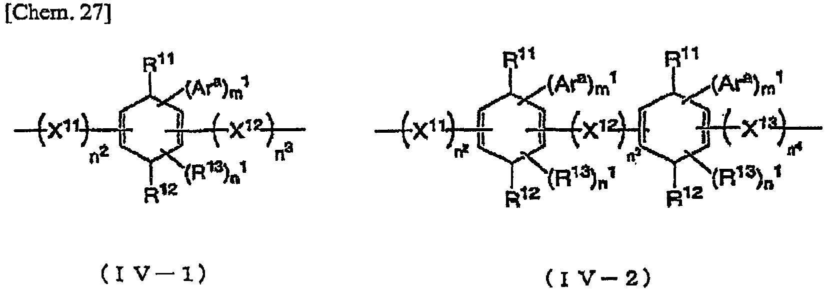
(In general formulae (IV-1) and (IV-2), R¹¹, R¹², Arⁿ, and m¹ have the same meanings as in general formula (III).
R¹³ has the same meaning as the R³ contained in general formula (II).
Symbol n¹ represents an integer of 0 to 3, and n² to n⁴ each independently represent an integer of 0 to 5.
X¹¹ to X¹³ each represent an aromatic hydrocarbon ring group which has 6-50 carbon atoms and may have a substituent or an aromatic heterocyclic group which has 3-50 carbon atoms and may have a substituent.
When a plurality of Ar^{a}s, R¹¹s, R¹²s, R¹³s, X¹¹s, X¹²s, or X¹³s are contained in the molecule, these groups may be the same or different.
The cyclohexadiene rings in general formulae (IV-1) and (IV-2) may have one or more substituents besides Ar^{a}, R¹¹ to R¹³, and X¹¹ to X¹³. However, none of these substituents forms a cyclic structure fused to the cyclohexadiene rings.)

It is preferred that the polymer of the invention should be one in which at least Ar¹³ or Ar¹⁵ in general formula (IV) contains a group represented by general formula (III). More specifically, it is preferred that at least Ar¹³ or Ar¹⁵ in general formula (IV) should be a group represented by the following general formula (IV-3). 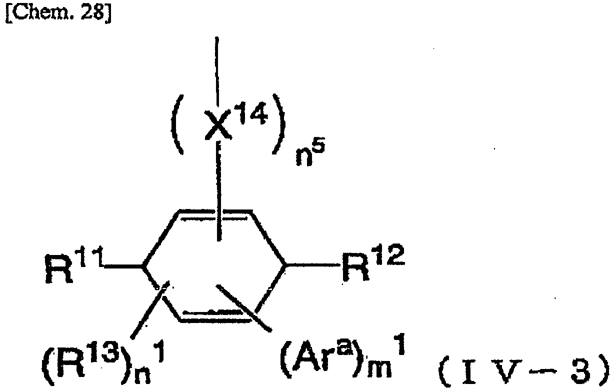
(In general formula (IV-3), R¹¹, R¹², Ar^{a}, and m¹ have the same meanings as in general formula (III).
R¹³ and n¹ have the same meanings as in general formulae (IV-1) and (IV-2).
X¹⁴ represents an aromatic hydrocarbon ring group which has 6-50 carbon atoms and may have a substituent or an aromatic heterocyclic group which has 3-50 carbon atoms and may have a substituent.
When a plurality of Ar^{a}s, R¹¹s, R¹²s, or X¹⁴s are contained in the molecule, these groups may be the same or different.
The cyclohexadiene ring in general formula (IV-3) may have one or more substituents besides Ar^{a}, R¹¹ to R¹³, and X¹⁴. However, none of these substituents forms a cyclic structure fused to the cyclohexadiene ring.)

Ar, R¹ to R³, m¹, and n¹ in general formulae (IV-1) and (IV-2) and general formula (IV-3) have the same meanings as in general formula (V) or (II), and examples and preferred examples thereof also are the same.

Symbols n² to n⁴ in general formulae (IV-1) to (IV-2) each independently represent an integer of 0 to 5.

In the case of use in luminescent-layer applications, it is preferred that n² to n⁴ should be 1 or 2, from the standpoint that an excited state in which visible-light luminescence occurs is obtained. In the case of use in a hole injection layer or a hole transport layer, it is preferred that n² to n⁴ should be 0 or 1, from the standpoint of heightening the rate of hole movement,

X¹¹ to X¹⁴ in general formulae (IV-1) to (IV-3) each independently represent an aromatic hydrocarbon ring group which has 6-50 carbon atoms and may have a substituent or an aromatic heterocyclic group which has 3-50 carbon atoms and may have a substituent.

Examples of the aromatic hydrocarbon ring group having 6-50 carbon atoms include a benzocyclohexadiene ring, naphthalene ring, anthracene ring, phenanthrene ring, perylene ring, tetracene ring, pyrene ring, benzpyrene ring, chrysene ring, triphenylene ring, fluoranthene ring, and fluorene ring.

Examples of the aromatic heterocyclic group having 3-50 carbon atoms include a furan ring, benzofuran ring, dibenzofuran ring, thiophene ring, benzothiophene ring, pyrrole ring, pyrazole ring, imidazole ring, oxadiazole ring, indole ring, carbazole rang, pyridine ring, pyrimidine ring, quinoline ring, isoquinoline ring, quinoxaline ring, perimidine ring, quinazoline ring, and quinazolinone ring.

In the case where X¹ to X⁴ are aromatic hydrocarbon ring groups or aromatic heterocyclic groups, the following groups are especially preferred as these groups from the standpoint of electrical stability. Especially preferred aromatic hydrocarbon ring groups include a benzocyclohexadiene ring, naphthalene ring, anthracene ring, perylene ring, tetracene ring, pyrene ring, fluorene ring, and the like. On the other hand, especially preferred aromatic heterocyclic groups include a furan ring, benzofuran ring, dibenzofuran ring, pyrrole ring, pyrazole ring, oxadiazole ring, carbazole ring, benzimidazole ring, pyridine ring, pyrazine ring, pyridazine ring, pyrimidine ring, and the like.

Examples of the substituents which may be possessed by the aromatic hydrocarbon ring groups or aromatic heterocyclic groups represented by X¹¹ to X¹⁴ include the substituents enumerated above in the section [Substituents Z]. Especially preferred of these, from the standpoints of improvement in film-forming properties and of electrical stability, are alkyl groups such as methyl, ethyl, n-propyl, 2-propyl, n-butyl, isobutyl, and t-butyl, aromatic hydrocarbon ring groups such as a benzocyclohexadiene ring, naphthalene ring, and anthracene ring, and aromatic heterocyclic groups such as a furan ring and a dibenzofuran ring.

The content of the repeating units containing any of general formulae (IV-1) to (IV-3) in the polymer chain is preferably 10% by mole or higher, more preferably 30% by mole or higher.

### <Examples of the Polymer>

Preferred examples of the repeating units possessed by the polymer of the invention are shown below. These examples are according to the present invention in as far as they fall within the scope of the appended claims.

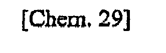

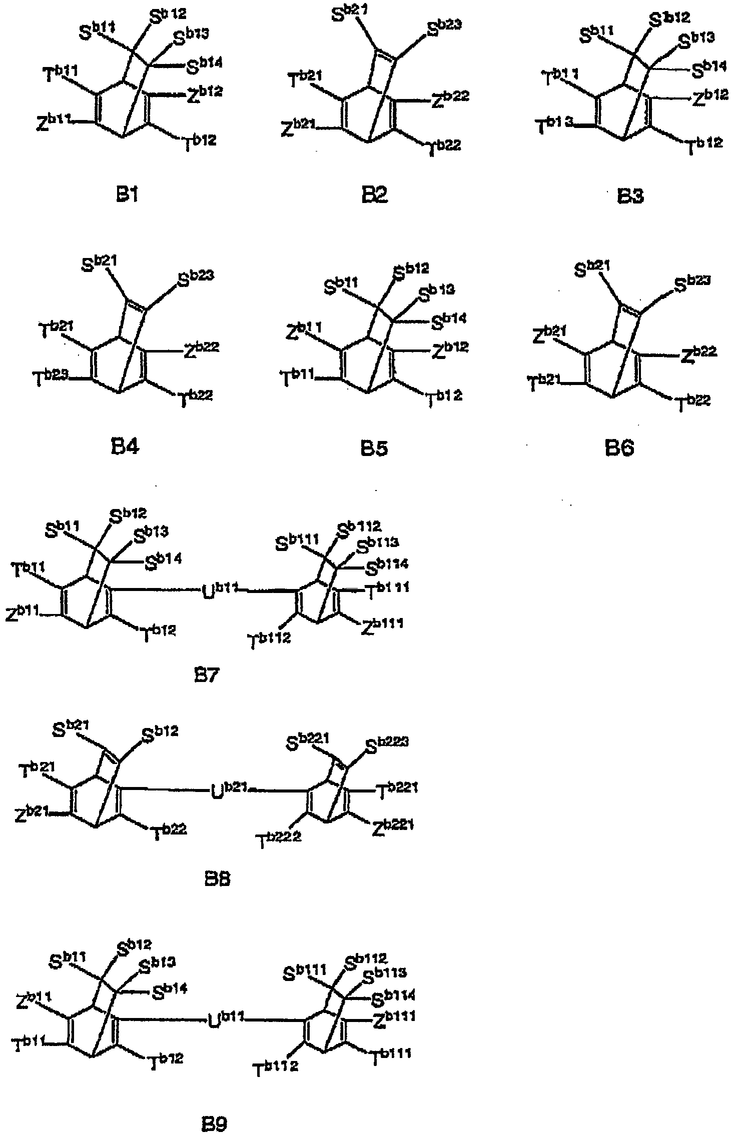

(Combinations of Z^{b11} to Z^{b12}, Z^{b21} to Z^{b22}, Z^{b111}, Z^{b221}, S^{b11} to S^{b14}, S^{b21} to S^{b23}, S^{b111} to S^{b114}, S^{b221} to S^{b223}, T^{b11} to T^{b13}, T^{b21} to T^{b23}. T^{b111} to T^{b112}, T^{b221} to T^{b222}, U^{b11}, and U^{b21} in general formulae (B1) to (B9) are shown in the following Tables 4 and 5.)

### [B1 to B6]

With respect to general formulae (B 1) to (B6), combinations of Z^{b11} to Z^{b12}, Z^{b21} to Z^{b22}, S^{b11} to S^{b14}, S^{b21} to S^{b23}, T^{b11} to T^{b13}, and T^{b21} to T^{b23} are shown in Table 4.

[Table 4]

**Table 4**

| | No.101 | No.102 | No.103 | No.104 | No.105 | No.106 |
|---|---|---|---|---|---|---|
| Z^{b11}, Z^{b12} | Covalent bond | Covalent bond | 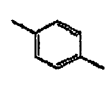 | 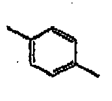 | 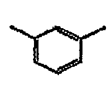 | 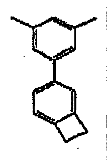 |
| Z^{b21}, Z^{b22} | | | | | | |
| T^{b11}∼ T^{b13} | | | | | | |
| T^{b21}∼T^{b23} | -H | -H | -H | -H | -H | -H |
| S^{b13},S^{b14},S^{b23} | | | | | | |
| S^{b11} or S^{b21} | -H | -CH₃ | -H | -CH₃ | -H | -H |
| S^{b12} | -H | -propyl-n | -H | -propyl-n | -H | -H |

### [B7 to B9]

With respect to general formulae (B7) to (B9), combinations of Z^{b11}, Z^{b21}, Z^{b111}, Z^{b221}, S^{b11} to S^{b14}, S^{b21}, S^{b111} to S^{b114}, S^{b211}, S^{b223}, T^{b11} to T^{b12}, T^{b21} to T^{b22}, T^{b111} to T^{b112}, T^{b221} to T^{b222}, U^{b11}, and U^{b21} are shown in Table 5.

**[Table 5]**

| | No.107 | No.108 | No.109 | No.110 | No.111 | No.112 | No.113 |
|---|---|---|---|---|---|---|---|
| Z^{b11} | Covalent bond | Covalent bond | Covalent bond | Covalent bond | Covalent bond | covalent bond | Covalent bond |
| Z^{b21} | | | | | | | |
| Z^{b111}, Z^{b221} | | | | | | | |
| T^{b11}∼T^{b12} | | | | | | | |
| T^{b111} ∼T^{b112} | | | | | | | |
| T^{b21}∼T^{b22} | -H | -H | -H | -H | -H | -H | -H |
| T^{b221}∼T^{b222} | | | | | | | |
| S^{b13},S^{b14} | | | | | | | |
| S^{b113}, S^{b114}, S^{b223} | | | | | | | |
| U^{b11},U^{b21} | 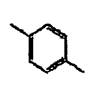 | 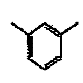 | 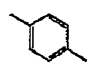 | 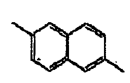 | 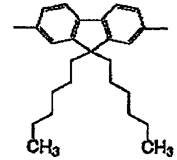 | 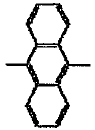 | 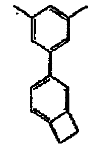 |
| S^{b11}, S^{b21} | -H | -H | -CH₃ | -H | -H | -H | -H |
| S^{b12} | -H | -H | -propyl-n | -H | -H | -H | -H |
| S^{b111}, S^{b221} | -H | -H | -CH₃ | -H | -H | -H | -H |
| S^{b112} | -H | -H | -propyl-n | -H | -H | -H | -H |

Especially preferred examples of the repeating units among those are shown below some of which fall under the scope of the claims.

### [Repeating Units X]

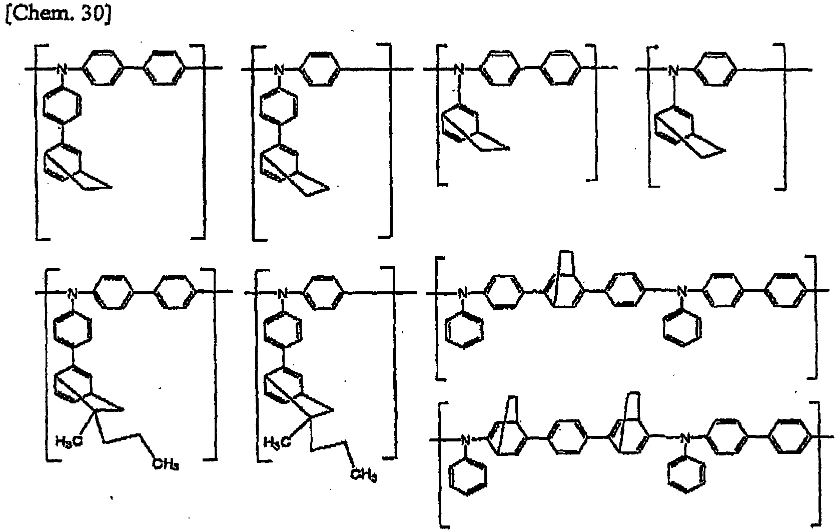

One kind only of repeating units selected from those repeating units may be contained in the polymer, or two or more kinds of repeating units may be contained in the polymer in any desired proportion and combination. Repeating units other than those repeating units may also be contained.

In the invention, the content of the repeating units shown above under [Repeating Units X] in the polymer chain is preferably 10% by mole or higher, more preferably 20% by mole or higher, even more preferably 40% by mole or higher.

So long as the content thereof is within that range, the polymer has satisfactory solubility in organic solvents.

Examples of other repeating units which may be contained in the polymer of the invention are shown below.

With respect to Structural Formulae B, divalent groups derived from Structural Formulae B are contained in the polymer.

### <Repeating Units A>

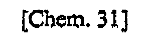

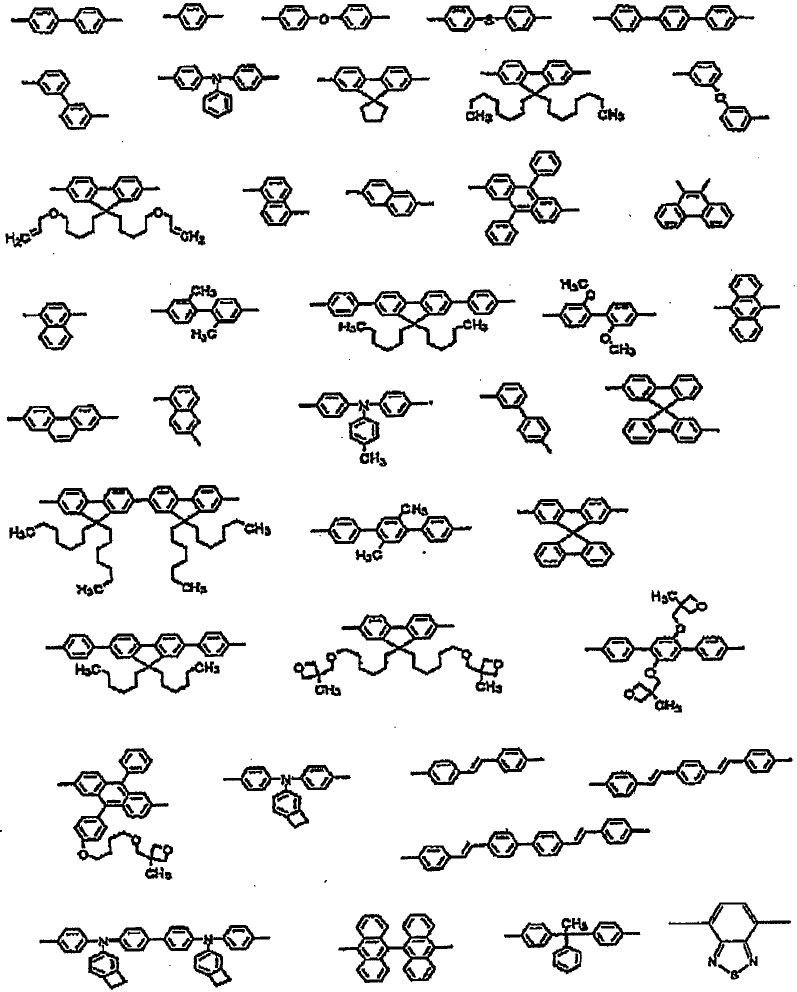

### <Structural Formulae B>

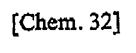

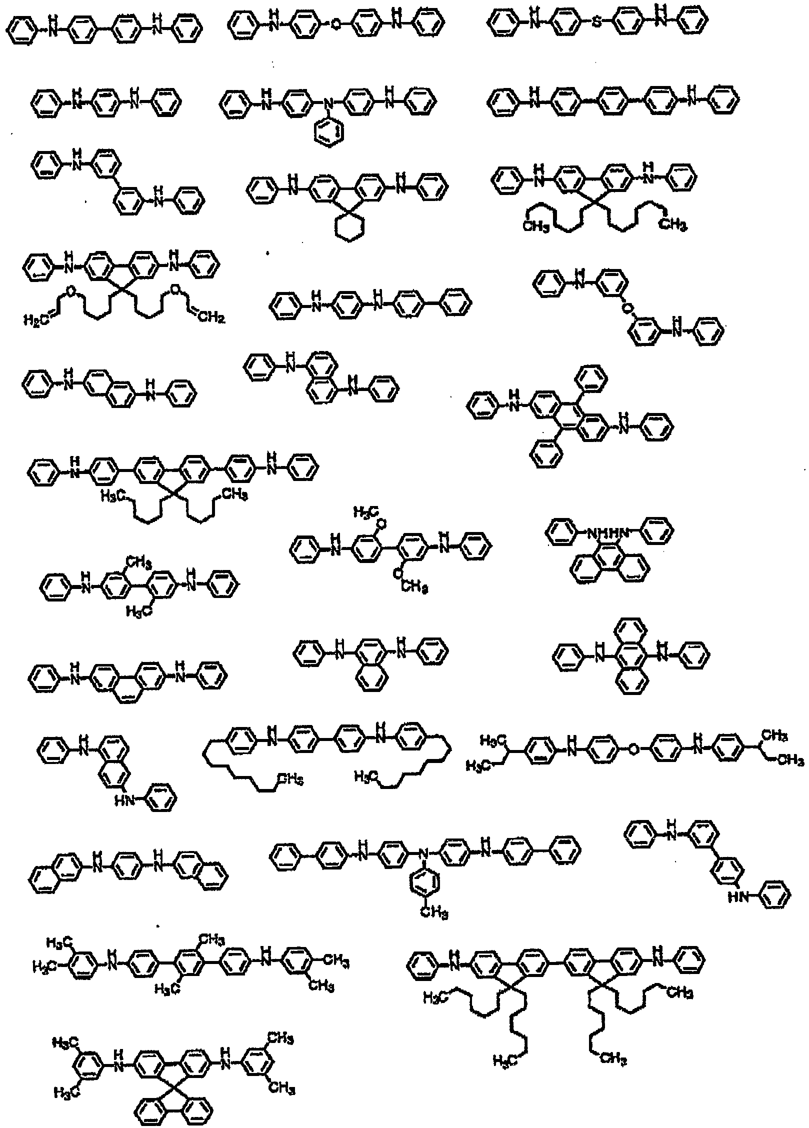

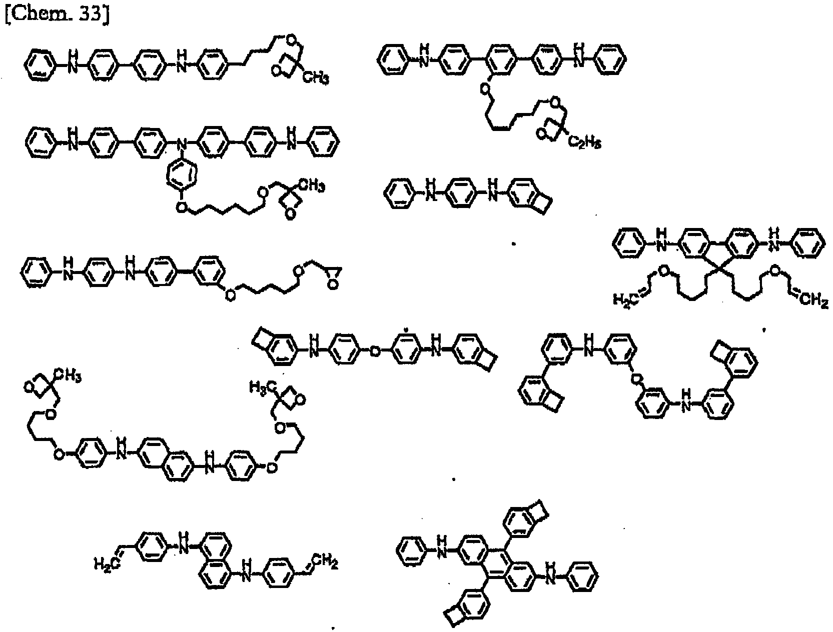

### <Methods for Synthesizing the Polymer>

Methods for synthesizing the polymer of the invention are not particularly limited, and may be suitably selected according to the structure of the polymer, etc.

For example, one or more halides represented by general formula (IIIa) alone are subjected to successive polymerization through a reaction in which Ar-Ar bonds are formed, as shown by the following reaction scheme. Thus, a polymer of the invention usable as a material for organic electroluminescent elements can be obtained. The reaction is conducted usually in the presence of a transition metal catalyst, e.g., copper or a palladium or nickel complex.

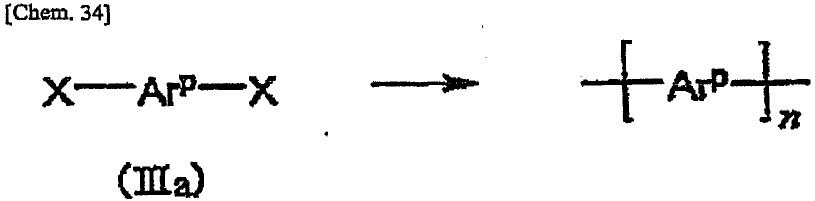

(In the reaction scheme, X represents a halogen atom or a sulfonic ester group, e.g., a CF₃SO₂O- group, and Ar^{p} represents a divalent aromatic hydrocarbon ring group or aromatic heterocyclic group which may have a substituent having a elimination group.)

A polymer of the invention is obtained also by subjecting a halide represented by general formula (IIIa) and a secondary amine compound represented by general formula (IIIb) to successive polymerization through a reaction in which N-Ar bonds are formed (for example, Buchwald-Hartwig coupling, Ullmann coupling, etc.), for example, as shown by the following reaction scheme.

The reaction in which N-Ar bonds are formed is usually conducted in the presence of a base, e.g., potassium carbonate, tert-butoxysodium, or triethylamine. According to need, the reaction may be conducted in the presence of a transition metal catalyst, e.g., copper or a palladium complex.

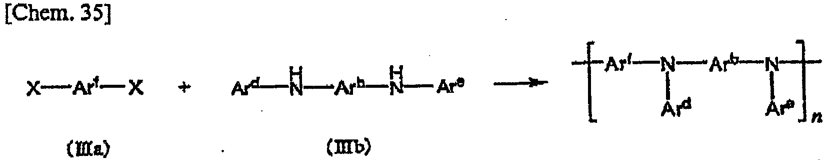

(In the reaction scheme, X represents a halogen atom or a sulfonic ester group, e.g., a CF₃SO₂O- group, and Ar^{d} and Ar^{e} each independently represent an aromatic hydrocarbon ring group which may have a substituent or an aromatic heterocyclic group which may have a substituent. Ar^{f} and Ar^{b} each independently represent a divalent aromatic hydrocarbon ring group which may have a substituent or a divalent aromatic heterocyclic group which may have a substituent. At least one of Ar^{f} to Ar^{c} is a group containing a elimination group.)

Examples of the divalent aromatic hydrocarbon ring groups which may have a substituent or the divalent aromatic heterocyclic groups which may have a substituent, these groups being represented by Ar^{f} and Ar^{b}, include the divalent aromatic hydrocarbon ring groups or divalent aromatic heterocyclic groups shown above as examples of the Ar^{a1} and Ar^{a2} contained in formula (X). With respect to the substituents thereof also, examples thereof include the substituents shown above as substituents of the Ar^{a1} and Ar^{a2} contained in formula (X).

Furthermore, a polymer of the invention is obtained also by subjecting, for example, a halide represented by general formula (IIIa) and a boron compound represented by general formula (IIIc) to successive polymerization through a reaction in which Ar-Ar bonds are formed (for example, Suzuki coupling, etc.). The reaction in which Ar-Ar bonds are formed is usually conducted in the presence of a base, e.g., potassium carbonate, tert-butoxysodium, or triethylamine. According to need, the reaction may be conducted in the presence of a transition metal catalyst, e.g., copper or a palladium complex.

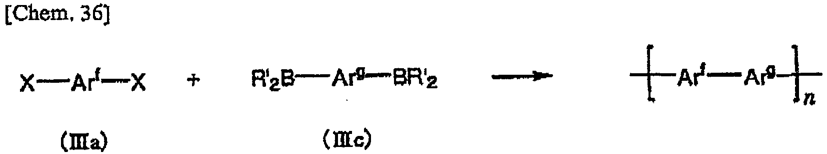

(In the reaction scheme, X represents a halogen atom or a sulfonic ester group, e.g., a CF₃SO₂O- group, and R' represents a hydroxy group or the R's represent alkoxy groups which may have been bonded to each other to form a ring. Ar^{f} and Ar^{g} each independently represent a divalent aromatic hydrocarbon ring group which may have a substituent or a divalent aromatic heterocyclic group which may have a substituent. At least Ar^{f} or Ar^{g} is a group containing a elimination group.)

In the reaction scheme shown above, Ar^{f} and Ar^{g} each represent an aromatic hydrocarbon ring group which may have a substituent or an aromatic heterocyclic group which may have a substituent.

The aromatic hydrocarbon ring group or the aromatic heterocyclic group is the same as in the Ar¹¹ to Ar¹⁵ contained in general formula (IV).

At least Ar^{f} or Ar^{g} is a group containing a elimination group. More specifically, at least Ar^{f} or Ar^{g} may be any group which contains a group represented by general formula (III).

Besides being produced by the polymerization methods described above, the polymer of the invention can be synthesized by known methods. For example, the polymer can be synthesized by the polymerization method described in JP-A-2001-223084, the polymerization method described in JP-A-2003-213002, or the polymerization method described in JP-A-2004-2740, or by the radical polymerization of compounds having an unsaturated double bond or successive polymerization based on a reaction in which ester bonds or amide bonds are formed.

As methods for purifying the polymer of the invention after synthesis thereof, known techniques can be used. Examples thereof include the methods described in Bunri Seisei Gijutsu Handobukku (1993, The Chemical Society of Japan, ed.), Kagaku Henkan Ho Niyoru Biryoseibun Oyobi Nan-Seisei Busshitsu No Kodo-Bunri (1988, published by Industrial Publishing & Consulting, Inc.), or Jikken Kagaku Koza (4th edition) 1, section Bunri To Seisei (1990, The Chemical Society of Japan, ed.). Specific examples thereof include extraction (including suspension washing, washing with boiling, ultrasonic cleaning, and acid/base cleaning), adsorption, occlusion, fusion, crystallization (including recrystallization or reprecipitation from solvent), distillation (atmospheric distillation, vacuum distillation), vaporization, sublimation (atmospheric sublimation, vacuum sublimation), ion exchange, dialysis, filtration, ultrafiltration, reverse osmosis, pressure osmosis, zone melting, electrophoresis, centrifugal separation, floatation, sedimentation, magnetic separation, and various chromatographic techniques (Sorting by shape; column, paper, thin film, capillary: sorting by mobile phase; gas, liquid, micelles, supercritical fluid. Separation mechanism; adsorption, distribution, ion exchange, molecular sieve, chelate, gel filtration, exclusion, affinity).

As methods for ascertaining products or determining the purity thereof, the following techniques can, for example, be applied according to need: a gas chromatograph (GC), high-performance liquid chromatograph (HPLC), high-speed amino acid analyzer (organic compounds), capillary electrophoresis (CE), size exclusion chromatograph (SEC), gel permeation chromatograph (GPC), cross fraction chromatograph (CFC), mass spectrometry (MS, LC/MS, GC/MS, MS/MS), nuclear magnetic resonance apparatus (NMR (1HNMR, 13CNMR)), Fourier transform infrared spectrophotometer (FT-IR), spectrophotometer for ultraviolet, visible, and near infrared regions (UV, VIS, NIR), electron spin resonance apparatus (ESR), transmission electron microscope (TEM-EDX), electron probe microanalyzer (EPMA), metallic-element analysis (ion chromatograph, inductively coupled plasma emission spectrometry (ICP-AES), atomic absorption spectrophotometry (AAS), fluorescent X-ray spectrometer (XRF)), non-metallic-element analysis, trace analysis (ICP-MS, GF-AAS, GD-MS), and the like.

### <Applications>

The organic compounds of the invention have excellent solubility in organic solvents because the organic compounds have one or more elimination groups. Consequently, the organic compounds can be evenly applied. The elimination groups can be eliminated at a relatively low temperature to reduce the solubility in organic solvents. Namely, a heat treatment after film formation can be conducted at a low temperature and, hence, the heating exerts little influence on the other layers, the substrate, etc. In addition, it becomes easy to form other layers by a wet film formation method on the organic layer formed by insolubilizing the organic compounds of the invention.

Furthermore, a residue of the elimination groups (eliminated elimination groups) is less apt to remain in the layer and is hence less apt to affect electrical properties, etc.

Consequently, by employing a structure having any of various functions including charge-transporting properties and luminescent properties, for example, as the Ar^{b} contained in general formula (V), as the Ar^{a} contained in general formula (III), or as a polymer having general formula (III) bonded thereto, it is possible to obtain a compound which has that function and can be formed into a film by a wet process.

Therefore, the organic compounds of the invention are useful as materials for forming electronic devices, i.e., as electronic-device materials.

Examples of the electronic devices include colored resists for color filters, transparent resists for color filters, e.g., ribs, overcoats, and photospacers, organic materials for electronic devices, such as organic insulating films, organic TFT materials, organic solar cell elements, and organic solar cells, materials for electrophotographic photoreceptors, and the whole organic devices including these. By using the polymer of the invention, these devices can be made to have excellent electrical stability, insulating properties, etc.

In particular, since the ethylene molecules or acetylene molecules which were eliminated by a heat treatment have a low molecular weight and are easily removed from the layer, the ethylene or acetylene does not cause a decrease in the electrical properties of the element obtained. Furthermore, since the insolubilization treatment can be conducted at a low temperature, outgassing can be inhibited. In view of these advantages, it is preferred that the electronic-device materials should be used for organic electroluminescent elements or organic solar cell elements.

It is, therefore, preferred that the organic compounds of the invention should be used as materials for organic electroluminescent elements or as materials for organic solar cell elements.

Incidentally, the organic compounds of the invention can be used, for example, as resist materials or the like. In the case where the organic compounds are to be used as resist materials, it is preferred that a carboxy group, a negative-acting photosensitive group, e.g., an acrylate group or a vinyl ether group, or a positive-acting photosensitive group, e.g., a carbonic ester group, naphthoquinonediamide group, acetal group, or o-nitrobenzene group, should be introduced into the molecule in order to impart solubility in alkaline developing solutions or photosensitivity.

Furthermore, the organic compounds of the invention are useful also as binder resins, surfactants, or the like for regulating viscosity or leveling properties in order to improve film-forming properties.

In the case where the compound of the invention is to be used as a material for organic electroluminescent elements, it is most preferred to use the compound as a luminescent-layer material for the following reasons.

In the luminescent layer of an organic electroluminescent element, there is a high possibility that impurities present therein in a slight amount might serve as a quencher or disturb a charge balance to thereby reduce the luminescent efficiency. It is therefore necessary that luminescent-layer materials should be highly purified as compared with materials for the other layers.

It is preferred that the organic compounds of the invention should be the compound described above (i.e., the compound represented by general formula (V)) from the standpoint that this compound has a single molecular weight, can hence be highly purified with ease, and has no end group.

Meanwhile, most luminescent materials having a high luminescent efficiency usually have high symmetry. There frequently are cases where such luminescent materials hence have low solubility in organic solvents and are difficult to purify to a high degree.

In contrast, the organic compounds of the invention can have enhanced solubility in organic solvents and can be highly purified. It is therefore possible to form a luminescent material having a high luminescent efficiency into a film by a wet film formation method.

### <Composition for Electron Devices>

The composition for electronic devices of the invention includes either of the organic compounds of the invention and a solvent, and is suitable for electronic devices. It is preferred to use the composition especially for organic electroluminescent elements or organic solar cell elements, among electronic devices.

Namely, it is preferred that the composition for electronic devices of the invention should be a composition for organic electroluminescent elements or a composition for organic solar cell elements.

The composition for organic electroluminescent elements and the composition for organic solar cell elements are explained below in detail.

### <Composition for Organic Electroluminescent Elements>

The composition for organic electroluminescent elements of the invention includes an organic compound of the invention and at least a solvent.

The composition for organic electroluminescent elements of the invention is suitable for use in producing an organic electroluminescent element including an anode, a cathode, and an organic layer interposed therebetween, for forming the organic layer from the composition by a wet film formation method.

An explanation is given separately on the case where the organic compound of the invention is the compound of the invention (hereinafter referred to as "composition A for organic electroluminescent elements of the invention") and the case where the organic compound of the invention is the polymer of the invention (hereinafter referred to as "composition B for organic electroluminescent elements of the invention").

Incidentally, the expression "composition for organic electroluminescent elements of the invention" means both "composition A for organic electroluminescent elements (of the invention)" and "composition B for organic electroluminescent elements (of the invention)".

### (Composition A for Organic Electroluminescent Elements)

The composition A for organic electroluminescent elements of the invention includes at least the compound of the invention and a solvent.

Incidentally, the composition A for organic electroluminescent elements may be a composition containing only one compound of the invention, or may be a composition containing two or more compounds of the invention in any desired proportion and combination. A mixture with the polymer of the invention may also be used.

The composition A for organic electroluminescent elements of the invention is suitable for use in producing an organic electroluminescent element including an anode, a cathode, and an organic layer interposed therebetween, for forming the organic layer from the composition by a wet film formation method.

Since luminescent-layer materials, in particular, luminescent materials (dopants), are required to have a highest purity, it is preferred that the organic layer formed from the composition A for organic electroluminescent elements of the invention by a wet film formation method should be a luminescent layer.

Namely, it is preferred that the composition A for organic electroluminescent elements should be a composition for luminescent-layer formation.

The content of the compound of the invention in the composition A for organic electroluminescent elements is generally 0.5% by weight or higher, preferably 1% by weight or higher, and is generally 50% by weight or lower, preferably 10% by weight or lower.

The solvent to be contained in the composition A for organic electroluminescent elements is a liquid ingredient having volatility which is used in order to form a luminescent-material-containing layer by a wet process.

The solvent is not particularly limited so long as the compound of the invention and the other solutes satisfactorily dissolve therein. However, examples thereof include the following.

Examples of the solvent include alkanes such as n-decane, cyclohexane, ethylcyclohexane, decalin, and bicyclohexane; aromatic hydrocarbons such as toluene, xylene, mesitylene, cyclohexylbenzene, and tetralin; halogenated aromatic hydrocarbons such as chlorobenzene, dichlorobenzene, and trichlorobenzene; aromatic ethers such as 1,2-dimethoxybenzene, 1,3-dimethoxybenzene, anisole, phenetole, 2-methoxytoluene, 3-methoxytoluene, 4-methoxytoluene, 2,3-dimethylanisole, 2,4-dimethylanisole, and diphenyl ether; aromatic esters such as phenyl acetate, phenyl propionate, methyl benzoate, ethyl benzoate, ethyl benzoate, propyl benzoate, and n-butyl benzoate; alicyclic ketones such as cyclohexanone, cyclooctanone, and fenchone; alicyclic alcohols such as cyclohexanol and cyclooctanol; aliphatic ketones such as methyl ethyl ketone and dibutyl ketone; aliphatic alcohols such as butanol and hexanol; and aliphatic ethers such as ethylene glycol dimethyl ether, ethylene glycol diethyl ether, and propylene glycol 1-monomethyl ether acetate (PGMEA).

Preferred of these are alkanes and aromatic hydrocarbons. One of these solvents may be used alone, or two or more thereof may be used in any desired combination and proportion.

From the standpoint of obtaining a more even film, it is preferred that the solvent should vaporize at an appropriate rate from the liquid film immediately after formation of the liquid film. In order for the solvent to satisfy this, the boiling point of the solvent is generally 80°C or higher, preferably 100°C or higher, and is generally 250°C or lower, preferably 220°C or lower, more preferably 200°C or lower.

The amount of the solvent to be used, based on the amount of the composition A for organic electroluminescent elements which is taken as 100% by weight, is preferably 10% by weight or more, more preferably 50% by weight or more, especially preferably 80% by weight or more, and is preferably 99.95% by weight or less, more preferably 99.9% by weight or less, especially preferably 99.8% by weight or less. In case where the content thereof is less than the lower limit, there is a possibility that the composition might have too high viscosity and have reduced applicability in film formation. On the other hand, in case where the content thereof exceeds the upper limit, the film obtained by applying the composition and then removing the solvent therefrom cannot have a desired thickness and there is hence a tendency that film formation is difficult.

In the case where the composition A for organic electroluminescent elements of the invention is used as a composition for luminescent-layer formation, this composition can contain a luminescent material and a charge-transporting compound besides the compound of the invention and the solvent.

In this case, the compound of the invention may be a luminescent material or a charge-transporting compound or may be both.

As the charge-transporting compound which is not the compound of the invention, use can be made of a charge-transporting compound which has hitherto been used as a material for organic electroluminescent elements. Examples thereof include naphthalene, perylene, anthracene, pyrene, triphenylene, chrysene, naphthacene, phenanthrene, coronene, fluoranthene, benzophenanthrene, fluorene, acetonaphthofluoranthene, coumarin, p-bis(2-phenylethenyl)benzene, derivatives of these, quinacridone derivatives, DCM (4-(dicyanomethylene)-2-methyl-6-(p-dimethylaminostyryl)-4H-pyran) type compounds, benzopyran derivatives, Rhodamine derivatives, benzothioxanthene derivatives, azabenzothioxanthene, fused aromatic ring compounds substituted with an arylamino group, and styryl derivatives substituted with an arylamino group.

The content of the other charge-transporting compound, based on the amount of the composition A for organic electroluminescent elements of the invention which is taken as 100% by weight, is generally 1% by weight or higher, and is generally 50% by weight or lower, preferably 30% by weight or lower.

According to need, the composition A for organic electroluminescent elements may further contain compounds other than the compounds, etc. shown above. For example, besides the solvents shown above, other solvents may be contained. Examples of such solvents include amides, such as N,N-dimethylformamide and N,N-dimethylacetamide, and dimethyl sulfoxide. One of such solvents may be used alone, or two or more thereof may be used in any desired combination and proportion.

Furthermore, the composition A may contain various additives such as a leveling agent and an antifoamer so as to have improved film-forming properties.

### (Composition B for Organic Electroluminescent Elements)

The composition B for organic electroluminescent elements of the invention includes at least the polymer of the invention and a solvent

The composition B for organic electroluminescent elements of the invention is suitable for use as a coating fluid for forming, by a wet film formation method, the organic layer of an organic electroluminescent element which includes an anode, a cathode, and an organic layer interposed therebetween.

Incidentally, the composition B for organic electroluminescent elements of the invention may be a composition containing only one polymer of the invention, or may be a composition containing two or more polymers of the invention in any desired proportion and combination.

It is especially preferred that the composition B for organic electroluminescent elements of the invention should be used for forming the hole injection layer or hole transport layer of the organic electroluminescent element.

In this description, when the organic electroluminescent element has one layer between the anode and the luminescent layer, this one layer is referred to as "hole transport layer". When the element has two or more layers between the anode and the luminescent layer, then the layer in contact with the anode is referred to as "hole injection layer" and the other layer(s) are inclusively referred to as "hole transport layer". There also are cases where the layers disposed between the anode and the luminescent layer are inclusively referred to as "hole injection/transport layer".

The solvent to be contained in the composition B for organic electroluminescent elements preferably is a solvent in which the polymer of the invention is soluble. The solvent may be one in which the polymer of the invention can dissolve in an amount of generally 0.1 % by weight or more, preferably 0.5% by weight or more, more preferably 1% by weight or more.

The composition B for organic electroluminescent elements of the invention contains the polymer of the invention in an amount which is generally 0.01 % by weight or more, preferably 0.05% by weight or more, more preferably 0.1% by weight or more, and is generally 50% by weight or less, preferably 20% by weight or less, more preferably 10% by weight or less.

The kind of the solvent to be contained in the composition B for organic electroluminescent elements of the invention is not particularly limited. Preferred examples of organic solvents in which the polymer of the invention is soluble include aromatic compounds such as toluene, xylene, mesitylene, and cyclohexylbenzene; halogenated solvents such as 1,2-dichloroethane, chlorobenzene, and o-dichlorobenzene; ether solvents including aliphatic ethers such as ethylene glycol dimethyl ether, ethylene glycol diethyl ether, and propylene glycol 1-monomethyl ether acetate (PGMEA) and aromatic ethers such as 1,2-dimethoxybenzene, 1,3-dimethoxybenzene, anisole, phenetole, 2-methoxytoluene, 3-methoxytoluene, 4-methoxytoluene, 2,3-dimethylanisole, and 2,4-dimethylanisole; aliphatic esters such as ethyl acetate, n-butyl acetate, ethyl lactate, and n-butyl lactate; and ester solvents such as phenyl acetate, phenyl propionate, methyl benzoate, ethyl benzoate, isopropyl benzoate, propyl benzoate, and n-butyl benzoate. One of these solvents may be used alone, or two or more thereof may be used in any desired proportion and combination.

The concentration of the solvent in the composition B for organic electroluminescent elements of the invention is generally 40% by weight or higher, preferably 60% by weight or higher, more preferably 80% by weight or higher.

There is a possibility that moisture might accelerate a decrease in the performance of the organic electroluminescent element, in particular, a decrease in luminance during continuous operation. It is therefore preferred to minimize the content of moisture remaining in the organic layer. Preferred of those solvents are ones which have a water solubility therein at 25°C of 1% by weight or less. More preferred are solvents which have a water solubility therein at 25°C of 0.1% by weight or less. The content of such solvents in the composition B for organic electroluminescent elements is generally preferably 10% by weight or higher, more preferably 30% by weight or higher, even more preferably 50% by weight or higher.

In general, organic electroluminescent elements employ a large number of materials which may deteriorate considerably by the action of moisture, e.g., the cathode. Consequently, the presence of moisture in the composition B for organic electroluminescent elements is undesirable also from the standpoint of preventing deterioration of the element. Examples of methods for reducing the water content of the composition B for organic electroluminescent elements include sealing with nitrogen gas, use of a drying agent, solvent dehydration conducted beforehand, and use of a solvent in which water is poorly soluble. Use of a solvent in which water is poorly soluble is preferred of these methods because the phenomenon in which during a coating step, the coating film of the solution absorbs atmospheric moisture to blush can be prevented in this case.

Preferred examples of the solvent to be contained in the composition B for organic electroluminescent elements of the invention include solvents having a surface tension at 20°C of preferably lower than 40 dyn/cm, more preferably 36 dyn/cm or lower, even more preferably 33 dyn/cm or lower.

In the case where a layer containing the polymer of the invention is to be formed by a wet film formation method, affinity for the underlying layer is important. Since evenness of film quality considerably affects the evenness and stability of luminescence of the organic electroluminescent element, the coating fluid to be applied by a wet film formation method is required to have a low surface tension so as to have higher leveling properties and be capable of forming an even coating film. By using such a solvent, an even layer containing the polymer of the invention can be formed.

Specific examples of such solvents having a low surface tension include the aforementioned aromatic solvents including toluene, xylene, mesitylene, and cyclohexylbenzene, ester solvents including ethyl benzoate, and ether solvents including anisole, and further include trifluoromethoxyanisole, pentafluoromethoxybenzene, 3-(trifluoromethyl)anisole, and ethyl pentafluorobenzoate.

Examples of the solvent to be contained in the composition B for organic electroluminescent elements of the invention include solvents having a vapor pressure at 25°C which is generally 1.3kPa (10 mmHg) or lower, preferably 0.67kPa (5 mmHg) or lower, and is generally 13Pa (0.1 mmHg) or higher. Use of such a solvent makes it possible to prepare a composition which is suitable for use in a process for producing an organic
electroluminescent element by a wet film formation method and which suits for the properties of the polymer of the invention. Specific examples of such solvents include the aforementioned aromatic solvents including toluene, xylene, and mesitylene, ether solvents, and ester solvents. The concentration of these solvents in the composition is generally 10% by weight or higher, preferably 30% by weight or higher, more preferably 50% by weight or higher.

Examples of the solvent to be contained in the composition B for organic electroluminescent elements of the invention further include a mixed solvent composed of a solvent having a vapor pressure at 25°C which is generally 0.27kPa (2mmHg) or higher, preferably 0.4kPa (3 mmHg) or higher, more preferably 0.53kPa (4 mmHg) or higher and is preferably 1.3kPa (10mmHg) or lower and a solvent having a vapor pressure at 25°C of generally lower than 0.27kPa (2 mmHg), preferably 0.13kPa (1 mmHg) or lower, more preferably 70Pa (0.5 mmHg)or lower. By using
such a mixed solvent, a homogeneous layer containing the polymer of the invention and further containing additives such as the electron-accepting compound which will be described later can be formed by a wet film formation method. The concentration of such a mixed solvent in the composition B for organic electroluminescent elements is generally 10% by weight or higher, preferably 30% by weight or higher, more preferably 50% by weight or higher.

In the case where the additives are used in the composition B for organic electroluminescent elements, it is preferred to use a solvent in which both the polymer of the invention and the additives can dissolve each in an amount of generally 0.1% by weight or more, preferably 0.5% by weight or more, more preferably 1% by weight or more.

The composition B for organic electroluminescent elements of the invention may contain an electron-accepting compound. Examples of this compound include the electron-accepting compounds which will be given later in the section Hole Injection Layer under Organic Electroluminescent Element. One or more of these electron-accepting compounds can be used.

Solvents other than the solvents described above can be used. For example, an amide, e.g., N,N-dimethylformamide or N,N-dimethylacetamide, dimethyl sulfoxide, and the like can be used.

Furthermore, the composition B for organic electroluminescent elements of the invention may contain various additives, e.g., applicability improves such as a leveling agent and an antifoamer. In the case where the composition B for organic electroluminescent elements is, for example, a composition for use in forming a luminescent layer, this composition may contain the luminescent material and other ingredients which will be explained later in the section Organic Electroluminescent Element

### (Method for Forming Organic Layer from the Composition for Organic Electroluminescent Elements)

An organic electroluminescent element is usually formed by superposing a large number of organic layers constituted of a polymer. Consequently, evenness of film quality is important.

When an organic layer is to be formed from the composition for organic electroluminescent elements of the invention, film formation is usually conducted by a wet film formation method.

The term "wet film formation method" in the invention means a method in which a film is formed by a wet process, such as, for example, spin coating, dip coating, die coating, bar coating, blade coating, roll coating, spray coating, capillary coating, ink-jet printing, screen printing, gravure printing, or flexographic printing. Preferred of these film formation methods are spin coating, spray coating, and ink-jet printing. This is because these techniques suit with the liquid nature of the composition for organic electroluminescent elements.

In the invention, in the case where another organic layer is to be further formed by a wet film formation method on the organic layer formed from the composition for organic electroluminescent elements of the invention, a heat treatment is usually conducted after the film formation in order to insolubilize the organic layer which has been formed.

Techniques for the heating are not particularly limited. The film formed from the composition for organic electroluminescent elements is heated at a temperature which is generally 100°C or higher, preferably 120°C or higher, and is generally 200°C or lower, preferably 190°C or lower. The heating period is generally 1 minute or longer but preferably 24 hours or shorter. Although methods for heating are not particularly limited, use may be made, for example, of a method in which the multilayer structure having the layer formed is put on a hot plate or heated in an oven. For example, use can be made of conditions under which the multilayer structure is heated on a hot plate at 120°C or higher for 1 minute or longer.

In combination with heating, irradiation with electromagnetic energy such as light may be conducted. Examples of methods for the irradiation with electromagnetic energy include a method in which an ultraviolet, visible, or infrared light source, e.g., an ultrahigh-pressure mercury lamp, high-pressure mercury lamp, halogen lamp, or infrared lamp, is used to directly irradiate the layer and a method in which a mask aligner or conveyor type irradiator that has any of those light sources built therein is used to irradiate the layer. With respect to irradiation with electromagnetic energy other than light, examples of methods therefor include a method in which an apparatus for irradiating with microwaves generated by a magnetron, i.e., the so-called electronic oven, is used for the irradiation.

With respect to irradiation period, it is preferred to set conditions necessary for reducing the solubility of the film. However, the film may be irradiated for a period of generally 0.1 second or longer but preferably 10 hours or shorter.

Heating and irradiation with electromagnetic energy, e.g., light, may be conducted alone or in combination. In the case where heating and the irradiation are conducted in combination, the sequence of performing these is not particularly limited.

It is preferred that heating and irradiation with electromagnetic energy including light should be conducted in a moisture-free atmosphere, e.g., a nitrogen gas atmosphere, in order to reduce the amount of moisture contained in the thus-treated layer and/or the amount of moisture adsorbed on the surface thereof. For the same purpose, in the case where heating and/or irradiation with electromagnetic energy such as light is conducted in combination, it is especially preferred that at least the step just before the formation of an organic luminescent layer should be conducted in a moisture-free atmosphere, e.g., a nitrogen gas atmosphere.

### (Composition for Organic Solar Cell Elements)

The composition for organic solar cell elements of the invention includes the polymer of the invention and a solvent. The composition may contain other ingredients according to need unless the effects of the invention are lessened.

Examples of the other ingredients include the p type semiconductors, e.g., tetrabenzoporphyrin and copper tetrabenzoporphyrin, and the n type semiconductors, e.g., fullerenes and octaazaporphyrin, which will be shown later under <Organic Solar Cell Element>.

### (Method for Forming Organic Layer from the Composition for Organic Solar Cell Elements)

For forming an organic layer using the composition for organic solar cell elements of the invention, the same method as that described above in the section (Method for Forming Organic Layer from the Composition for Organic Electroluminescent Elements) may be used. Preferred modes thereof also are the same.

### <Organic Electroluminescent Element>

The organic electroluminescent element of the invention includes an anode, a cathode, and an organic layer interposed between the anode and the cathode, and is characterized in that the organic layer is a layer formed by a wet film formation method from the composition for organic electroluminescent elements described above. In the invention, it is especially preferred that a hole injection layer, a hole transport layer, or a luminescent layer, among organic layers, should be formed from the composition for organic electroluminescent elements by a wet film formation method.

It is preferred that the layer to be formed from the composition A for organic electroluminescent elements of the invention by a wet film formation method should be a luminescent layer.

Furthermore, it is especially preferred that the layer to be formed from the composition B for organic electroluminescent elements of the invention should be a hole injection layer or a hole transport layer.

### [Configuration of the Organic Electroluminescent Element]

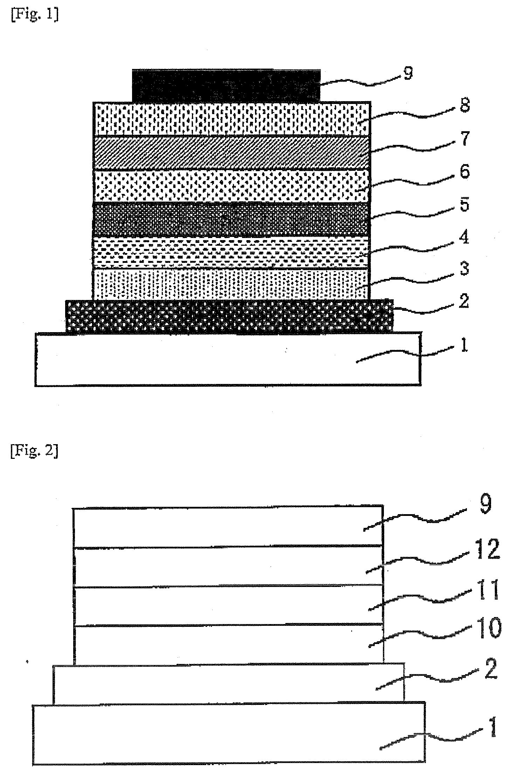

The layer configuration of an organic electroluminescent element of the invention, methods for formation thereof, etc. are explained below by reference to Fig. 1.

Fig. 1 is a diagrammatic sectional view showing an example of the structure of an organic electroluminescent element according to the invention. In Fig. 1, numeral 1 denotes a substrate, 2 an anode, 3 a hole injection layer, 4 a hole transport layer, 5 a luminescent layer, 6 a hole blocking layer, 7 an electron transport layer, 8 an electron injection layer, and 9 a cathode.

### (Substrate)

The substrate 1 serves as the support of the organic electroluminescent element, and use may be made, for example, of a plate of quartz or glass, a metal plate, a metal foil, a plastic film or sheet, or the like. One of these materials may be used alone, or two or more thereof may be used in any desired proportion and combination. Especially preferred of these are a glass plate and a plate of a transparent synthetic resin such as a polyester, polymethacrylate, polycarbonate, or polysulfone. In the case of using a synthetic-resin substrate, it is necessary to take account of gas barrier properties. In case where the substrate 1 has too low gas barrier properties, there are cases where the surrounding air might pass through the substrate 1 to deteriorate the organic electroluminescent element. Too low gas barrier properties are hence undesirable. Consequently, one of preferred methods is to form a dense silicon oxide film on at least one surface of a synthetic-resin substrate to ensure gas barrier properties.

### [Anode]

The anode 2 serves to inject holes into layers located on the luminescent layer 5 side.

This anode 2 is usually constituted of a metal, e.g., aluminum, gold, silver, nickel, palladium, or platinum, a metal oxide, e.g., an indium and/or tin oxide, a metal halide, e.g., copper iodide, carbon black, a conductive polymer, e.g., poly(3-methylthiophene), polypyrrole, or polyaniline, or the like. One of these materials may be used alone, or two or more thereof may be used in any desired proportion and combination.

Usually, the anode 2 is frequently formed by sputtering, vacuum deposition, or the like. In the case where an anode 2 is to be formed using fine particles of a metal, e.g., silver, fine particles of copper iodide or the like, carbon black, fine particles of a conductive metal oxide, fine particles of a conductive polymer, or the like, use may be made of a method in which such fine particles are dispersed in an appropriate binder resin solution and the dispersion is applied to a substrate 1 to form an anode 2. Furthermore, in the case of a conductive polymer, an anode 2 can be formed by directly forming a thin film on a substrate 1 through electrolytic polymerization or by applying the conductive polymer to a substrate 1 *(*Appl. Phys. Lett., Vol.60, p.2711,1992).

The anode 2 usually has a single-layer structure. However, the anode 2 can have a multilayer structure composed of a plurality of materials, according to need.

The thickness of the anode 2 varies depending on the degree of transparency required. When transparency is required, it is preferred that the anode 2 should be regulated so as to have a visible-light transmittance of generally 60% or higher, preferably 80% or higher. In this case, the thickness of the anode 2 is generally 5 nm or more, preferably 10 nm or more, and is generally 1,000 nm or less, preferably about 500 nm or less. When the anode 2 may be opaque, this anode 2 can have any desired thickness and may be identical with the substrate 1. Furthermore, it is possible to superpose a different conductive material on the anode 2.

It is preferred that the surface of the anode 2 should be subjected to an ultraviolet (UV)/ozone treatment or a treatment with an oxygen plasma or argon plasma for the purposes of removing impurities adherent to the anode 2 and regulating ionization potential to improve hole injection properties.

### [Hole Injection Layer]

The hole injection layer 3 is a layer which transports holes from the anode 2 to the luminescent layer 5, and is usually formed on the anode 2.

Methods for forming the hole injection layer 3 according to the invention are not particularly limited, and either a vacuum deposition method or a wet film formation method may be used. However, from the standpoint of diminishing dark spots, it is preferred to form the hole injection layer 3 by a wet film formation method. In this case, it is preferred to use the composition B for organic electroluminescent elements of the invention.

The thickness of the hole injection layer 3 is generally 5 nm or more, preferably 10 nm or more, and is generally 1,000 nm or less, preferably 500 nm or less.

### <Formation of Hole Injection Layer by Wet Film Formation Method>

In the case where a hole injection layer 3 is to be formed by a wet process, the hole injection layer 3 is formed usually by mixing materials for constituting the hole injection layer 3 with an appropriate solvent (solvent for hole injection layer) to prepare a composition for film formation (composition for hole injection layer formation), applying this composition for hole injection layer formation by a suitable technique to the layer (usually, the anode) which is to underlie the hole injection layer 3, and then drying the resultant coating film.

### (Hole-Transporting Compound)

The composition for hole injection layer formation usually contains a hole-transporting compound, as a material for constituting the hole injection layer, and a solvent.

The hole-transporting compound may usually be a high-molecular compound or a low-molecular compound so long as the compound has hole-transporting properties and is for use in the hole injection layers of organic electroluminescent elements. In the invention, the polymer of the invention or the like can, for example, be used as the hole-transporting compound.

From the standpoint of a barrier to charge injection from the anode 2 into the hole injection layer 3, it is preferred that the hole-transporting compound should be a compound having an ionization potential of 4.5 eV to 6.0 eV. Other examples of the hole-transporting compound include aromatic amine compounds, phthalocyanine derivatives, porphyrin derivatives, oligothiophene derivatives, polythiophene derivatives, benzylphenyl compounds, a compound including tertiary amines linked with a fluorene group, hydrazone compounds, silazane compounds, silanamine derivatives, phosphamine derivatives, and quinacridone compounds.

Any one of such hole-transporting compounds may be contained alone as a material for the hole injection layer 3, or two or more thereof may be contained as the material. In the case where two or more hole-transporting compounds are contained, any desired combination of such compounds may be used, However, it is preferred to use one or more aromatic tertiary amine high-molecular compounds in combination with one or more other hole-transporting compounds.

Of the compounds shown above as examples, aromatic amine compounds are preferred from the standpoints of noncrystallinity and visible-light transmittance. In particular, aromatic tertiary amine compounds are preferred. The term aromatic tertiary amine compound means a compound having an aromatic tertiary amine structure, and includes a compound having a group derived from an aromatic tertiary amine.

The kind of aromatic tertiary amine compound is not particularly limited. However, a high-molecular compound (polymeric compound made up of consecutive repeating units) having a weight-average molecular weight of 1,000-1,000,000 is more preferred from the standpoint of even luminescence based on the effect of surface smoothing. Preferred examples of the aromatic tertiary amine high-molecular compound include high-molecular compounds having a repeating unit represented by the following formula (I).

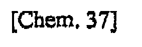

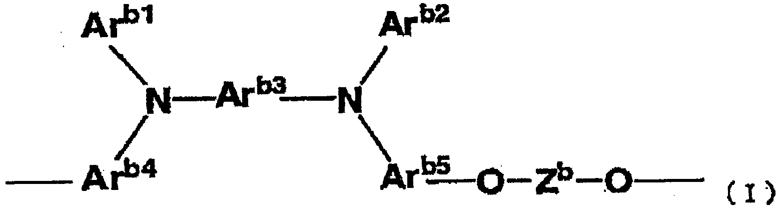

(In formula (I), Ar^{b1} and Ar^{b2} each independently represent an aromatic hydrocarbon ring group which may have a substituent or an aromatic heterocyclic group which may have a substituent. Ar^{b3} to Ar^{b5} each independently represent an aromatic hydrocarbon ring group which may have a substituent or an aromatic heterocyclic group which may have a substituent. Z^{b} represents a linking group selected from the following linking groups. Of Ar^{b1} to Ar^{b5}, two groups bonded to the same nitrogen atom may be bonded to each other to form a ring.)

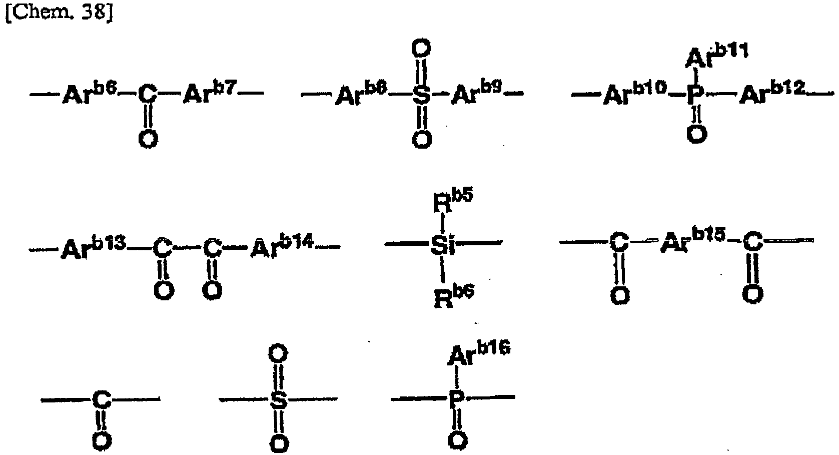

(In the formulae, Ar^{b6} to Ar^{b16} each independently represent a mono- or divalent group derived from an aromatic hydrocarbon ring which may have a substituent or an aromatic heterocycle which may have a substituent. R^{b5} and R^{b6} each independently represent a hydrogen atom or any desired substituent.)

Ar^{b1} to Ar^{b16} each can be a mono- or divalent group derived from any aromatic hydrocarbon ring or aromatic heterocycle. These groups may be the same or different. These groups may further have any substituent

The aromatic hydrocarbon ring groups and aromatic heterocyclic groups represented by Ar^{b1} to Ar^{b16} preferably are groups derived from a benzocyclohexadiene ring, naphthalene ring, phenanthrene ring, thiophene ring, or pyridine ring, from the standpoints of the solubility, heat resistance, and suitability for hole injection and transport of the high-molecular compound. More preferred are groups derived from a benzocyclohexadiene ring or a naphthalene ring.

The aromatic hydrocarbon ring groups and aromatic heterocyclic groups represented by Ar^{b1} to Ar^{b16} may have further substituents. It is preferred that the molecular weights of the substituents should be generally about 400 or lower, in particular, about 250 or lower. Preferred examples of the substituents are alkyl groups, alkenyl groups, alkoxy groups, aromatic hydrocarbon ring groups, aromatic heterocyclic groups, and the like.

In the case where R^{b5} and R^{b6} are any desired substituents, examples of the substituents include alkyl groups, alkenyl groups, alkoxy groups, silyl group, siloxy group, aromatic hydrocarbon ring groups, and aromatic heterocyclic groups.

Specific examples of the aromatic tertiary amine high-molecular compounds having a repeating unit represented by general formula (I) include the compounds described in international Publication No. WO 2005/089024.

The concentration of the hole-transporting compound in the composition for hole injection layer formation is not limited unless the effects of the invention are lessened. However, from the standpoint of the evenness of film thickness, the concentration thereof is generally 0.01% by weight or higher, preferably 0.1% by weight or higher, more preferably 0.5% by weight or higher, and is generally 70% by weight or lower, preferably 60% by weight or lower, more preferably 50% by weight or lower. In case where the concentration thereof is too high, there is a possibility that unevenness of film thickness might result. In case where the concentration thereof is too low, there is a possibility that the resultant hole injection layer might have defects.

### (Electron-Accepting Compound)

It is preferred that the composition for hole injection layer formation should contain an electron-accepting compound as a constituent material for the hole injection layer 3.

The electron-accepting compound preferably is a compound which has oxidizing ability and has the ability to accept one electron from the hole-transporting compound described above. Specifically, compounds having an electron affinity of 4 eV or higher are preferred, and compounds having an electron affinity of 5 eV or higher are more preferred.

Examples of such electron-accepting compounds include one or more compounds selected from the group consisting of triarylboron compounds, metal halides, Lewis acids, organic acids, onium salts, salts of an arylamine with a metal halide, and salts of an arylamine with a Lewis acid. More specifically, examples thereof include onium salts substituted with organic groups, such as 4-isopropyl-4'-methyldiphenyliodonium tetrakis(pent afluorophenyl)borate and triphenylsulfonium tetrafluoroborate (International Publication No. 2005/089024); inorganic compounds having a high valence, such as iron(III) chloride (JP-A-11-251067) and ammonium peroxodisulfate; cyano compounds such as tetracyanoethylene; aromatic boron compounds such as tris(pent afluorophenyl)borane (JP-A-2003-31365); fullerene derivatives; and iodine.

These electron-accepting compounds oxidize the hole-transporting compound and can thereby improve the conductivity of the hole injection layer 3.

In the hole injection layer 3 or in the composition for hole injection layer formation, the content of the electron-accepting compound is generally 0.1% by mole or higher, preferably 1% by mole or higher, based on the hole-transporting compound. However, the content thereof is generally 100% by mole or lower, preferably 40% by mole or lower.

### (Other Constituent Materials)

Besides the hole-transporting compound and electron-accepting compound described above, other ingredients may be incorporated as materials for the hole injection layer 3 unless the incorporation thereof considerably lessens the effects of the invention. Examples of the other ingredients include various luminescent materials, electron-transporting compounds, binder resins, and applicability improvers. One of such other ingredients may be used alone, or two or more thereof may be used in any desired combination and proportion.

### (Solvent)

It is preferred that the solvent contained in the composition for use in hole injection layer formation by a wet film formation method should include at least one compound in which the constituent materials for the hole injection layer described above can dissolve. It is also preferred that the boiling point of the solvent should be generally 80°C or higher, preferably 100°C or higher, and be generally 250°C or lower, in particular 200°C or lower. In case where the boiling point of the solvent is too low, there is a possibility that the composition might dry at too high a rate, resulting in impaired film quality. In case where the boiling point of the solvent is too high, it is necessary to use a higher temperature in the drying step and this may adversely affect other layers or the substrate.

Examples of the solvent include ether solvents, ester solvents, aromatic hydrocarbon solvents, and amide solvents.

Examples of the ether solvents include aliphatic ethers such as ethylene glycol dimethyl ether, ethylene glycol diethyl ether, and propylene glycol 1-monomethyl ether acetate (PGMEA); and aromatic ethers such as 1,2-dimethoxybenzene, 1,3-dimethoxybenzene, anisole, phenetole, 2-methoxytoluene, 3-methoxytoluene, 4-methoxytoluene, 2,3-dimethylanisole, and 2,4-dimethylanisole.

Examples of the ester solvents include aromatic esters such as phenyl acetate, phenyl propionate, methyl benzoate, ethyl benzoate, propyl benzoate, and n-butyl benzoate.

Examples of the aromatic hydrocarbon solvents include toluene, xylene, cyclohexylbenzene, 3-isopropylbiphenyl, 1,2,3,4-tetramethylbenzene, 1,4-diisopropylbenzene, cyclohexylbenzene, and methylnaphthalene. Examples of the amide solvents include N,N-dimethylformamide and N,N-dimethylacetamide.

Furthermore, dimethyl sulfoxide and the like are also usable.

One of these solvents may be used alone, or two or more thereof may be used in any desired combination and proportion.

### (Methods of Film Formation)

After the composition for hole injection layer formation has been prepared, this composition is applied, by a wet process, to the layer (usually, the anode 2) which is to underlie the hole injection layer 3, and the resultant coating film is dried. Thus, a hole injection layer 3 is formed.

The temperature to be used in the film formation step is preferably 10°C or higher and is preferably 50°C or lower, from the standpoint of preventing crystals from generating in the composition and thereby causing film defects.

The relative humidity in the film formation step is not limited unless the effects of the invention are considerably lessened. However, the relative humidity is generally 0.01 ppm or higher and is generally 80% or less.

After the film formation, the film of the composition for hole injection layer formation is dried usually by heating, etc. In particular, when the composition for organic electroluminescent elements of the invention is used for forming a hole injection layer 3, a heating step is usually conducted. Examples of means for heating usable in the heating step include a clean oven, hot plate, infrared rays, halogen lamp heater, and irradiation with microwaves. Of these, a clean oven and a hot plate are preferred from the standpoint of evenly heating the whole film.

With respect to heating temperature in the heating step, it is preferred to heat the film at a temperature not lower than the boiling point of the solvent used in the composition for hole injection layer formation, unless this drying considerably lessens the effects of the invention. In the case where a material of the invention for organic electroluminescent elements is contained in the hole injection layer, it is preferred to heat the film at a temperature not lower than the temperatures at which the elimination group is eliminable. Furthermore, in the case where the solvent used for the composition for hole injection layer formation is a mixed solvent including two or more solvents, it is preferred to heat the film at a temperature not lower than the boiling point of at least one of the solvents. When an increase in the boiling point of solvents is taken into account, it is preferred to heat the film in the heating step at a temperature which is preferably 120°C or higher and is preferably 410°C or lower.

Heating period in the heating step is not limited so long as the heating temperature is not lower than the boiling point of the solvent of the composition for hole injection layer formation and the coating film is sufficiently insolubilized. However, the heating period is preferably 10 seconds or longer, and is generally 180 minutes or shorter. In case where the heating period is too long, components of other layers tend to diffuse. In case where the heating period is too short, the resultant hole injection layer 3 tends to be inhomogeneous. Heating may be conducted two times.

### [Hole Transport Layer]

Methods for forming the hole transport layer 4 according to the invention are not particularly limited, and either a vacuum deposition method or a wet film formation method may be used. However, from the standpoint of diminishing dark spots, it is preferred to form the hole transport layer 4 by a wet film formation method. In this case, it is preferred that the composition B for organic electroluminescent elements of the invention described above should be used to form the layer by a wet process.

In the case where there is a hole injection layer 3, a hole transport layer 4 can be formed on the hole injection layer 3. When there is no hole injection layer 3, then a hole transport layer 4 can be formed on the anode 2. The organic electroluminescent element of the invention may have a configuration in which the hole transport layer 4 has been omitted.

For forming the hole transport layer 4, it is preferred to use a material which has high hole-transporting properties and can efficiently transport injected holes. In order for a material to have such properties, it is preferred that the material should have a low ionization potential, be highly transparent to visible light, and have a high hole mobility and excellent stability, and that impurities functioning as a trap are less apt to generate during production of the material or during use. Furthermore, since the hole transport layer 4 is in contact with the luminescent layer 5 in many cases, it is preferred that the material constituting the hole transport layer 4 should not function to cause extinction of luminescence from the luminescent layer 5 or to form an exciplex with the luminescent layer 5 and thereby reduce efficiency.

As such a material for the hole transport layer 4, use can be made, for example, of the polymer of the invention or materials which have conventionally been used as constituent materials for hole transport layers. Examples of the materials which have conventionally been used include the hole-transporting compounds shown above as examples for use in the hole injection layer 3 described above. Examples thereof further include aromatic diamines which contain two or more tertiary amines and in which the nitrogen atoms have two or more fused aromatic rings bonded thereto as substituents, the aromatic diamines being represented by 4,4'-bis[N-(1-naphthyl)-N-phenylamino]biphenyl (JP-A-5-234681), aromatic amine compounds having a starburst structure, such as 4,4',4"-tris(1-naphthylphenylamino)triphenylamine (J. Lumin., Vol. 72-74, p.985,1997), the aromatic amine compound constituted of the tetramer of triphenylamine (Chem. Commun., p.2175, 1996), spiro compounds such as 2,2',7,7'-tetrakis(diphenylamino)-9,9'-spirobifluorene (Synth. Metals, Vol.91, p.209, 1997), and carbazole derivatives such as 4,4'-N,N'-dicarbazolebiphenyl. Other examples include polyvinylcarbazole, polyvinyltriphenylamine (JP-A-7-53953), and poly(arylene ether sulfone)s containing tetraphenylbenzidine (Polym. Adv. Tech., Vol.7, p.33, 1996).

In the case where a hole transport layer 4 is to be formed by a wet process, a composition for hole transport layer formation is prepared, thereafter formed into a film by a wet process, and then dried by heating, in the same manner as in the formation of the hole injection layer 3.

Besides the hole-transporting compound described above, a solvent is contained in the composition for hole transport layer formation. The solvent to be used may be the same as that used in the composition for hole injection layer formation. Film formation conditions, heating/drying conditions, and the like may also be the same as in the formation of the hole injection layer 3. As the composition for hole transport layer formation, use can be made of the composition for organic electroluminescent elements of the invention.

Also in the case where a hole transport layer 4 is to be formed by vacuum deposition, conditions for the deposition and other conditions may be the same as in the formation of the hole injection layer 3.

The hole transport layer 4 may contain various luminescent materials, electron-transporting compounds, binder resins, applicability improvers, etc., besides the hole-transporting compound.

The hole transport layer 4 may also be a layer formed by crosslinking a crosslinkable compound. The crosslinkable compound is a compound which has a crosslinkable group and forms a polymer through crosslinking.

Examples of the crosslinkable group include cyclic ethers such as oxetane and epoxy; unsaturated double bonds such as vinyl, trifluorovinyl, styryl, acryloyl, methacryloyl, and oinnamoyl; and a benzocyclobutane.

The crosslinkable compound may be any of a monomer, an oligomer, and a polymer. One crosslinkable compound may be contained alone, or two or more crosslinkable compounds may be contained in any desired combination and proportion.

Examples of the crosslinkable group include cyclic ethers such as oxetane and epoxy; unsaturated double bonds such as vinyl, trifluorovinyl, styryl, acryloyl, methacryloyl, and cinnamoyl; and a benzocyclobutane.

As the crosslinkable compound, it is preferred to use a hole-transporting compound having a crosslinkable group. Examples of the hole-transporting compound include nitrogen-containing aromatic compound derivatives such as pyridine derivatives, pyrazine derivatives, pyrimidine derivatives, triazine derivatives, quinoline derivatives, phenanthroline derivatives, carbazole derivatives, phthalocyanine derivatives, and porphyrin derivatives; triphenylamine derivatives; silole derivatives; oligothiophene derivatives; fused-ring aromatic derivatives; and metal complexes. Preferred of these are: nitrogen-containing aromatic derivatives such as pyridine derivatives, pyrazine derivatives, pyrimidine derivatives, triazine derivatives, quinoline derivatives, phenanthroline derivatives, and carbazole derivatives; and triphenylamine derivatives, silole derivatives, fused-ring aromatic derivatives, metal complexes, and the like. In particular, triphenylamine derivatives are more preferred.

For forming a hole transport layer 4 through crosslinking of a crosslinkable compound, use is generally made of a method which includes dissolving or dispersing the crosslinkable compound in a solvent to prepare a composition for hole transport layer formation, forming this composition into a film by a wet process, and crosslinking the crosslinkable compound.

The composition for hole transport layer formation may contain an additive which accelerates the crosslinking reaction, besides the crosslinkable compound. Examples of the additive which accelerates the crosslinking reaction include polymerization initiators and polymerization accelerators, such as alkylphenone compounds, acylphosphine oxide compounds, metallocene compounds, oxime ester compounds, azo compounds, and onium salts; and photosensitizers such as fused-ring hydrocarbons, porphyrin compounds, and diaryl ketone compounds.

The composition may further contain an applicability improver such as a leveling agent or an antifoamer, an electron-accepting compound, a binder resin, and the like.

The amount of the crosslinkable compound contained in the composition for hole transport layer formation is generally 0.01% by weight or more, preferably 0.05% by weight or more, more preferably 0.1% by weight or more, and is generally 50% by weight or less, preferably 20% by weight or less, more preferably 10% by weight or less.

The composition for hole transport layer formation which contains a crosslinkable compound in such a concentration is applied to the layer to be an underlying layer (usually, the hole injection layer 3) to form a film. Thereafter, the crosslinkable compound is crosslinked by means of heating and/or irradiation with electromagnetic energy, such as light, and thereby converted into a polymer.

Conditions including temperature and humidity for the film formation may be the same as in the wet formation of the hole injection layer 3.

Techniques for heating to be conducted after film formation are not particularly limited. Examples thereof include drying by heating and vacuum drying. In the case of drying by heating, heating temperature conditions include a temperature of generally 120°C or higher but preferably 400°C or lower.

The heating period is generally 1 minute or longer but preferably 24 hours or shorter. Although methods for heating are not particularly limited, use may be made, for example, of a method in which the multilayer structure having the layer formed is put on a hot plate or heated in an oven. For example, use can be made of conditions under which the multilayer structure is heated on a hot plate at 120°C or higher for 1 minute or longer.

In the case of irradiation with electromagnetic energy such as light, examples of methods therefor include a method in which an ultraviolet, visible, or infrared light source, e.g., an ultrahigh-pressure mercury lamp, high-pressure mercury lamp, halogen lamp, or infrared lamp, is used to directly irradiate the layer and a method in which a mask aligner or conveyor type irradiator that has any of those light sources built therein is used to irradiate the layer. With respect to irradiation with electromagnetic energy other than light, examples of methods therefor include a method in which an apparatus for irradiating with microwaves generated by a magnetron, i.e., the so-called electronic oven, is used for the irradiation. With respect to irradiation period, it is preferred to set conditions necessary for reducing the solubility of the film. However, the film may be irradiated for a period of generally 0.1 second or longer but preferably 10 hours or shorter.

Heating and irradiation with electromagnetic energy, e.g., light, may be conducted alone or in combination. In the case where heating and the irradiation are conducted in combination, the sequence of performing these is not particularly limited.

The thickness of the hole transport layer 4 thus formed is generally 5 nm or more, preferably 10 nm or more, and is generally 300 nm or less, preferably 100 nm or less.

### [Luminescent Layer]

A luminescent layer 5 is disposed on the hole injection layer 3, or is disposed on the hole transport layer 4 when the hole transport layer 4 has been disposed. The luminescent layer 5 is a layer which, between the electrodes placed in an electric field, is excited by recombination of holes injected from the anode 2 with electrons injected from the cathode 9 and which thus functions as the main luminescence source.

### <Luminescent-Layer Materials>

The luminescent layer 5 contains at least a material having the property of luminescing (luminescent material) as a constituent material therefor, and preferably further contains a compound having the property of transporting holes (hole-transporting compound) or a compound having the property of transporting electrons (electron-transporting compound) as another constituent material. It is also possible to use a luminescent material as a dopant material and to use a hole-transporting compound, electron-transporting compound, or the like as a host material.

It is preferred that the compound of the invention should be used as those luminescent-layer materials from the standpoint of attaining excellent evenness of the luminescent layer, excellent heat resistance after insolubilization, and driving stability of the element The luminescent layer 5 may further contain other ingredients unless the effects of the invention are considerably lessened. In the case of forming the luminescent layer 5 by a wet film formation method, it is preferred to use one or more materials which each have a low molecular weight.

### (Luminescent Material)

As the luminescent material, any desired known material can be used. For example, the luminescent material may be a fluorescent material or a phosphorescent material. However, a phosphorescent material is preferred from the standpoint of inner-quantum efficiency. The material for organic electroluminescent elements of the invention may be used as the luminescent material. It is also possible to use luminescent materials in combination in such a manner that, for example, a fluorescent material is used for blue and a phosphorescent material is used for green or red.

It is preferred that the symmetry or stiffness of the molecule of a luminescent material should be reduced or an oleophilic substituent, e.g., an alkyl group, should be introduced into the molecule, for the purpose of improving solubility in solvents.

Examples of fluorescent dyes among luminescent materials are shown below, but the fluorescent dyes should not be construed as being to the following examples.

Examples of fluorescent dyes which give blue luminescence (blue fluorescent dyes) include naphthalene, chrysene, perylene, pyrene, anthracene, coumarin, p-bis(2-phenylethenyl)benzene, and derivatives of these.

Examples of fluorescent dyes which give green luminescence (green fluorescent dyes) include quinacridone derivatives, coumarin derivatives, and aluminum complexes such as Al(C₉H₆NO)₃.

Examples of fluorescent dyes which give yellow luminescence (yellow fluorescent dyes) include rubrene and perimidone derivatives.

Examples of fluorescent dyes which give red luminescence (red fluorescent dyes) include DCM
(4-(dicyanomethylene)-2-methyl-6-(p-dimethylaminostyryl)-4H-pyran) type compounds, benzopyran derivatives, Rhodamine derivatives, benzothioxanthene derivatives, and azabenzothioxanthene.

Examples of phosphorescent materials include organometallic complexes containing a metal selected from Groups 7 to 11 of the long-form periodic table (hereinafter, the term "periodic table" means the long-form periodic table unless otherwise indicated).

Preferred examples of the metal selected from Groups 7 to 11 of the periodic table include ruthenium, rhodium, palladium, silver, rhenium, osmium, iridium, platinum, and gold.

Preferred ligands of the complexes are ligands including a (hetero)aryl group and, linked thereto, pyridine, pyrazole, phenanthroline, or the like, such as a heteroarylpyridine ligand and a (hetero)arylpyrazole ligand. Especially preferred are a phenylpyridine ligand and a phenylpyrazole ligand. The term (hereto)aryl herein means an aryl group or a heteroaryl group.

Specific examples of the phosphorescent materials include tris(2-phenylpyridine)iridium, tris(2-phenylpyridine)ruthenium, tris(2-phenylpyridine)palladium, bis(2-phenylpyridine)platinum, tris(2-phenylpyridine)osmium, tris(2-phenylpyridine)rhenium, platinum octaethylporphyrin, platinum octaphenylporphyrin, palladium octaethylporphyrin, and palladium octaphenylporphyrin.

The compound to be used as a luminescent material may have any desired molecular weight unless the effects of the invention are considerably lessened thereby. However, the molecular weight thereof is generally 10,000 or lower, preferably 5,000 or lower, more preferably 4,000 or lower, even more preferably 3,000 or lower, and is generally 100 or higher, preferably 200 or higher, more preferably 300 or higher, even more preferably 400 or higher. When the molecular weight of the luminescent material is too low, there are cases where this material has considerably reduced heat resistance or is causative of gas generation or use of this material in film formation results in reduced film quality. There also are cases where the organic electroluminescent element suffers a morphological change due to migration, etc. On the other hand, in case where the molecular weight of the luminescent material is too high, there is a tendency that the material for organic electroluminescent elements is difficult to purify or dissolution of the material in a solvent necessitates a prolonged time period.

Any one of the luminescent materials described above may be used alone, or two or more thereof may be used in any desired combination and proportion.

The content of the luminescent material in the luminescent layer 5 is not limited unless the effects of the invention are considerably lessened. However, the content thereof is generally 0.05% by weight or higher, and is generally 35% by weight or lower. In case where the content thereof is too low, there is the possibility of resulting in luminescence unevenness. In case where the content thereof is too high, there is the possibility of resulting in a decrease in luminescent efficiency. In the case where two or more luminescent materials are used in combination, the total content of these materials is regulated so as to be within that range.

### (Hole-Transporting Compound)

The luminescent layer 5 may contain a hole-transporting compound as a constituent material therefor. Examples of low-molecular hole-transporting compounds among bole-transporting compounds include the compound of the invention described above and the low-molecular hole-transporting compounds for use in the hole injection layer 3. Also usable besides these are compounds such as aromatic diamines which contain two or more tertiary amines and in which the nitrogen atoms have two or more fused aromatic rings bonded thereto as substituents, the aromatic diamines being represented by 4,4'-bis[N-(1-naphthyl)-N-phenylamino]biphenyl (JP-A-5-234681), aromatic amine compounds having a starburst structure, e.g., 4,4',4"-tris(1-naphthylphenylamino)triphenylamine *(*Journal of Luminescence, Vol.72-74, p.985, 1997), the aromatic amine compound constituted of the tetramer of triphenylamine *(*Chemical Communications, p.2175, 1996), and spiro compounds, e.g., 2,2'7,7'-tetrakis(diphenylamino)-9,9'-spirobifluorene *(*Synthetic Metals, Vol.91, p.209, 1997).

In the luminescent layer 5, only one hole-transporting compound may be used alone, or two or more hole-transporting compounds may be used in any desired combination and proportion.

The content of the hole-transporting compound in the luminescent layer 5 is not limited unless the effects of the invention are considerably lessened. However, the content thereof is generally 0.1% by weight or higher, and is generally 65% by weight or lower. In case where the content of the hole-transporting compound is too low, there is a possibility that the clement might be more susceptible to short-circuiting. In case where the content thereof is too high, there is the possibility of resulting in film thickness unevenness. In the case where two or more hole-transporting compounds are used in combination, the total content of these materials is regulated so as to be within that range.

### (Electron-Transporting Compound)

The luminescent layer 5 may contain an electron-transporting compound as a constituent material therefor. Examples of low-molecular electron-transporting compounds among electron-transporting compounds include the compound of the invention, 2,5-bis(1-naphthyl)-1,3,4-oxadiazole (BND), 2,5-bis(6'-(2',2"-bipyridyl))-1,1-dimethy1-3,4-diphenylsilole(PyPySPyPy), bathophenanthroline (BPhen), 2,9-dimethyl-4,7-diphenyl-1,10-phenanthroline (BCP; bathocuproine), 2-(4-biphenylyl)-5-(p-tert-butylphenyl)-1,3,4-oxadiazole (tBu-PBD), or 4,4'-bis(9-carbazole)biphenyl (CBP). In the luminescent layer 5, only one electron-transporting compound may be used alone, or two or more electron-transporting compounds may be used in any desired combination and proportion.

The content of the electron-transporting compound in the luminescent layer 5 is not limited unless the effects of the invention are considerably lessened. However, the content thereof is generally 0.1% by weight or higher, and is generally 65% by weight or lower. In case where the content of the electron-transporting compound is too low, there is a possibility that the element might be more susceptible to short-circuiting. In case where the content thereof is too high, there is the possibility of resulting in film thickness unevenness. In the case where two or more electron-transporting compounds are used in combination, the total content thereof is regulated so as to be within that range.

### <Formation of Luminescent Layer>

In the case where a luminescent layer 5 is to be formed by a wet film formation method according to the invention, the materials are dissolved in a suitable solvent to prepare a composition for luminescent-layer formation, and this composition is formed into a film. Thus, a luminescent layer is formed. In this case, the composition for organic electroluminescent elements of the invention may be used.

As the luminescent-layer solvent which is to be contained in the composition for luminescent-layer formation, which is for forming a luminescent layer 5 by a wet film formation method according to the invention, any desired solvent can be used so long as a luminescent layer can be formed therewith. Suitable examples of the luminescent-layer solvent arc the same as the solvents explained above with regard to the composition for hole injection layer formation.

The proportion of the luminescent-layer solvent to the composition for luminescent-layer formation, which is for forming a luminescent layer 5, is not limited unless the effects of the invention are considerably lessened. However, the proportion thereof is generally 0.01% by weight or higher, and is generally 70% by weight or lower. In the case where a mixture of two or more solvents is used as the luminescent-layer solvent, the total content of these solvents is regulated so as to be within that range.

The solid concentration of the luminescent material, hole-transporting compound, electron-transporting compound, etc. in the composition for luminescent-layer formation is generally 0.01% by weight or higher, and is generally 70% by weight or lower. In case where the concentration thereof is too high, there is the possibility of resulting in film thickness unevenness. In case where the concentration thereof is too low, there is the possibility of resulting in a film having defects.

After the composition for luminescent-layer formation has been formed into a film by a wet process, the resultant coating film is dried to remove the solvent, thereby forming a luminescent layer. Specifically, the same method as that described above with regard to the formation of the hole injection layer may be used. Modes of the wet film formation method are not limited unless the effects of the invention are considerably lessened, and any of the modes described above can be used.

The luminescent layer 5 has any desired thickness unless the effects of the invention are considerably lessened. However, the thickness thereof is generally 3 nm or more, preferably 5 nm or more, and is generally 200 nm or less, preferably 100 nm or less. In case where the thickness of the luminescent layer 5 is too small, there is a possibility that the film might have defects. In case where the thickness thereof is too large, there is the possibility of resulting in an increase in operating voltage.

### [Hole Blocking Layer]

A hole blocking layer 6 may be disposed between the luminescent layer 5 and the electron injection layer 8 which will be described later. The hole blocking layer 6 is a layer superposed on the luminescent layer 5 so as to be in contact with that interface of the luminescent layer 5 which faces the cathode 9.

This hole blocking layer 6 has the function of blocking holes sent from the anode 2 and preventing the holes from reaching the cathode 9, and further has the function of efficiently transporting, toward the luminescent layer 5, electrons injected from the cathode 9.

Examples of properties which are required of the material constituting the hole blocking layer 6 include a high electron mobility and a low hole mobility, a large energy gap (difference between HOMO and LUMO), and a high excited-triplet level (T1). Examples of materials for the hole blocking layer which satisfy such requirements include the compound and polymer of the invention, metal complexes such as mixed-ligand complexes, e.g., bis(2-methyl-8-quinolinolato)(phenolato)aluminum and bis(2-methyl-8-quinolinolato)(triphenylsilanolato)aluminum, and dinuclear metal complexes such as bis(2-methyl-8-quinolato)aluminum-µ-oxobis(2-methyl-8-quinolilato)aluminum, styryl compounds such as distyrylbiphenyl derivatives (JP-A-11-242996), triazole derivatives such as 3-(4-biphenylyl)-4-phenyl-5-(4-tert-butylphenyl)-1,2,4-triazole (JP-A-7-41759), or phenanthroline derivatives such as bathocuproine (JP-A-10-79297). Furthermore, the compound having at least one pyridine ring substituted in the 2-, 4-, and 6-positions which is described in International Publication No. 2005/022962 is also preferred as a material for the hole blocking layer 6.

One material only may be used for forming the hole blocking layer 6, or two or more materials may be used for forming the layer 6 in any desired combination and proportion.

Methods for forming the hole blocking layer 6 are not limited. Consequently, the hole blocking layer 6 can be formed by a wet film formation method, vapor deposition method, or another method.

The thickness of the hole blocking layer 6 is not limited unless the effects of the invention are considerably lessened. However, the thickness thereof is generally 0.3 nm or more, preferably 0.5 nm or more, and is generally 100 nm or less, preferably 50 nm or less.

### [Electron Transport Layer]

An electron transport layer 7 may be disposed between the luminescent layer 5 and the electron injection layer 8 which will be described later.

The electron transport layer 7 is disposed for the purpose of further improving the luminescent efficiency of the element, and is constituted of one or more compounds which, between the electrodes placed in an electric field, can efficiently transport, toward the luminescent layer 5, electrons injected from the cathode 9.

As electron-transporting compounds for the electron transport layer 7, use is usually made of compounds which attain a high efficiency of electron injection from the cathode 9 or electron injection layer 8 and which have a high electron mobility and can efficiently transport injected electrons. Examples of compounds satisfying such requirements include metal complexes such as the aluminum complex of 8-hydroxyquinoline (JP-A-59-194393), metal complexes of 10-hydroxybenzo[h]quinoline, oxadiazole derivatives, distyrylbiphenyl derivatives, silole derivatives, 3-hydroxyflavone metal complexes, 5-hydroxyflavone metal complexes, benzoxazole metal complexes, benzthiazole metal complexes, trisbenzimidazolylbenzene (U.S. Patent No. 5,645,948), quinoxaline compounds (JP-A-6-207169), phenanthroline derivatives (JP-A-5-331459), 2-t-butyl-9,10-N,N'-dicyanoanthraquinonediimine, n-type amorphous silicon hydride carbide, n-type zinc sulfide, and n-type zinc selenide.

One material only may be used for forming the electron transport layer 7, or two or more materials may be used for forming the layer 7 in any desired combination and proportion.

Methods for forming the electron transport layer 7 are not limited. Consequently, the electron transport layer 7 can be formed by a wet film formation method, vapor deposition method, or another method.

The thickness of the electron transport layer 7 is not limited unless the effects of the invention are considerably lessened. However, the thickness thereof is generally 1 nm or more, preferably 5 nm or more, and is generally 300 nm or less, preferably 100 nm or less.

### [Electron Injection Layer]

The electron injection layer 8 serves to efficiency inject, into the luminescent layer 5, electrons injected from the cathode 9. From the standpoint of efficiently injecting electrons, it is preferred that the material constituting the electron injection layer 8 should be a metal having a low work function. For example, an alkali metal such as sodium or cesium, an alkaline earth metal such as barium or calcium, or the like is used. The thickness thereof is generally 0.1 nm or more and is preferably 5 nm or less.

Furthermore, doping of an organic electron transport compound represented by a nitrogen-containing heterocyclic compound, e.g., bathophenanthroline, or a metal complex, e.g., the aluminum complex of 8-hydroxyquinoline, with an alkali metal such as sodium, potassium, cesium, lithium, or rubidium (described in JP-A-10-270171, JP-A-2002-100478, JP-A-2002-100482, etc.) is preferred because this doping improves suitability for electron injection and transport and enables the layer to combine the improved suitability and excellent film quality. The thickness of the film in this case is generally 5 nm or more, especially preferably 10 nm or more, and is generally 200 nm or less, especially preferably 100 nm or less.

One material only may be used for forming the electron injection layer 8, or two or more materials may be used for forming the layer 8 in any desired combination and proportion.

Methods for forming the electron injection layer 8 are not limited. Consequently, the electron injection layer 8 can be formed by a wet film formation method, vapor deposition method, or another method.

### [Cathode]

The cathode 9 serves to inject electrons into a layer located on the luminescent layer 5 side (e.g., the electron injection layer 8 or the luminescent layer 5).

As the material of the cathode 9, the materials usable for the anode 2 can be used. However, metals having a low work function are preferred from the standpoint of efficiently injecting electrons. For example, suitable metals such as tin, magnesium, indium, calcium, aluminum, and silver and alloys of these are used. Specific examples thereof include electrodes of alloys having a low work function, such as magnesium-silver alloys, magnesium-indium alloys, and aluminum-lithium alloys.

One material only may be used for forming the cathode 9, or two or more materials may be used for forming the cathode 9 in any desired combination and proportion.

The thickness of the cathode 9 is generally the same as that of the anode 2.

For the purpose of protecting the cathode 9 made of a metal having a low work function, a layer of a metal which has a high work function and is stable to the air may be formed on the cathode 9. This layer formation is preferred because the stability of the element is enhanced thereby. For this purpose, a metal such as, for example, aluminum, silver, copper, nickel, chromium, gold, or platinum is used. One of these materials may be used alone, or two or more thereof may be used in any desired combination and proportion.

### [Other Layers]

The organic electroluminescent element according to the invention may have other configurations unless the configurations depart from the spirit of the invention. For example, unless the performance of the element is impaired, the element may have any desired layer other than the layers explained above, between the anode 2 and the cathode 9, or any layer may have been omitted. For example, the organic electroluminescent elements produced in some of the Examples which will be given later have the configuration of the organic electroluminescent element shown in Fig. 1 from which the hole blocking layer 6 and the electron transport layer 7 have been omitted.

### <Electron Blocking Layer>

Examples of the layers which may be possessed by the organic electroluminescent element besides the layers described above include an electron blocking layer.

The electron blocking layer is disposed between the hole injection layer 3 or hole transport layer 4 and the luminescent layer 5. The electron blocking layer serves to block electrons sent from the luminescent layer 5 and prevent the electrodes from reaching the hole injection layer 3. The electron blocking layer thus functions to heighten the probability of recombination of holes with electrons within the luminescent layer 5 and to confine the resultant excitons in the luminescent layer 5. The electron blocking layer further has the function of efficiently transporting, toward the luminescent layer 5, holes injected from the hole injection layer 3. To dispose the electron blocking layer is effective especially when a phosphorescent material or a blue luminescent material is used as a luminescent material.

Examples of properties which are required of the electron blocking layer include high hole-transporting properties, a large energy gap (difference between HOMO and LUMO), and a high excited-triplet level (T1). Furthermore, in the invention, when the luminescent layer 5 is to be formed as an organic layer according to the invention by a wet film formation method, the electron blocking layer also is required to have suitability for the wet film formation. Examples of materials usable for forming such an electron blocking layer include copolymers of dioctylfluorene and triphenylamine which are represented by F8-TFB (International Publication No. WO 2004/084260).

One material only may be used for forming the electron blocking layer, or two or more materials may be used for forming the layer in any desired combination and proportion.

Methods for forming the electron blocking layer are not limited. Consequently, the electron blocking layer can be formed by a wet film formation method, vapor deposition method, or another method.

### <Others>

Furthermore, to interpose an ultrathin insulating film (0.1-5 nm) made of, for example, lithium fluoride (LiF), magnesium fluoride (MgF₂), lithium oxide (Li₂O), or cesium(II) carbonate (CsCO₃) at the interface between the cathode 9 and the luminescent layer 5 or electron transport layer 7 is an effective technique for improving the efficiency of the element (see, for example, Applied Physics Letters, Vol. 70, p.152, 1997; JP-A-10-74586; IEEE Transactions on Electron Devices, Vol.44, p.1245,1997; and *SID 04 Digest,* p.154).

Moreover, in any of the layer configurations explained above, the constituent elements excluding the substrate may be superposed in the reverse order. For example, in the case of the layer configuration shown in Fig. 1, the constituent elements other than the substrate 1 may be disposed on the substrate 1 in the order of: the cathode 9, electron injection layer 8, electron transport layer 7, hole blocking layer 6, luminescent layer 5, hole transport layer 4, hole injection layer 3, and anode 2.

It is also possible to constitute an organic electroluminescent element according to the invention by superposing the constituent elements other than the substrate between two substrates, at least one of which is transparent.

A structure composed of a stack of stages each composed of constituent elements other than substrates (luminescent units) (i.e., a structure composed of a plurality of stacked luminescent units) is also possible. In this case, when a carrier generation layer (CGL) made of, for example, vanadium pentoxide (V₂O₅) is disposed in place of the interfacial layers located between the stages (i.e., between the luminescent units) (when the anode is ITO and the cathode is aluminum, the interfacial layers are these two layers), then the barrier between the stages is reduced. This configuration is more preferred from the standpoints of luminescent efficiency and operating voltage.

Furthermore, the organic electroluminescent element according to the invention may be configured so as to be a single organic electroluminescent element, or may be applied to a configuration in which a plurality of organic electroluminescent elements have been disposed in an array arrangement The organic electroluminescent element may also be applied to a configuration in which anodes and cathodes have been disposed in an X-Y matrix arrangement.

Each of the layers described above may contain ingredients other than those described above as materials for the layer, unless the effects of the invention are considerably lessened thereby.

The organic electroluminescent element of the invention may be used in organic EL displays. The organic electroluminescent element obtained according to the invention can be used to fabricate an organic EL display, for example, by the method described in Yuka EL Dispurei (Ohmsha, Ltd., published on August 20, 2004, written by TOKITO Shizuo, ADACHI Chihaya, and MURATA Hideyuki).

Furthermore, the organic electroluminescent element of the invention is thought to be applied to flat panel displays (e.g., displays for OA computers and wall-mounted TV receivers), vehicle-mounted display elements, cell phone displays, light sources taking advantage of the feature of a surface light emitter (e.g., the light source of a copier and the backlights of a liquid-crystal display and instrument), display panels, and marker lights, and has a high technical value.

### <Organic Solar Cell Element>

The organic solar cell element of the invention is an organic solar cell element which includes an anode, a cathode, and one or more organic layers interposed therebetween, wherein the organic layers include a layer (elimination layer) formed by a wet film formation method from a composition for organic electroluminescent elements which contains the polymer of the invention.

It is preferred that the organic solar cell of the invention should be one which includes a photoelectric conversion layer and a hole extraction layer as organic layers and in which the hole extraction layer is the elimination layer.

Fig. 2 is a diagrammatic sectional view illustrating an example of the structure of an organic solar cell element according to the invention. In Fig. 2, numeral 1 denotes a substrate, 2 an anode, 10 a hole extraction layer, 11 a photoelectric conversion layer, 12 an electron extraction layer, and 9 a cathode.

### <Substrate (A)>

The substrate (A) is a supporting member which supports an organic semiconductor element (B). Examples of materials for forming the substrate (A) include inorganic materials such as glass, sapphire, and titania; organic materials such as poly(ethylene terephthalate), poly(ethylene naphthalate), polyethersulfones, polyimides, nylons, polystyrene, poly(vinyl alcohol), ethylene/vinyl alcohol copolymers, fluororesin films, polyvinyl chloride, polyethylene, polypropylene, cycloolefin polymers, cellulose, acetyl cellulose, poly(vinylidene chloride), aramids, poly(phenylene sulfide), polyurethanes, polycarbonates, poly(meth)acrylic resins, phenol resins, epoxy resins, polyarylates, and polynorbornene; and metallic materials such as stainless steel, titanium, nickel, silver, gold, copper, and aluminum.

Preferred of these, from the standpoint of ease of forming the organic semiconductor element (B), are glass, poly(ethylene terephtalate), poly(ethylene naphthalate), polyimides, poly(meth)acrylic resin films, stainless steel, and aluminum.

One material for the substrate may be used alone, or two or more materials for the substrate may be used in any desired combination and proportion. Reinforcing fibers such as carbon fibers or glass fibers may be incorporated into those organic materials to enhance the mechanical strength thereof. Also usable is a composite material such as, for example, a composite material obtained by subjecting the surface of any of those metallic materials to coating or laminating in order to impart insulating properties thereto.

### <Organic Semiconductor Element (B)>

The organic semiconductor element (B) will be described below with respect to an organic-thin-film organic solar cell element (in this description, this solar cell element is also referred to simply as "organic solar cell element"). However, the organic semiconductor element (B) should not be construed as excluding other organic electron devices, unless this considerably impairs the invention.

The organic-thin-film organic solar cell element is configured at least of an organic semiconductor layer including an organic semiconductor disposed between a pair of electrodes. The element has been configured so that the organic semiconductor layer absorbs light to generate electric power and the generated electric power is led out through the electrodes.

### [Organic Semiconductor Layer]

The layer can be formed from any desired organic semiconductor. Organic semiconductors are classified into p type and n type by semiconductor characteristics. The terms "p type" and "n type" indicate that whether holes or electrons contribute to electrical conduction, and whether a material is of the p type or the n type depends on the electronic state, doped state, and trap state of the material. Consequently, although examples of organic semiconductors are shown below, there are cases where organic semiconductors cannot always be clearly classified into the p type and the n type. Some organic semiconductors by themselves show both the p type characteristics and the n type characteristics.

Examples of p type semiconductors include porphyrin compounds such as tetrabenzoporphyrin, copper tetrabenzoporphyrin, and zinc tetrabenzoporphyrin; phthalocyanine compounds such as phthalocyanine, copper phthalocyanine, and zinc phthalocyanine; naphthalocyanine compounds; polyacenes such as tetracene and pentacene; and oligothiophenes, such as sexithiophene, and derivatives containing any of these compounds as a framework. Examples thereof further include polymers such as polythiophenes including poly(3-alkylthiophene)s, polyfluorene, polyphenylenevinylene, polytriallylamine, polyacetylene, polyaniline, and polypyrrole.

Examples of n type semiconductors include fullerenes (C60, C70, and C76); octaazaporphyrin; the perfluorinated forms of the p type semiconductors enumerated above; aromatic carboxylic acid anhydrides and imides thereof, such as naphthalenetetracarboxylic anhydride, naphthalenetetracarboxylic acid diimide, perylenetetracarboxylic anhydride, and perylenetetracarboxylic acid diimide; and derivatives containing any of these compounds as a framework.

The specific configuration of the organic semiconductor layer is not limited so long as the layer contains at least a p type semiconductor and an n type semiconductor. The organic semiconductor layer may be constituted of a single-layer film alone, or may be composed of two or more superposed films. For example, an n type semiconductor and a p type semiconductor may be incorporated into respective films, or an n type semiconductor and a p type semiconductor may be incorporated into the same film.

Furthermore, one n type semiconductor and one p type semiconductor may be used, or two or more n type or p type semiconductors may be used in any desired combination and proportion.

Specific examples of the configuration of the organic semiconductor layer include: the bulk heterojunction type which has a layer (i layer) containing a p type semiconductor and an n type semiconductor as separate phases; the multilayer type (hetero-pn-junction type) which has the interface between a layer containing a p type semiconductor (p layer) and a layer containing an n type semiconductor (p layer); the Schottky type; and combinations of these. Of these, the bulk heterojunction type and a combination of the bulk heterojunction type and the multilayer type (p-i-n junction type) are preferred because these configurations show high performance.

The thicknesses of the p layer, i layer, and n layer in the organic semiconductor layer are not limited. However, it is preferred that the thicknesses thereof should be generally 3 nm or more, in particular, 10 nm or more, and be generally 200 nm or less, in particular, 100 nm or less. Increasing the thicknesses of those layers tends to result in enhanced film evenness, while reducing the thicknesses thereof tends to improve transmittance and reduce series resistance.

### [Hole Extraction Layer]

The hole extraction layer is a layer to be disposed at the electrode interface which faces the organic semiconductor layer, for the purpose of improving electrical properties, etc.

As a material for forming the hole extraction layer, it is especially preferred to use the polymer of the invention. However, other materials can also be used. Examples of the other materials include poly(ethylene-dioxythiophene)/poly(styrenesulfonic acid) (PEDOT/PSS), molybdenum oxide, lithium fluoride, and 2,9-dimethyl-4,7-diphenyl-1,10-phenanthroline.

### [Electrodes]

The electrodes can be formed from any desired materials having electrical conductivity. Examples of materials for the electrodes include metals such as platinum, gold, silver, aluminium, chromium, nickel, copper, titanium, magnesium, calcium, barium, and sodium or alloys of these; metal oxides such as indium oxide and tin oxide or alloys of these (ITO); conductive polymers such as polyaniline, polypyrrole, polythiophene, and polyacetylene; materials obtained by incorporating a dopant, such as an acid, e.g., hydrochloric acid, sulfuric acid, or a sulfonic acid, a Lewis acid, e.g., FeCl₃, atoms of a halogen, e.g., iodine, or atoms of a metal, e.g., sodium or potassium, into those conductive polymers; and conductive composite materials obtained by dispersing conductive particles, such as metal particles, carbon black, fullerene, or carbon nanotubes, in a matrix such as a polymer binder. Of these, materials having a large value of work function, such as gold and ITO, are preferred for the electrode where holes are captured. Meanwhile, for the electrode where electrons are captured, materials having a small value of work function, such as aluminum, are preferred. To optimize work function has an advantage that the holes and electrons which have generated upon light absorption are satisfactorily captured.

It is preferred that, of the pair of electrodes, at least the light-receiving-side electrode should have light-transmitting properties from the standpoint of electric-power generation. However, when use of an electrode which is not transparent does not exert a considerable adverse influence on power generation performance, as in the case where the electrode has an area which is small as compared with the area of the power generation layer, then the electrode need not be always transparent. Examples of transparent electrode materials include oxides such as ITO and indium-zinc oxide (IZO) and thin metal films. There are no limitations on specific ranges of the light transmittance of such electrode. However, when the power generation efficiency of the organic solar cell element is taken into account, the light transmittance thereof, excluding the loss caused by partial reflection occurring at the optical interfaces, is preferably 80% or more.

One electrode material may be used alone, or two or more electrode materials may be used in any desired combination and proportion.

There are no limitations on methods for forming the electrodes. For example, the electrodes can be formed by a dry process such as, e.g., vacuum deposition or sputtering. It is also possible to form the electrodes by a wet process using, for example, a conductive ink or the like. In this case, any desired conductive ink can be used. For example, a conductive polymer, a dispersion of metal particles, or the like can be used.

Furthermore, an electrode constituted of two or more superposed layers may be used, and properties (e.g., electrical properties or wettability) may be improved by a surface treatment.

### [Other Layers]

The organic solar cell element shown above as an embodiment may have layers other than the organic semiconductor layer and electrodes described above. The positions in which the other layers are to be formed are not limited unless such layers inhibit the organic solar cell element from generating electric power.

### <Evaluation of Performance of the Organic Solar Cell Element>

The performance of the organic solar cell element of the invention can be evaluated by subjecting the element to the accelerated test shown below and examining the resultant change in photoelectric conversion characteristics through the test.

Evaluation Method: An accelerated test is conducted by disposing the element in a high-temperature high-humidity environment within an environmental tester (e.g., SH-241, manufactured by ESPEC Corp.). It is preferred that the high-temperature high-humidity environment should be a 40°C 90% RH atmosphere or an 85°C 85% RH atmosphere. Although a test period can be suitably selected according to the materials constituting the device, it is preferred to conduct the test for 24 hours or longer. The photoelectric conversion characteristics are evaluated in the following manner. The organic thin-film solar cell is irradiated with light using a solar simulator at an irradiance of 100 mW/cm² under the conditions of AM1.5G, and is examined for current/voltage characteristics. A current/voltage curve is obtained from the examination, and an energy conversion efficiency (PCE), short-circuit current, open-circuit voltage, FF (fill factor), series resistance, and shunt resistance can be determined from the curve.

Examples of equations for comparing the photoelectric conversion characteristics determined before the accelerated test with the photoelectric conversion characteristics determined after the test include: (Degree of PCE change) = (PCE after accelerated test)/(PCE before accelerated test).

Specifically, the degree of change in energy conversion efficiency (PCE), which is defined by the equation, of the organic solar cell element according to the invention is generally 0.86 or higher, preferably 0.88 or higher, more preferably 0.90 or higher, in terms of the value after the accelerated test relative to the initial performance.

The organic solar cell element according to the invention further has the following performance. Even when a load is imposed on the element, a layer (C) containing a scavenger does not come into contact with the electrodes of the element. The element is hence highly inhibited from deteriorating. For evaluating this performance, use may be made of a method in which the layer (C) containing a scavenger is pushed from the gas barrier film side toward the organic semiconductor element (B) and the layer (C) in this state is examined as to whether the layer is in contact with the element (B).

The organic solar cell element according to the invention has satisfactory weatherability. Even when subjected to an outdoor exposure test or to a weatherability test using a weatherometer, the element retains the performance and shows high durability. It is thought that the satisfactory weatherability of the element is attributable to the presence of the corrosion-preventive layer which inhibits electrode deterioration. Furthermore, in the case where a weatherable protective sheet has been laminated, the organic solar cell element has higher weatherability. Examples

The invention will be explained in more detail by reference to Examples.

### [Synthesis Examples]

### (Synthesis Example 1) Synthesis of Compound DNOTf

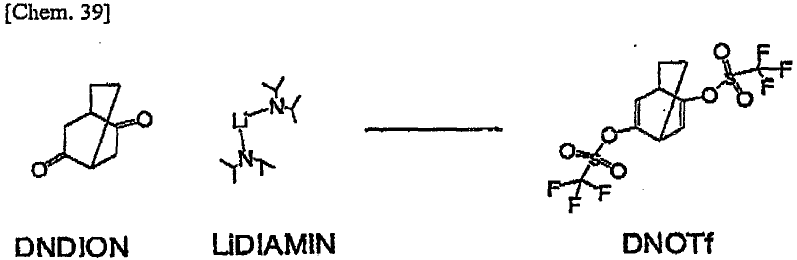

In a nitrogen stream, LiDIAMIN (4.15 g) was added to a solution-state mixture of DNDION (1.0 g) (manufactured by Aldrich Chemical Co.) and tetrahydrofuran (20 mL) with stirring at -78°C, and the resultant mixture was stirred for 1 hour. Thereafter, N-(2-pyridyl)triflimid (7.88 g) was added thereto, and this mixture was stirred at room temperature for 2 days. Fifty milliliters of cold water was added to the reaction solution, and the mixture was extracted with ether. Furthermore, the ether solution was washed with water, dried with MgSO₄, and concentrated by an evaporator. The concentrate was purified with a silica column. Thus, DNOTf having a purity of 80% by weight was obtained in an amount of 1.2 g.

### (Results of NMR Analysis)

### Compound DNOTf: 1H NMR (CDCl3, 00 MHz) δ 1.6-1.9 (m, 4H), 3.6-3.8 (m, 2H), 6.1-6.2 (s, 2H)

### (Synthesis Example 2) Synthesis of Compounds BiNpANDN and NpANDN

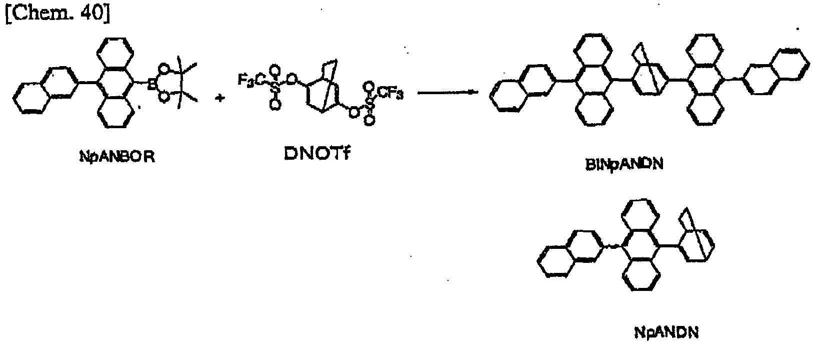

Ethanol (3 g), NpANBOR (0.6 g), the DNOTf (0.415 g), sodium carbonate (1.19 g), and water (6 g) were added to toluene (10 g) which had undergone 10-minute nitrogen bubbling, and this mixture was stirred for 30 minutes. Thereafter, tetrakistriphenylphosphinepalladiumdi (0.065 g) was added thereto by hand and dissolved therein, and the resultant solution was stirred at 70°C for 5 hours. This solution was returned to room temperature, and 100 mL of water was added thereto. The crystals which precipitated were taken out by filtration and washed with ethanol. The crystals were further purified with recycling preparative HPLC Type LC-9204, manufactured by Japan Analytical Industry Co., Ltd. (preparative column, JAIGEL-1H-40). Thus, compound BiNpANDN (0.2 g) and compound NpANDN (0.1 g) were obtained.

### (Results of Mass Analysis)

Mass analysis was conducted using mass spectrometer JMS-700/MStation, manufactured by JEOL Ltd. (accelerating voltage, 70 eV; change in emitter current, from 0 A to 0.9 A; range of scanned mass numbers, m/z 100-1,300; 2.0 sec/scan).

The results were: m/z = M⁺ 710.3 (BiNpANDN) and m/z = M⁺ 488 (NpANDN).

### (EXAMPLE 1)

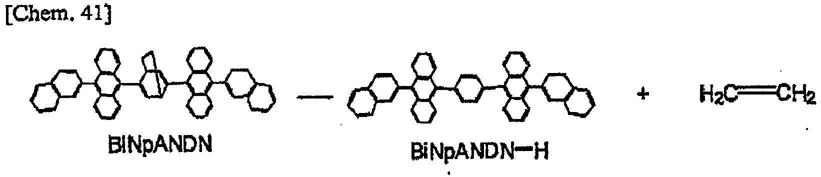

The compound BiNpANDN was heated at 180°C for 60 minutes. As a result, compound BiNpANDN-H, which is the compound formed by elimination of ethylene from that compound, was obtained. The compound yielded was identified by NMR analysis.

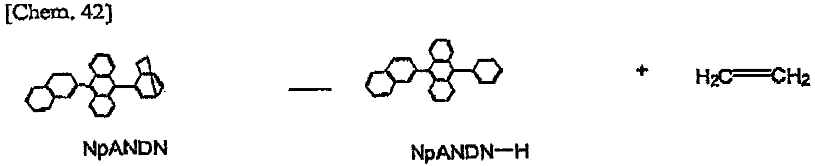

The compound NpANDN was heated at 150°C for 60 minutes. As a result, compound NpANDN-H, which is the compound formed by elimination of ethylene from that compound, was obtained. The compound yielded was identified by NMR analysis.

### (Results of NMR Analysis)

### Compound NpANDN-H: 1H NMR (CDCl₃, 400 MHz) 8 7.0-8.1 (m, 20H)

### Compound BiNpANDN-H: 1HNMR (CDCl₃, 400 MHz) 8 7.0-8.2 (m, 34H)

### (Differential Scanning Calorimetry)

An examination was made with a differential scanning calorimeter (DSC 6220, manufactured by SII Nano Technology Inc.). It was ascertained that thermal elimination from the BiNpANDN and NpANDN began to occur efficiently at temperatures of 120°C and 150°C, respectively.

### <Evaluation of Solubility in Organic Solvents>

Thirteen milligrams of the compound BiNpAN and 13 mg of the compound BiNpAN-H were introduced respectively into two 5-mL sample bottles containing 1 g of cyclohexylbenzene. The resultant dispersions were subjected to a 2-minute stirring treatment with an ultrasonic stirrer. As a result, the compound BiNpAN dissolved completely, while 80% or more of the BiNpAN-H remained undissolved.

Nineteen milligrams of the compound NpAN and 19 mg of the compound NpAN-H were introduced respectively into two 5-mL sample bottles containing 1 g of a cyclohexylbenzene/hexane (80/20 by weight) mixture solution. The resultant dispersions were subjected to a 2-minute stirring treatment with an ultrasonic stirrer. As a result, the compound NpAN dissolved completely, while 80% or more of the NpAN-H remained undissolved.

### (EXAMPLE 2)

First, a substrate obtained by patterning a transparent conductive film of indium-tin oxide (ITO) deposited on a glass substrate (deposited by sputtering; manufactured by Sanyo Vacuum Industries Co., Ltd.) into stripes having a width of 2 mm was cleaned by subjecting the substrate to ultrasonic cleaning with an aqueous surfactant solution, rinsing with ultrapure water, ultrasonic cleaning with ultrapure water, and rinsing with ultrapure water in this order, subsequently dried by nitrogen blowing, and finally subjected to ultraviolet/ozone cleaning.

### (Formation of Hole Injection Layer)

A polymer represented by the following formula (XXI) (weight-average molecular weight, 60,000) and the compound represented by the following formula (XXII) were dissolved in ethyl benzoate as a solvent in amounts of 2% by weight and 0.4% by weight, respectively. Thus, a coating fluid for hole injection layer formation which had a concentration of 2% by weight was produced.

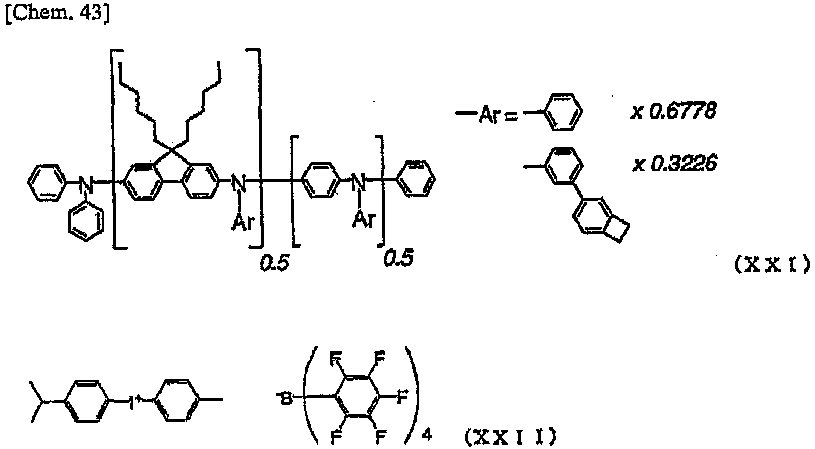

Using the coating fluid for hole injection layer formation, a hole injection layer was formed on the cleaned ITO substrate by spin coating. The spin coating was conducted in the air having a temperature of 23°C and a relative humidity of 50%, under the conditions of a rotation speed of 1,500 rpm and a rotation period of 30 seconds. After the application, the coating film was heated and dried at 80°C for 1 minute on a hot plate. Thereafter, the unnecessary part on the electrode was wiped off, and the residual coating film was baked at 230°C for 1 hour in the air within an oven. Thus, a hole injection layer (Al) having a thickness of 30 nm was formed.

### (Formation of Hole Transport Layer)

Next, a polymer represented by the following formula (XXIII) (weight-average molecular weight, 95,000) was dissolved in cyclohexylbenzene as a solvent in an amount of 1.4% by weight to produce a coating fluid for hole transport layer formation which had a concentration of 1.4% by weight

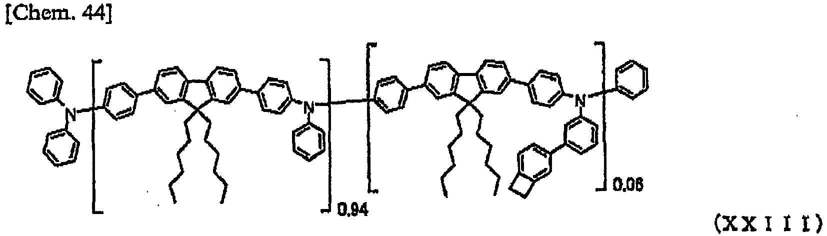

The substrate coated with the hole injection layer was placed in a gloved nitrogen box, and a hole transport layer was formed on the hole injection layer by spin coating using the coating fluid for hole transport layer formation. The rotation speed was 1,500 rpm, and the rotation period was 120 seconds. After the application, the unnecessary part on the electrode was wiped off, and the residual coating film was baked at 230°C for 1 hour on a hot plate. Thus, a hole transport layer (B1) having a thickness of 20 nm was formed.

All of the preparation of the coating fluid for hole transport layer formation, the spin coating, and the baking were conducted in a gloved nitrogen box having an oxygen concentration of 1.0 ppm and a moisture concentration of 1.0 ppm while avoiding exposure to the air.

### (Formation of Luminescent Layer)

Subsequently, a coating fluid for luminescent-layer formation, which was for forming a luminescent layer through coating fluid application, was prepared.

The BiNpANDN and the compound represented by the following formula (XXIV) were dissolved, in a ratio of 100/20 by weight, in cyclohexylbenzene as a solvent to prepare a coating fluid (D1) for luminescent-layer formation which had a concentration of 4.5% by weight. The coating fluid (D1) for luminescent-layer formation was prepared in a gloved nitrogen box having an oxygen concentration of 1.0 ppm and a moisture concentration of 1.0 ppm.

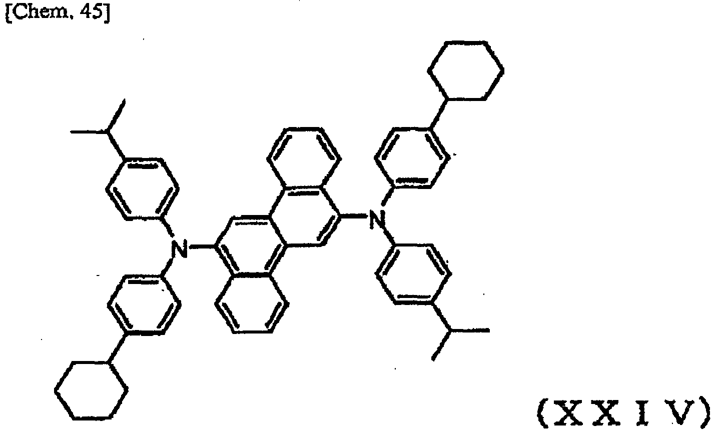

Using the coating fluid (D1) for luminescent-layer formation, a luminescent layer was formed on the hole transport layer (B1) by spin coating. The spin coating was conducted in a gloved nitrogen box having an oxygen concentration of 1.0 ppm and a moisture concentration of 1.0 ppm, under the conditions of a rotation speed of 1,200 rpm and a rotation period of 120 seconds. After the application, the unnecessary part on the electrode was wiped off, and the residual coating film was heated under vacuum at 180°C for 1 hour on a hot plate to convert the BiNpANDN into BiNpANDN-H. Thus, a luminescent layer was formed.

### (Formation of Hole Blocking Layer)

This substrate was temporarily taken out of the apparatus in the air and disposed in the chamber of a vacuum deposition apparatus. Immediately thereafter, the chamber was roughly evacuated with a rotary pump and then evacuated with a cryopump. A mask for deposition was disposed on a given area of the substrate, and necessary deposition materials placed in respective molybdenum boats were disposed beforehand in the chamber.

The molybdenum boat in which the material represented by the following formula (XXV) had been placed was heated by voltage application to vapor-deposit the material on the luminescent layer. The degree of vacuum during the deposition was 1.0×10⁻⁴ Pa, and the deposition rate was 0.8 Å/s. Thus, a hole blocking layer was formed in a thickness of 10 nm.

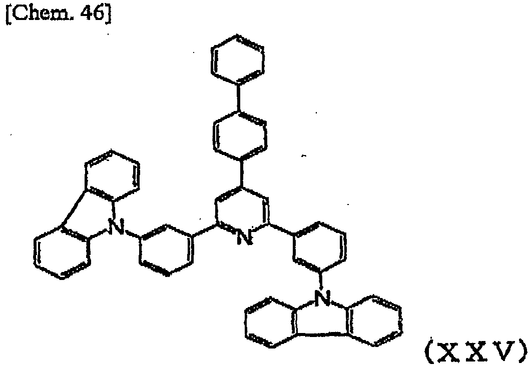

### (Formation of Electron Transport Layer)

Next, the molybdenum boat in which Alq₃, which is represented by the following formula (XXVI), had been placed was heated by voltage application to vapor-deposit the compound on the hole blocking layer. The degree of vacuum during the deposition was 1.0×10⁻⁴ Pa, and the deposition rate was 1.5 Å/s. Thus, an electron transport layer was formed in a thickness of 30 nm.

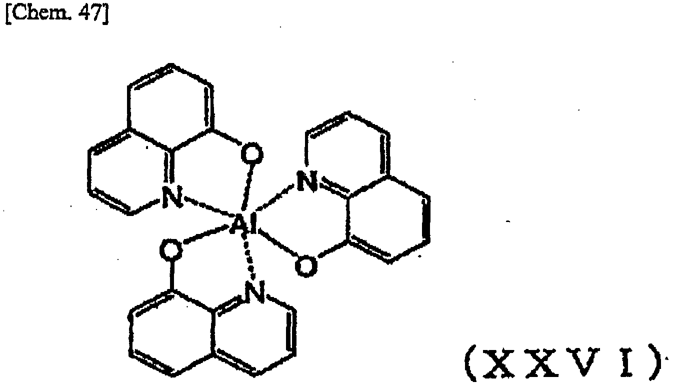

### (Formation of Cathode)

Subsequently, the substrate was temporarily taken out of the apparatus in the air, and a shadow mask in the form of stripes with a width of 2 mm, as a mask for cathode deposition, was disposed thereon so that these stripes were perpendicular to the ITO stripes of the anode. This assemblage was disposed in a deposition apparatus. The chamber was roughly evacuated with a rotary pump and then evacuated with a cryopump. The degree of vacuum was 4.5×10⁻⁴ Pa. A cathode was formed in the following manner. First, a molybdenum boat in which lithium fluoride (LiF) had been placed was heated by voltage application to deposit the fluoride on the electron transport layer. The deposition conditions included a degree of vacuum during the deposition of 4.5×10⁻⁴ Pa and a deposition rate of 0.1 Å/s, and a film of the fluoride was deposited in a thickness of 0.5 nm. Finally, a molybdenum boat in which aluminum had been placed was heated by voltage application to deposit a cathode. The deposition conditions included a degree of vacuum during the deposition of 6.0×10⁻⁴ Pa and a deposition rate of 2.5 Å/s, and an aluminum film was deposited in a thickness of 80 nm.

### (Sealing)

Next, the substrate was temporarily taken out of the apparatus in the air and transferred to a gloved box which had undergone nitrogen displacement. In the gloved box which had undergone nitrogen displacement, a moisture absorbent sheet was applied to the recessed part of a sealing glass plate, and a UV-curable resin was applied to the periphery of the recessed part of the sealing glass plate with a dispenser. The substrate which had undergone the deposition was brought into close contact with this sealing glass plate so that the area coated by the deposition was sealed with the sealing glass plate, and the UV-curable resin was cured by irradiation with UV light using a UV lamp.

Thus, an organic electroluminescent element was obtained.

### (Ascertainment of Luminescence of the Element)

A voltage was applied to the element, and blue luminescence was ascertained.

### (EXAMPLE 3)

### (Production of Organic Electroluminescent Element)

An organic electroluminescent element was produced in the same manner as in Example 1, except that the formation of a luminescent layer was changed to the following.

### (Formation of Luminescent Layer)

A hundred percents by weight the NpANDN and 10% by weight the compound represented by formula (XXIV), which was given above, were dissolved in a solvent prepared by mixing toluene with cyclohexylbenzene in a weight ratio of 8/2, thereby preparing a coating fluid (D2) for luminescent-layer formation which had a concentration of 1.5% by weight. The coating fluid (D2) for luminescent-layer formation was prepared in a gloved nitrogen box having an oxygen concentration of 1.0 ppm and a moisture concentration of 1.0 ppm.

Using the coating fluid (D2) for luminescent-layer formation, a luminescent layer was formed on the hole transport layer (B1) by spin coating. The rotation speed was 1,200 rpm, and the rotation period was 60 seconds. After the application, the unnecessary part on the electrode was wiped off, and the residual coating film was heated at 140°C for 1 hour on a hot plate to convert the NpANDN into NpANDN-H. Thus, a luminescent layer was formed. The spin coating and the heating were conducted in a gloved nitrogen box having an oxygen concentration of 1.0 ppm and a moisture concentration of 1.0 ppm.

### (Ascertainment of Luminescence of the Element)

A voltage was applied to this element, and blue luminescence was ascertained.

### (Synthesis Example 3) Synthesis of DOAN

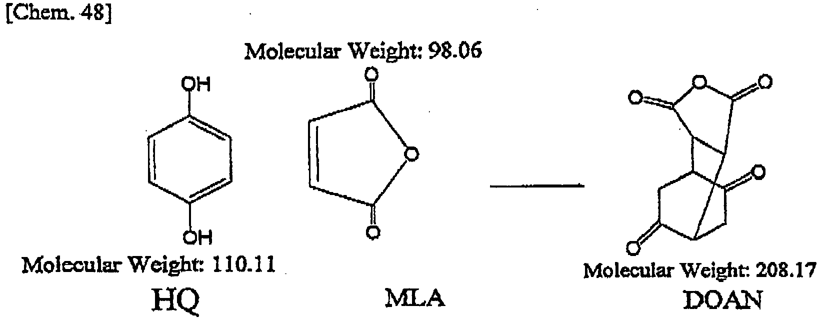

MLA (476 g) was added to HQ (308 g), and the mixture was stirred at 190°C for 5 hours in a nitrogen stream. This mixture was cooled to 50°C. Thereafter, ethyl acetate (300 mL) and ether (650 mL) were added thereto, and the resultant mixture was refluxed for 30 minutes and then returned to room temperature. The crystals which had precipitated were taken out by filtration. Thus, 44 g of DOAN (purity, 95%) was obtained.

### (Synthesis Example 4) Synthesis of DION-2

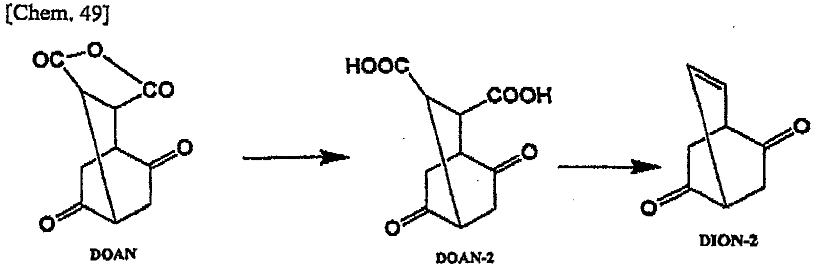

Water (40 g) was added to the DOAN (42 g), and the water was distilled off with refluxing at 70°C under vacuum. The residue was further dried to obtain DOAN-2 as a product of ring-opening of the anhydride group.

Subsequently, lead tetraacetate (212 g) and 1,4-dioxane (460 g) were added to the DOAN-2 at room temperature in a nitrogen stream, and the resultant solution was cooled to 15°C. Thereto was added 2-N aqueous nitric acid solution (400 mL). After the dropwise addition, the mixture was allowed to stand at 60°C for 10 minutes. This solution was rapidly cooled and then extracted with chloroform, and the extract was washed with saturated sodium carbonate solution and then concentrated. Furthermore, the concentrate was purified with a silica column. Thus, 15 g of DION-2 (purity, 97%) was obtained.

### (Results ofNMR Analysis)

### Compound DION-2: 1H NMR (CDCl₃, 400 MHz) δ 2.25-2.5 (m, 4H), 3.35-3.5 (m, 2H), 6.5-6.6 (d, 2H)

### (Synthesis Example 5) Synthesis of DION

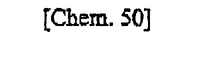

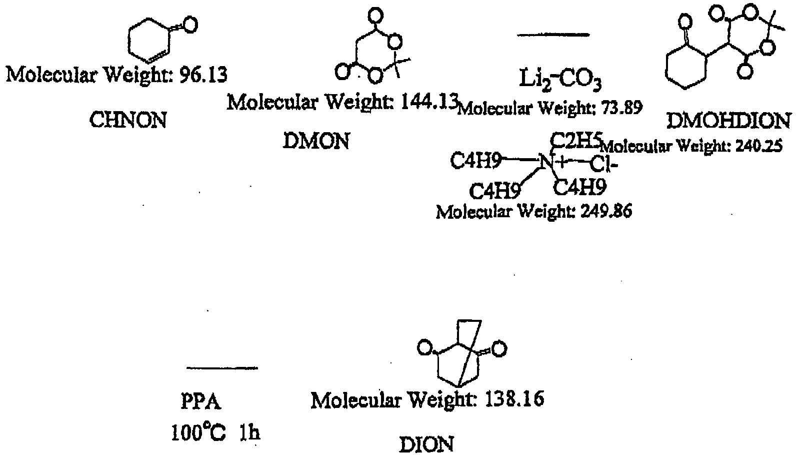

DMON (107 g) was added to acetonitrile (300 mL). While this mixture was being stirred at room temperature in a nitrogen stream, tributylethylammonium chloride (106 g) and lithium carbonate (48 g) were added thereto. After the resultant mixture was continuously stirred for 20 minutes, CHNON (40 g) was added dropwise thereto, and this mixture was stirred for 30 hours at room temperature in a nitrogen stream. Ethyl acetate (700 mL) and water (700 mL) were further added to the resultant liquid reaction mixture, and this mixture was stirred. Thereafter, the pH of the liquid reaction mixture was adjusted to 1-2 with 11 N aqueous hydrogen chloride solution. Subsequently, the reaction mixture was subjected to liquid separation, and the organic layer was dried with MgSO₄, concentrated to cause crystallization, and then subjected to filtration and washing with methanol. Thus, 85 g (yield, 85%) of DMOHDION (purity, 97%) was obtained.

Subsequently, polyphosphoric acid (PPA) (63 g) and acetic acid (55 g) were added to the DMOHDION (85 g), and the mixture was stirred for 60 minutes in a 100°C nitrogen stream. After the mixture was returned to room temperature, saturated aqueous sodium chloride solution (160 mL) which had been cooled was added thereto. The resultant mixture was extracted with toluene, and the extract was washed with saturated aqueous sodium hydrogen carbonate solution. The extract was further dried with sodium sulfate and concentrated. Thus, 36 g (yield, 60%) of DION (purity, 95%) was obtained.

### (Results of NMR Analysis)

### Compound DION: 1H NMR (CDCl₃, 400 MHz) δ 1.75-1.9 (m, 2H), 2.05-2.2 (m, 2H), 2.3-2.7 (m, 5H), 3.1-3.25 (s, 1H)

### (Synthesis Example 6) Synthesis Example for DN26OTf

In a nitrogen stream, lithium diisopropylamine (LiDIAMIN) (4.15 g) was added to a solution-state mixture of DNDION (1.0 g) (manufactured by Aldrich Chemical Co.) and tetrahydrofuran (20 mL) with stirring at -78°C, and the resultant mixture was stirred for 1 hour. Thereafter, N-(2-pyridyl)triflimid (7.88 g) was added thereto, and this mixture was stirred at room temperature for 2 days. Fifty milliliters of cold water was added to the reaction solution, and the mixture was extracted with ether. Furthermore, the ether solution was washed with water, dried with MgSO₄, and concentrated with an evaporator. The concentrate was purified with a silica column. Thus, DN26OTf having a purity of 80% by weight was obtained in an amount of 1.2 g.

### (Results of NMR Analysis)

### Compound DN26OTf: 1H NMR (CDCl₃, 400 MHz) δ 1.55-1.7 (m, 1H), 1.75-1.9 (m, 1H), 6.1-6.2 (d, 2H)

### (Synthesis Example 7) Synthesis of CzPhDNPhCz

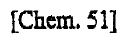

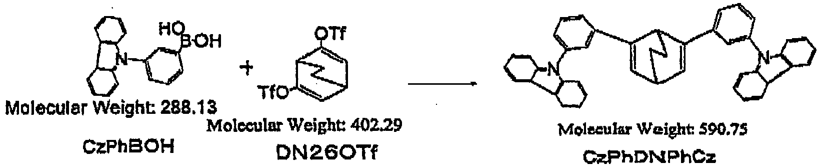

Synthesis was conducted in the same manner as in Synthesis Example 2, except that CzPhBOH and the DN260Tf were used in place of the NpANBOR and the DNOTf, respectively, in the same molar amounts. Thus, compound CzPhDNPhCz (0.2 g) was obtained.

### (Results of NMR Analysis)

### Compound CzPhDNPhCz: 1H NMR (CDCl₃, 400 MHz) δ 1.5-1.7 (m, 4H), 4.8-4.9 (m, 1H), 4.65-4.8 (m, 1H), 6.7-6.8 (d, 2H), 7.2-8.2 (m, 24)

### (Differential Scanning Calorimetry)

An examination was made with a differential scanning calorimeter (DSC 6220, manufactured by SIT Nano Technology Inc.). It was ascertained that thermal elimination began to occur efficiently at a temperature of 150°C.

### (Synthesis Example 8) Synthesis of NpANPh-DN-PhANNp and NpANPh-DN

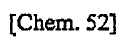

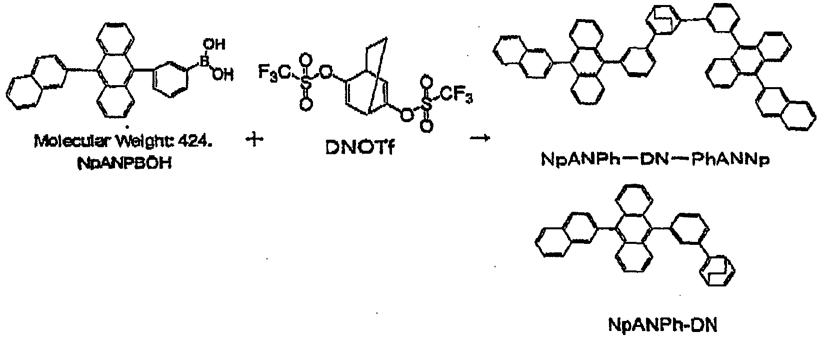

Synthesis was conducted in the same manner as in Synthesis Example 2, except that NpANPBOH was added in place of the NpANBOR in the same molar amount. Thus, compound NpANPh-DN-PhANNp (0.17 g) and compound NpANPh-DN (0.11 g) were obtained.

### (Results ofNMR Analysis)

### Compound NpANPh-DN-PhANNp: 1H NMR (CDCl₃, 400 MHz) 8 1.5-1.7 (m, 4H), 4.8-4.9 (m, 1H), 4.65-4.8 (m, 1H), 6.7-6.8 (d, 2H), 7.2-8.2 (m, 38)

### Compound NpANPh-DN: 1H NMR (CDCl3, 400 MHz) δ 1.5-1.7 (m, 4H), 4.8-4.9 (m, 1H), 4.65-4.8 (m, 1H), 6.7-6.8 (d, 2H), 7.2-8.2 (m, 19)

### (Differential Scanning Calorimetry)

An examination was made with a differential scanning calorimeter (DSC 6220, manufactured by SII Nano Technology Inc.). It was ascertained that thermal elimination from the NpANPh-DN-PhANNp and NpANPh-DN began to occur efficiently at temperatures of 146°C and 157°C, respectively.

### (Reference Example 1) DNCO Synthesis 1

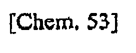

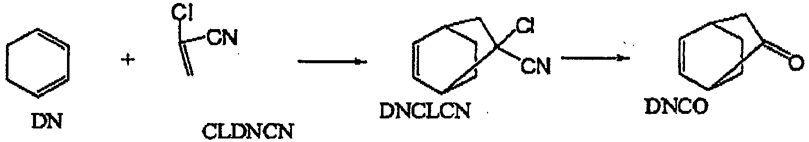

Cyclohexadiene (DN) (102.4 g), chloroacetonitrile (CLDNCN) (146.2 g), and 2,6-dibutyl-4-methylphenol (DBT) were stirred together at 70°C for 40 hours in a nitrogen stream. Thereafter, water was added thereto, and the resultant mixture was subjected to liquid separation. The oily layer was washed with 0.2-N hydrogen chloride solution. This oily layer was further washed with saturated aqueous sodium chloride solution, dried with Na₂SO₄, and concentrated by evaporation. The concentrate was purified with a silica column. As a result, 80 g of DNCLCN (purity, 90%) was obtained.

Subsequently, Bu₄NHSO₄ (84.1 g), 3-N aqueous NaOH solution (395 g), and 500 mL of dichloromethane were added to 80 g of the DNCLCN. and this mixture was stirred at room temperature for 40 hours in an air stream. Thereafter, 0.1-N aqueous hydrogen chloride solution was added thereto, and the resultant mixture was subjected to liquid separation. The organic layer was purified with a silica column to obtain 30 g of mDNCO (purity, 90%).

### (Results ofNMR Analysis): 1H NMR (CDCl3, 400 MHz) δ 1.45-2.1 (m, 6H). 2.9-3.2 (m, 2H). 6.1-6.5 (m, 2H)

### (Reference Example 2) DNCO Synthesis 2

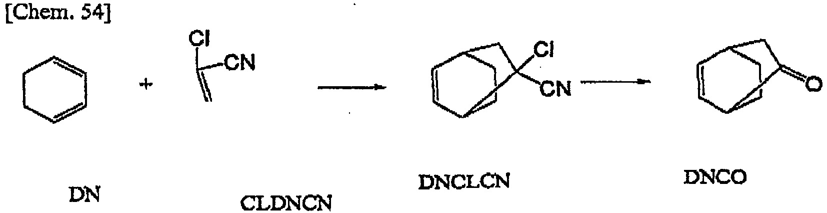

To DNCLCN (60 g) (purity, 80%) obtained in the same manner as in Reference Example 1 were added 700 mL of ethanol and Na₂S-9H₂O (103.5 g). This mixture was refluxed for 10 hours in a nitrogen stream. Thereafter, the mixture was concentrated by evaporation, and the concentrate was purified with a silica column. Thus, 20 g of the target compound mDNCO (purity, 90%) was obtained.

### (Results ofNMR Analysis): 1H NMR (CDCl₃, 400 MHz) δ 1.45-2.1 (m, 6H), 2.9-3.2 (m, 2H), 6.1-6.5 (m, 2H)

### (Reference Example 3) DION-2 Synthesis 2

The same procedure as in Reference Example 2 was conducted, except that 2-trimethoxysilylcyclohexadiene was used in place of the DN in the same molar amount. Thus, DION-2 (10 g) was obtained.

### (Results ofNMR Analysis): 1H NMR (CDCl₃, 400 MHz) δ 1.8-2.2 (m, 4H), 2.45-2.65 (d, 4H), 2.7-2.9 (s, 2H)

### (Reference Example 4) DNCO Synthesis 3

### (Synthesis of DNCOOBz)

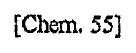

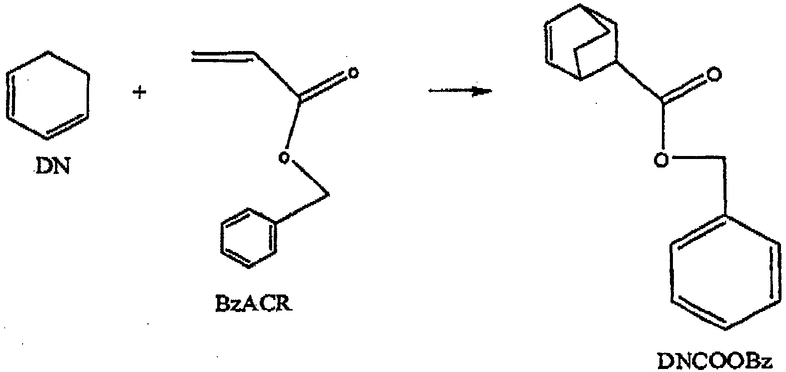

To cyclohexadiene DN (43 g) were added BzACR (Biscoat 160; Osaka Organic Chemical) (24 g) and hydroxybenzoquinone (0.1 g). The resultant mixture was reacted at 100°C for 20 hours in a nitrogen stream. Thereafter, the reaction mixture was purified with a silica column to obtain 74 g (yield, 90%) of DNCOOBz (purity, 98%).

### (Synthesis of DNCOOH)

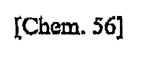

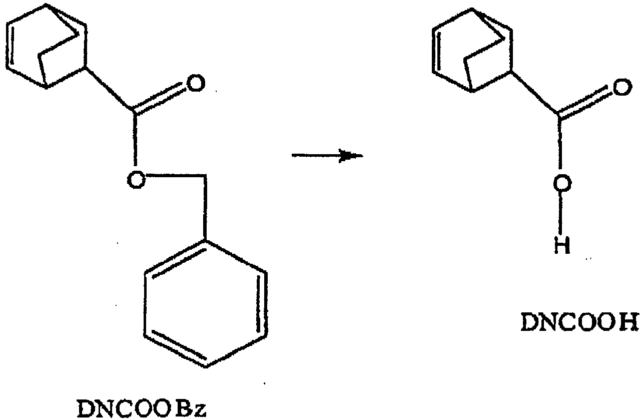

The DNCOOBz (17 g) and sodium hydroxide (14 g) were added to an ethanol/water (4/1 by weight) mixture (175 g), and the resultant mixture was stirred at room temperature for 12 hours in a nitrogen stream. To the resultant liquid reaction mixture were added 20 mL of 1-N aqueous sodium hydrogen carbonate solution and 40 mL of dichloromethane. This mixture was subjected to liquid separation, and the aqueous layer was acidified with an aqueous hydrochloric acid solution so as to result in a pH of 1. Thereafter, this aqueous layer was extracted with dichloroethane, and the extract was dried with sodium sulfate and then concentrated by evaporation. Thus, 11.2 g (yield, 90% by weight) of DNCOOH (purity, 95%) was obtained.

### (Synthesis of DNCO)

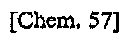

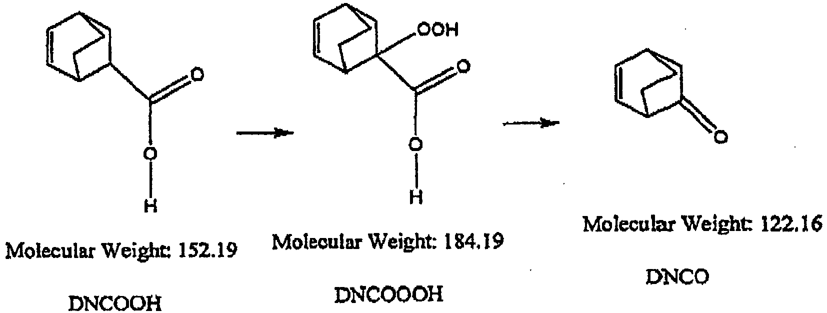

In a nitrogen stream, LiDIAMIN (10.366 g) was added to a solution-state mixture of tetrahydrofuran (20 mL) with stirring at -78°C. This mixture was stirred for 1 hour. Thereafter, a solution prepared by mixing hexamethylphosphoric triamide (HMPT) (1 mL) with tetrahydrofuran (10 mL) was added dropwise thereto, and the DNCOOH (0.951 g) was further added dropwise. The resultant mixture was allowed to stand for 3 hours.

Subsequently, dry air was bubbled into the mixture, which was allowed to stand overnight. Thereafter, the mixture was decanted and concentrated by evaporation. Tetrahydrofuran and 10% HC 1 solution were added to the concentrate, and the resultant mixture was subjected to liquid separation. The tetrahydrofuran solution was extracted and then dried with magnesium sulfate, and 10 mL of dichloromethane was further added thereto. In a nitrogen stream, N,N-dimethylformamide dimethyl acetal (2.383 g) was added to the mixture with stirring at -78°C. The resultant mixture was gradually returned to room temperature and continuously stirred for 12 hours.

Fifty milliliters of cold water was added to the resultant reaction solution, and this mixture was extracted with ether. Furthermore, the ether solution was washed with water, dried with magnesium sulfate, and concentrated with an evaporator. The concentrate was purified with a silica gel column. Thus, DNCO having a purity of 90% by weight was obtained in an amount of 0.42 g.

### (Results of NMR. Analysis): 1H NMR (CDCl₃, 400 MHz) δ 1.45-2.1 (m, 6H), 2.9-3.2 (m, 2H), 6.1-6.5 (m, 2H)

### (Synthesis Example 9) Synthesis of Compound DNOTf

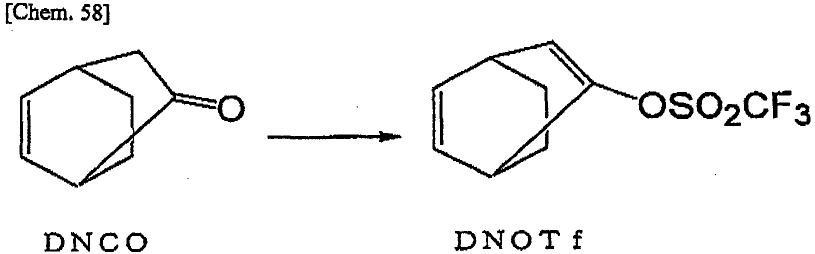

In a nitrogen stream, LiDIAMIN (4.15 g) was added to a solution-state mixture of mDNCO (1.0 g) (manufactured by Aldrich Chemical Co.) and tetrahydrofuran (20 mL) with stirring at -78°C, and the resultant mixture was stirred for 1 hour. Thereafter, N-(2-pyridyl)triflimid (7.88 g) was further added, and this mixture was then stirred at room temperature for 2 days.

Thereafter, 50 mL of cold water was added to the reaction solution, and the resultant mixture was extracted with ether. Furthermore, the ether solution was washed with water, dried with MgSO₄, and concentrated with an evaporator. The concentrate was purified with a silica gel column to obtain 1.2 g of DNOTf having a purity of 80% by weight

### (Results of NMR Analysis)

### Compound DNOTf: 1H NMR (CDCl₃, 400 MHz) δ 1.3-1.9 (m, 4H), 3.6-3.8 (m, 2H), 6.0-6.5 (m, 3H)

### (Synthesis Example 10) Synthesis of Aniline Derivative 1 (AcNHPhDN)

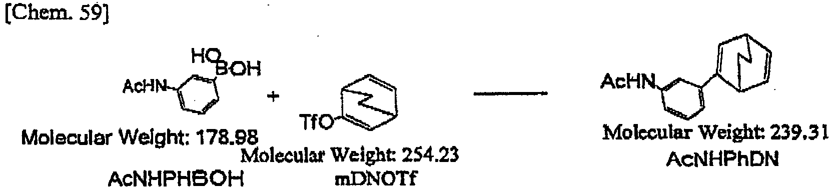

To a toluene (60 mL)/ethanol (30 mL) mixed solvent were added mDNOTf (3.47 g) and AcNHPHBOH (4.18 g). Nitrogen was bubbled thereinto for 40 minutes.

An aqueous sodium carbonate solution (prepared by dissolving 6.6 g of sodium hydrogen carbonate in 30 mL of water and then subjecting the solution to 30-minute nitrogen bubbling) was further added to the reaction solution.

Subsequently, 0.3 g of Pd(PPh₃)₄ (tetrakis(triphenylphosphine)palladium(0)) was added thereto, and the resultant reaction solution was refluxed for 2 hours. The organic layer was separated out and washed with water twice. The organic layer was dried under vacuum, and the residue was purified by column chromatography to obtain AcNHPhDN (2.09 g; yield, 70%).

### (Differential Scanning Calorimetry)

An examination was made with a differential scanning calorimeter (DSC 6220, manufactured by SII Nano Technology Inc.). It was ascertained that thermal elimination began to occur efficiently at a temperature of 143°C.

### (Synthesis Example 10) Synthesis of Aniline Derivative 2

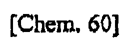

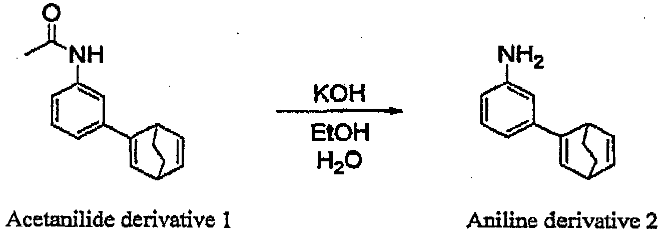

An aqueous potassium hydroxide solution (potassium hydroxide, 33 g; water 33 mL) was added to an ethanol solution (80 mL) of acetanilide derivative 1 (1.2 g). Thereafter, the mixture was stirred with heating at 80°C for 12 hours. After the reaction mixture was allowed to cool, toluene (200 mL) and water (200 mL) were added thereto to extract the target compound with the organic layer. The organic layer was washed with water and then dried with magnesium sulfate, and the toluene was distilled off under vacuum. Thus, aniline derivative 2 (1.0 g) was obtained.

### (Synthesis Example 11) Synthesis of Polymer 3

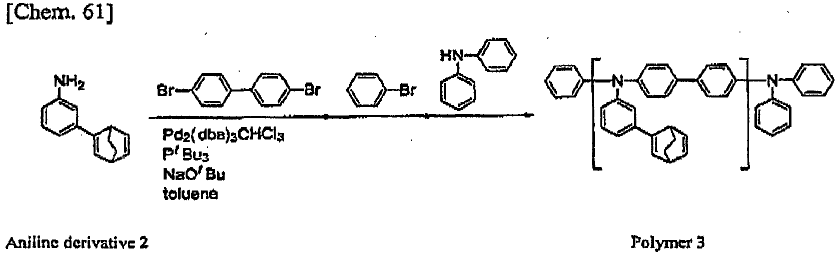

The aniline derivative 2 (1.0 g), 4,4'-dibromobiphenyl (0.79 g), tert-butoxysodium (1.66 g), and toluene (15 mL) were introduced into a reaction vessel. The atmosphere in the system was sufficiently replaced with nitrogen, and the contents were heated to 60°C (solution A). Tri-tert-butylphosphine (0.081 g) was added to a solution in 3-mL toluene of a tris(dibenzylideneacetone)dipalladium chloroform complex (0.052 g), and this mixture was heated to 60°C (solution B). In a nitrogen stream, solution B was added to solution A, and the mixture was heated with reflexing for 2 hours.

Thereto was added 4,4'-dibromobiphenyl (0.40 g). This mixture was heated with refluxing for 2 hours.

Furthermore, 4,4'-dibromobiphenyl (0.16 g) was added thereto, and this mixture was heated with refluxing for 2 hours.

Subsequently, bromobenzene (0.31 g) was added thereto, and this mixture was heated with refluxing for 2 hours. Thereafter, diphenylamine (0.84 g) was added thereto, and this mixture was heated with refluxing for 2 hours. The resultant liquid reaction mixture was allowed to cool and then dropped into 300 mL of ethanol to precipitate crude polymer 3. This polymer was taken out by filtration and dried.

The crude polymer 3 was redissolved in toluene, and this toluene solution was washed with 1 N hydrochloric acid and water. Thereafter, the toluene was distilled off under vacuum, and the residue was dissolved in tetrahydrofuran (20 mL). This solution was added to an ethanol/28% ammonia water (60 mL/6 mL) mixture to cause reprecipitation. The precipitate was taken out by filtration and dried. Thereafter, this crude polymer 3 was redissolved in toluene (150 mL), purified by column chromatography, and then dissolved in tetrahydrofuran (20 mL). This solution was added to ethanol (80 mL) to cause reprecipitation. The precipitate was taken out by filtration and dried. Thus, polymer 3 (0.51 g) was obtained. The weight-average molecular weight, number-average molecular weight, and dispersity ratio of this polymer 3 are shown below.
Weight-average molecular weight (Mw) = 8,000
Number-average molecular weight (Mn) = 5,410
Dispersity ratio (Mw/Mn) = 1.48

### (EXAMPLE 4)

### (Production of Organic Electroluminescent Element)

An organic electroluminescent element was produced in the same manner as in Example 2, except that the formation of a luminescent layer was changed to the following.

### (Formation of Luminescent Layer)

The NpANPhDN and the compound represented by formula (XXIV), which was given above, were dissolved, in a weight ratio of 100/10, in a solvent prepared by mixing toluene with cyclohexylbenzene in a weight ratio of 8/2, thereby preparing a coating fluid (D2) for luminescent-layer formation which had a concentration of 1.5 wt%. The coating fluid (D2) for luminescent-layer formation was prepared in a gloved nitrogen box having an oxygen concentration of 1.0 ppm and a moisture concentration of 1.0 ppm.

Using the coating fluid (D2) for luminescent-layer formation, a luminescent layer was formed on the hole transport layer (B1) by spin coating. The rotation speed was 1,200 rpm, and the rotation period was 60 seconds. After the application, the unnecessary part on the electrode was wiped off, and the residual coating film was heated at 180°C for 1 hour on a hot plate to convert the NpANPhDN into NpANPhDN-H. Thus, a luminescent layer was formed. The spin coating and the heating were conducted in a gloved nitrogen box having an oxygen concentration of 1.0 ppm and a moisture concentration of 1.0 ppm.

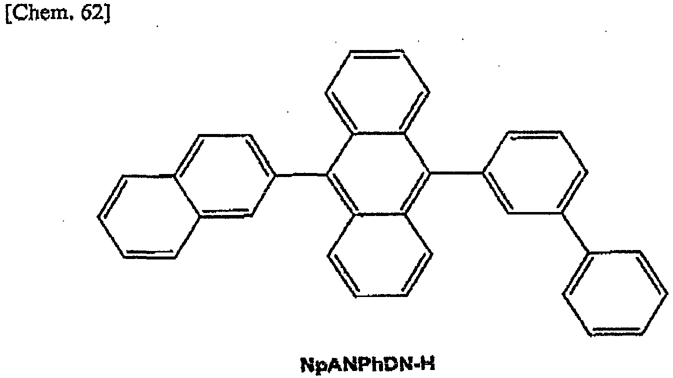

### (Ascertainment of Luminescence of the Element)

A voltage was applied to this element, and blue luminescence was ascertained.

### (EXAMPLE 5)

### (Production of Organic Electroluminescent Element)

An organic electroluminescent element was produced in the same manner as in Example 2, except that the formation of a luminescent layer was changed to the following.

### (Formation of Luminescent Layer)

The NpANPh-DN-PhANNp and the compound represented by formula (XXIV), which was given above, were dissolved, in a weight ratio of 100/10, in a solvent prepared by mixing toluene with cyclohexylbenzene in a weight ratio of 8/2, thereby preparing a coating fluid (D3) for luminescent-layer formation which had a concentration of 1.5 wt%. The coating fluid (D3) for luminescent-layer formation was prepared in a gloved nitrogen box having an oxygen concentration of 1.0 ppm and a moisture concentration of 1.0 ppm.

Using the coating fluid (D3) for luminescent-layer formation, a luminescent layer was formed on the hole transport layer (B1) by spin coating. The rotation speed was 1,200 rpm, and the rotation period was 60 seconds. After the application, the unnecessary part on the electrode was wiped off, and the residual coating film was heated at 180°C for 1 hour on a hot plate to convert the NpANPh-DN-PhANNp into NpANPh-DN-PhANNp-H. Thus, a luminescent layer was formed. The spin coating and the heating were conducted in a gloved nitrogen box having an oxygen concentration of 1.0 ppm and a moisture concentration of 1.0 ppm.

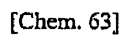

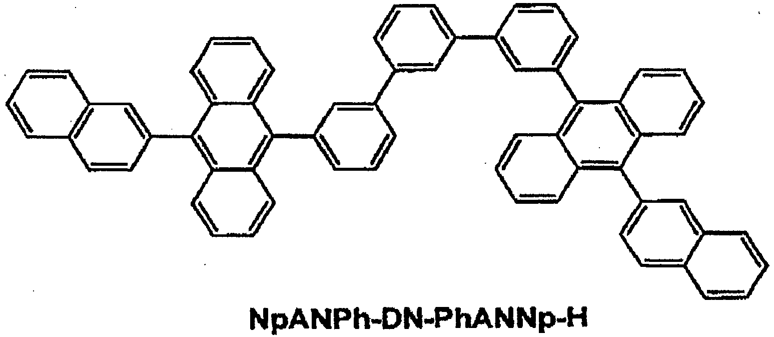

### (Ascertainment of Luminescence of the Element)

A voltage was applied to this element, and blue luminescence was ascertained.

### (EXAMPLE 6)

### (Evaluation of Solubility of Polymer 3 in Organic Solvent)

Five milligrams of the compound polymer 3 was introduced into each of two 5-mL sample bottles. One of the sample bottles was heated at 180°C for 1 hour and then returned to room temperature. Subsequently, 1 g of toluene was added to each of the two bottles, and the resultant mixtures each were subjected to a 2-minute stirring treatment with an ultrasonic stirrer. As a result, the polymer 3 in the bottle which had not undergone the heating dissolved completely, while 90% or more of the polymer 3 in the bottle which had undergone the heating remained undissolved.

### (Evaluation of Element Obtained Using Polymer 3)

First, a substrate obtained by patterning a transparent conductive film of indium-tin oxide (ITO) deposited on a glass substrate (deposited by sputtering; manufactured by Sanyo Vacuum Industries Co., Ltd.) into stripes having a width of 2 mm was cleaned with an aqueous surfactant solution and ultrapure water and then subjected to ultravioletlozone cleaning.

### (Formation of Hole Injection Layer)

A hundred parts by weight of a polymer represented by the following formula (101) (weight-average molecular weight, 60,000) and 20 parts by weight of the compound represented by the following formula (102) were dissolved in ethyl benzoate to prepare a coating fluid for hole injection layer formation which had a concentration of 2% by weight.

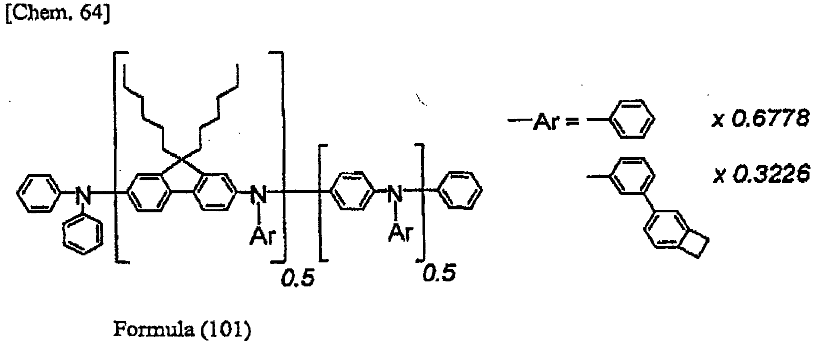

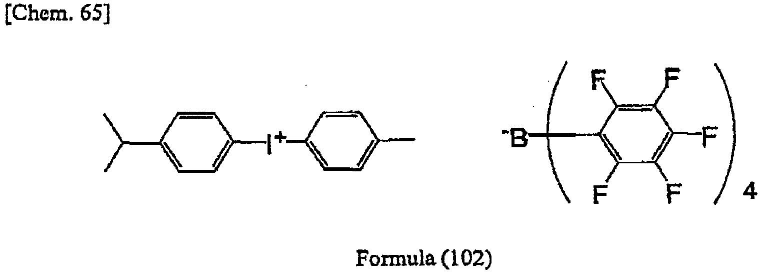

Using the coating fluid for hole injection layer formation, a hole injection layer was formed on the cleaned ITO substrate by spin coating. The spin coating was conducted in the air having a temperature of 23°C and a relative humidity of 50%, under the conditions of a rotation speed of 2,000 rpm and a rotation period of 30 seconds. After the application, the coating film was heated and dried at 80°C for 1 minute on a hot plate. Thereafter, the unnecessary part on the electrode was wiped off, and the residual coating film was baked at 230°C for 1 hour in the air within an oven. Thus, a hole injection layer having a thickness of 30 nm was formed.

### (Formation of Hole Transport Layer)

Next, a hole transparent layer was formed on the hole injection layer.

The substrate coated with the hole injection layer was placed in a gloved nitrogen box having an oxygen concentration of 1.0 ppm and a moisture concentration of 1.0 ppm. A coating fluid for hole transport layer formation was prepared. In the gloved box, this coating fluid was applied by spin coating and subjected to a baking step.

### (Preparation of Coating Fluid for Hole Transport Layer Formation)

A polymer represented by the following formula (103) (polymer 3; weight-average molecular weight, 8,000) was dissolved in toluene to prepare a coating fluid for hole transport layer formation which had a concentration of 0.5% by weight.

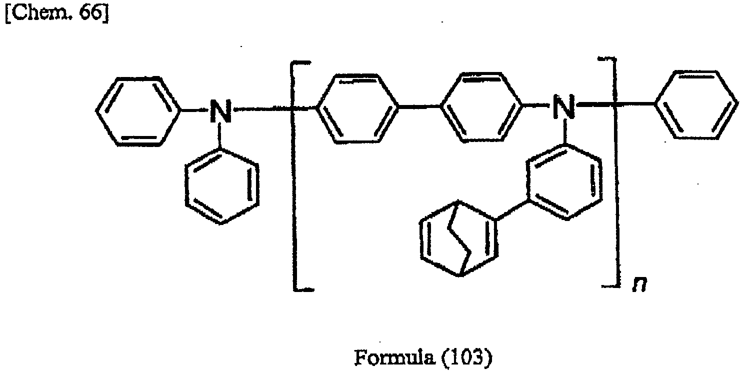

A hole injection layer was formed on the hole injection layer by spin coating using the coating fluid for hole transport layer formation. The rotation speed was 1,500 rpm, and the rotation period was 30 seconds. After the application, the unnecessary part on the electrode was wiped off, and the residual coating film was then baked at 180°C for 1 hour on a hot plate. Thus, a hole transport layer having a thickness of 24 nm was formed.

### (Formation of Luminescent Layer)

Subsequently, a luminescent layer was formed on the hole transport layer. A coating fluid for luminescent-layer formation was prepared, and spin coating and baking both were conducted in a gloved nitrogen box having an oxygen concentration of 1.0 ppm and a moisture concentration of 1.0 ppm, while avoiding exposure to the air.

### (Preparation of Coating Fluid for Luminescent-Layer Formation)

A hundred parts by weight of the compound represented by the following formula (104) and 10 parts by weight of the compound represented by the following formula (105) were dissolved in cyclohexylbenzene as a solvent to prepare a 3.4-wt% solution. Thus, a coating fluid for luminescent-layer formation was prepared.

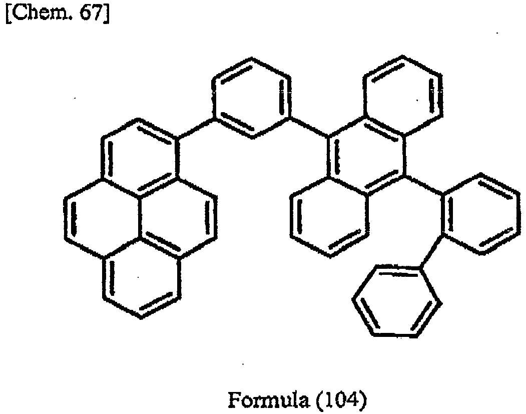

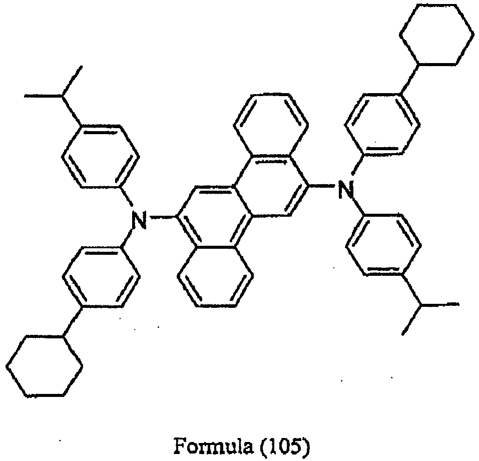

Using the coating fluid for luminescent-layer formation, a luminescent layer was formed on the hole transport layer by spin coating. The rotation speed was 1,200 rpm, and the rotation period was 120 seconds. After the application, predrying was conducted at 130°C for 10 seconds on a hot plate, and the unnecessary part on the electrode was thereafter wiped off. Subsequently, the residual coating film was heated and dried under vacuum at 130°C for 1 hour on a hot plate to form a luminescent layer having a thickness of 45 nm.

This substrate was temporarily taken out of the apparatus in the air and rapidly disposed in the chamber of a vacuum deposition apparatus. The chamber was roughly evacuated with a rotary pump and then evacuated with a cryopump. The degree of vacuum was 1.0×10⁻⁴ Pa. A mask for deposition was disposed on a given area of the substrate, and necessary deposition materials placed in respective crucibles were disposed in the chamber beforehand.

### (Formation of Hole Blocking Layer)

The crucible in which the material represented by the following formula (106) had been placed was heated with a heater wire to vapor-deposit the material on the luminescent layer. The degree of vacuum during the deposition was 1.0×10⁻⁴ Pa, and the deposition rate was 0.7 Å/s. Thus, a hole blocking layer was formed in a thickness of 10 nm.

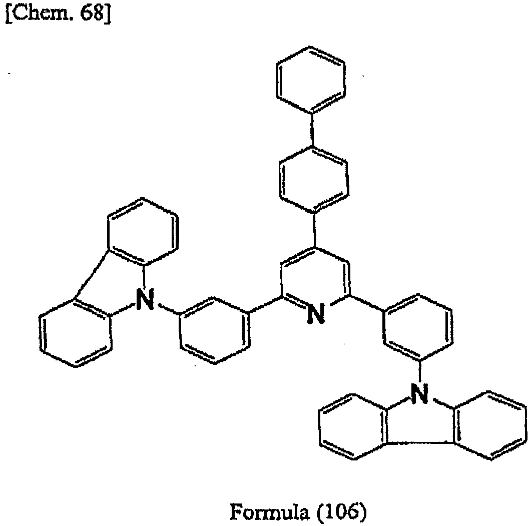

### (Formation of Electron Transport Layer)

Subsequently, the crucible in which the compound represented by the following formula (107) had been placed was heated with a heater wire to vapor-deposit the compound on the hole blocking layer. The degree of vacuum during the deposition was 1.0×10⁻⁴ Pa, and the deposition rate was 1.0 Å/s. Thus, an electron transport layer was formed in a thickness of 30 nm.

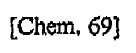

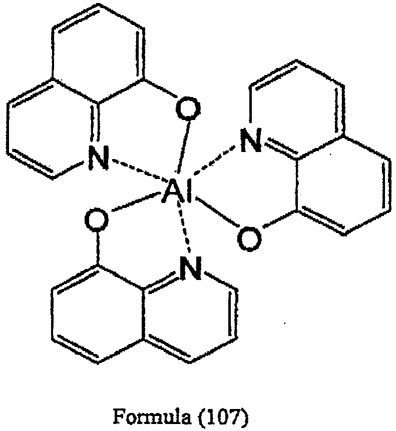

### (Formation of Cathode)

Subsequently, the substrate was temporarily taken out of the apparatus in the air, and a shadow mask in the form of stripes with a width of 2 mm, as a mask for cathode deposition, was disposed thereon so that these stripes were perpendicular to the ITO stripes of the anode. This assemblage was rapidly disposed in a deposition apparatus.

The chamber was roughly evacuated with a rotary pump and then evacuated with a cryopump. The degree of vacuum was 3.0×10⁻⁴ Pa.

A cathode was formed in the following manner. First, a molybdenum boat in which lithium fluoride (LiF) had been placed was heated by voltage application to deposit the fluoride on the electron transport layer. The deposition conditions included a degree of vacuum during the deposition of 3.0×10⁻⁴ Pa and a deposition rate of 0.1 Å/s, and a film of the fluoride was deposited in a thickness of 0.5 nm. Finally, a molybdenum boat in which aluminum had been placed was heated by voltage application to deposit a cathode. The deposition conditions included a degree of vacuum, during the deposition of 4.0×10⁻⁴ Pa and a deposition rate of 3.0 Å/s, and an aluminum film was deposited in a thickness of 80 nm.

### (Sealing)

Next, the substrate was temporarily taken out of the apparatus in the air and rapidly transferred to a gloved box which had undergone nitrogen displacement. In the gloved box which had undergone nitrogen displacement, a moisture absorbent sheet was applied to the recessed part of a sealing glass plate, and a UV-curable resin was applied to the periphery of the recessed part of the sealing glass plate with a dispenser. The substrate which had undergone the deposition was brought into close contact with this sealing glass plate so that the area coated by the deposition was sealed with the sealing glass plate, and the UV-curable resin was cured by irradiation with UV light using a UV lamp.

Thus, an organic electroluminescent element was obtained.

### (Evaluation of the Element)

A voltage was applied to this element. As a result, even blue luminescence from the luminescent surface was obtained.

When this element was caused to luminesce at a luminance of 1,000 cd/m², then the voltage was 7.2 V and the luminescent efficiency of current was 4.0 cd/A.

### (COMPARATIVE EXAMPLE 1)

An organic electroluminescent element was produced in the same manner as in Example 6, except that the hole transport layer was not formed This element was evaluated.

### (Evaluation of the Element)

A voltage was applied to this element. As a result, blue luminescence from the luminescent surface was obtained. However, when the element was caused to luminesce at a luminance of 1,000 cd/m², the voltage was as high as 11.5 V and the luminescent efficiency of current was 0.5 cd/A.

Those results show that the organic electroluminescent element having a layer formed using the polymer of the invention has a low voltage and a high luminescent efficiency.

### (EXAMPLE 7 (Reference))

A glass substrate having a size of 25 mm × 37.5 mm was washed with ultrapure water, dried with dry nitrogen, and cleaned with UV/ozone.

Subsequently, a composition (E1) for luminescent-layer formation, which was for forming a luminescent layer through coating fluid application, was prepared in the following manner.

One hundred parts by weight of the compound represented by the following formula (201), 1 part by weight of the compound represented by the following formula (202) as a green luminescent material, and 0.5 parts by weight of the compound represented by the following formula (203) as a red luminescent material were dissolved in chloroform as a solvent so as to result in a concentration of 2.0 wt%. Thus, a composition (El) for luminescent-layer formation was prepared.

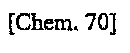

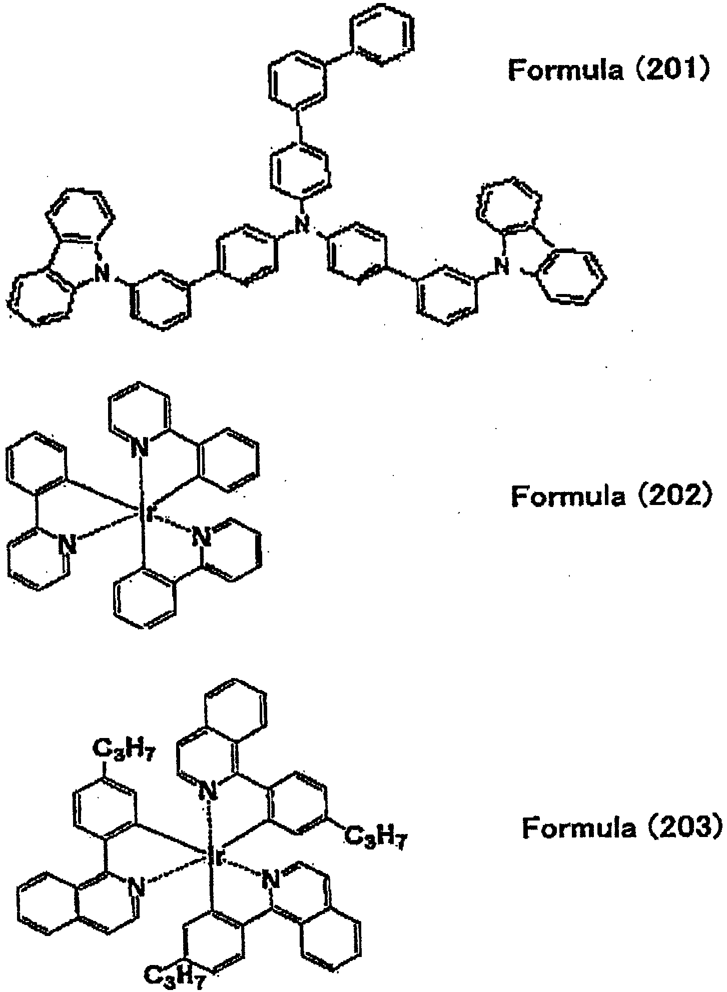

The composition (El) for luminescent-layer formation was applied on the substrate by spin coating to form a luminescent layer (E1'). The spin coating was conducted at a spinner rotation speed of 1,500 rpm for a spinner rotation period of 120 seconds.

After the application, the coating film was heated and dried at 160°C for 1 hour. A portion of the resultant film which had a width of about 2 mm was scraped off, and the thickness of the film L₀ (nm) was measured with thickness meter Tencor P-15 (manufactured by KLA-Tencor Corp.). Thickness L₀ was found to be 270 nm.

The substrate which had undergone the measurement of thickness L₀ was set on a spinner and rotated at 1,500 rpm. Thereafter, cyclohexane was dropped onto the area where the film thickness measurement had been made, and this substrate was further rotated for 120 seconds. Subsequently, film thickness L₁ (nm) was measured again in the same area, and was ascertained to be 260 nm.

The results of the measurements of L₀ and L₁ are shown in Table 6.

### (EXAMPLE 8 (Reference))

A test sample was produced and examined for film thickness in the same manners as in Example 7, except that the composition (El) for luminescent-layer formation was replaced with a composition (F1) for luminescent-layer formation.

### (Preparation of Composition (F1) for Luminescent-Layer Formation)

One hundred parts by weight of the compound represented by formula (201), 4 parts by weight of the compound represented by formula (202) as a green luminescent material, and 1 part by weight of the compound represented by formula (203) as a red luminescent material were dissolved in chloroform as a solvent so as to result in a concentration of 1.0 wt%. Thus, a composition (F1) for luminescent-layer formation was prepared.

L₀ and L₁ were measured in the same manner as in Example 7, and the results thereof are shown in Table 6.

### (EXAMPLE 9 (Reference))

A test sample was produced and examined for film thickness in the same manners as in Example 7, except that the composition (El) for luminescent-layer formation was replaced with a composition (G1) for luminescent-layer formation.

### (Preparation of Composition (G1) for Luminescent-Layer Formation)

Seventy-five parts by weight of the compound represented by formula (201), 25 parts by weight of the compound represented by the following formula (205), 1 part by weight of the compound represented by formula (202) as a green luminescent material, and 0.5 parts by weight of the compound represented by formula (203) as a red luminescent material were dissolved in chloroform as a solvent so as to result in a concentration of 2.0 wt%. Thus, a composition (G1) for luminescent-layer formation was prepared.

L₀ and L₁ were measured in the same manner as in Example 7, and the results thereof are shown in Table 6.

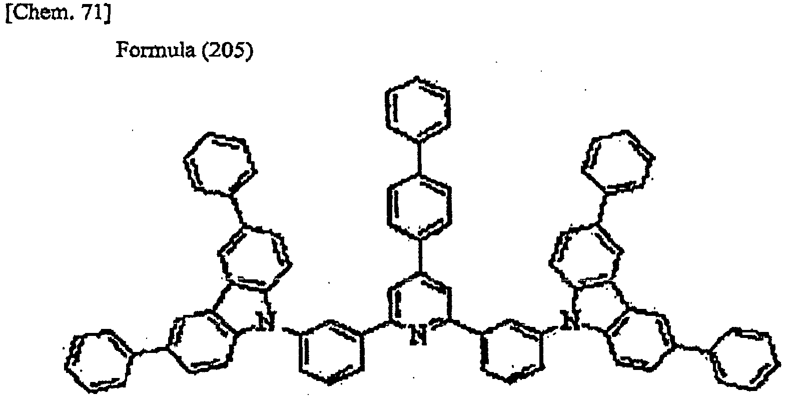

### (EXAMPLE 10 (Reference))

A test sample was produced and examined for film thickness in the same manners as in Example 7, except that the composition (El) for luminescent-layer formation was replaced with a composition (H1) for luminescent-layer formation.

### (Preparation of Composition (H1) for Luminescent-Layer Formation)

A hundred parts by weight of the compound represented by formula (201) and 5 parts by weight of the compound represented by formula (202) as a green luminescent material were dissolved in chloroform as a solvent so as to result in a concentration of 1.0 wt%. Thus, a composition (H1) for luminescent-layer formation was prepared.

L₀ and L₁ were measured in the same manner as in Example 7, and the results thereof are shown in Table 6.

**[Table 6]**

| | L₀ (nm) | L₁ (nm) |
|---|---|---|
| Example 7 | 270 | 269 |
| Example 8 | 130 | 100 |
| Example 9 | 260 | 230 |
| Example 10 | 150 | 120 |

### (EXAMPLE 11)

### (Production of Organic Electroluminescent Element of Multicolor Luminescent-Layer Superposition Type)

First, a substrate obtained by patterning a transparent conductive film of indium-tin oxide (ITO) deposited on a glass substrate (deposited by sputtering; manufactured by Sanyo Vacuum Industries Co., Ltd.) into stripes having a width of 2 mm was cleaned by subjecting the substrate to ultrasonic cleaning with an aqueous surfactant solution, rinsing with ultrapure water, ultrasonic cleaning with ultrapure water, and rinsing with ultrapure water in this order, subsequently dried by nitrogen blowing, and finally subjected to ultraviolet/ozone cleaning.

### (Formation of Hole Injection Layer)

A hole injection layer was formed in the same manner as in Example 6.

### (Formation of Hole Transport Layer)

Subsequently, 1.4 parts by weight of a polymer (weight-average molecular weight, 55,000) having the repeating structures represented by the following formula (108) was dissolved in cyclohexylbenzene as a solvent so as to result in a concentration of 1.4% by weight. Thus, a coating fluid for hole transport layer formation was produced.

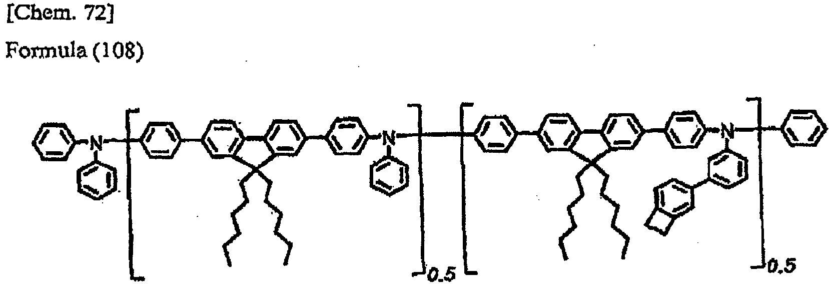

The substrate coated with the hole injection layer was placed in a gloved nitrogen box, and a hole transport layer was formed on the hole injection layer by spin coating using the coating fluid for hole transport layer formation. The rotation speed was 1,500 rpm, and the rotation period was 120 seconds. After the application, the unnecessary part on the electrode was wiped off, and the residual coating film was baked at 230°C for 1 hour on a hot plate. Thus, a hole transport layer having a thickness of 20 nm was formed.

### (Formation of Luminescent Layer (El'))

The composition (El) for luminescent-layer formation which had been prepared in Example 7 was applied on the hole transport layer by spin coating under the same film formation conditions as in Example 7. Thus, a luminescent layer (El') having a thickness of 270 nm was obtained.

### (Formation of Luminescent Layer (J2'))

A composition (J2) for luminescent-layer formation, which was for forming a luminescent layer (J2') through coating fluid application, was prepared.

One hundred parts by weight of the compound represented by the following formula (109) (compound NpANDN shown above) and 10 parts by weight of the compound represented by the following formula (110) as a blue luminescent material were dissolved in cyclohexane as a solvent to prepare a composition (J2) for luminescent-layer formation which had a concentration of 1.0 wt%.

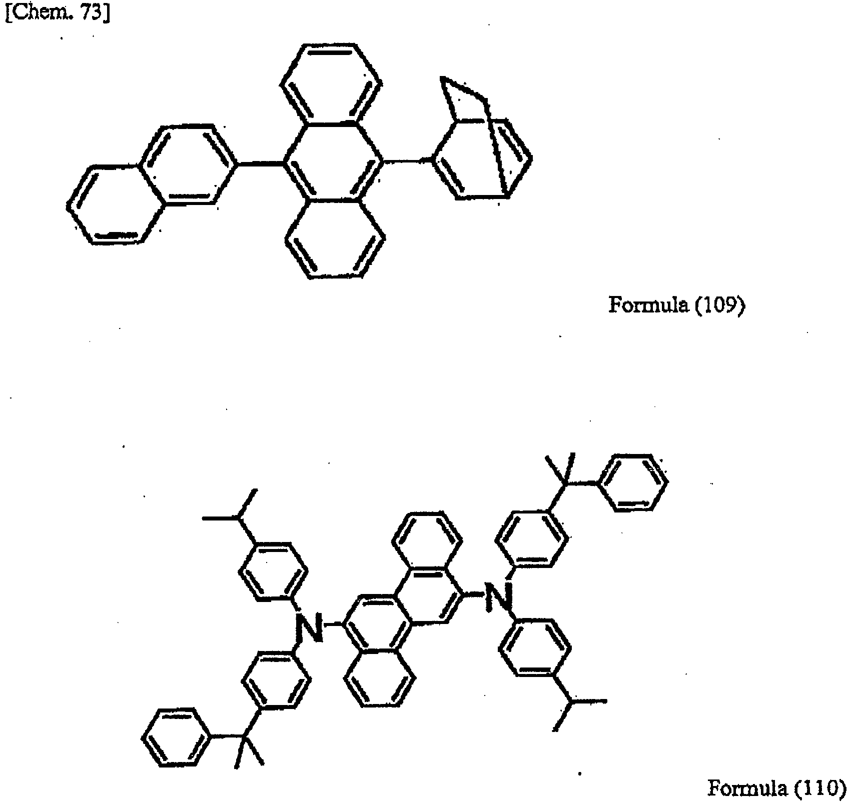

The composition (J2') for luminescent-layer formation was applied on the luminescent layer (E1') by spin coating.

The spin coating was conducted at a rotation speed of 1,500 rpm for a rotation period of 30 seconds. After the spin coating, the unnecessary part on the electrode was wiped off, and the residual coating film was heated at 140°C for 1 hour on a hot plate to thereby dry the coating film and convert the compound represented by formula (109) into the compound represented by the following formula (109-1). Thus, a luminescent layer (J2') having a thickness of 50 nm was formed.

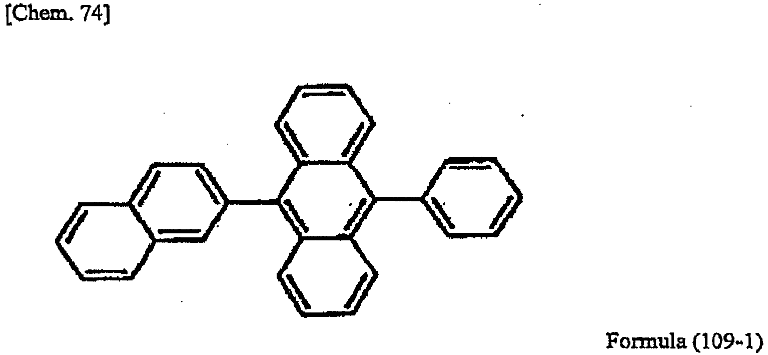

### (Formation of Hole Blocking Layer and Electron Transport Layer)

A hole blocking layer and an electron transport layer were formed in the same manner as in Example 6.

### (Formation of Cathode and Sealing)

A cathode was formed and sealing was conducted, in the same manner as in Example 6.

### (Ascertainment of Luminescence of the Element)

A voltage was applied at a current density of 10 mA/cm² to the element obtained. As a result, luminescence including a green component and a red component was ascertained to occur from the luminescent layer (E1'), and luminescence including a blue component was ascertained to occur from the luminescent layer (J2').

In Table 7 are shown the chromaticity of the luminescence and the luminescence intensity of each of the blue component, green component, and red component of the luminescence spectrum normalized with the peak intensity which was the maximum luminescence intensity.

### (EXAMPLE 12)

An organic electroluminescent element was produced in the same manner as in Example 11, except that the composition (E1) for luminescent-layer formation used in Example 11 was replaced with the composition (F1) for luminescent-layer formation used in Example 8.

### (Ascertainment of Luminescence of the Element)

The element obtained was caused to luminesce in the same manner as in Example 11. The results thereof are shown in Table 7.

### (EXAMPLE 13)

An organic electroluminescent element was produced in the same manner as in Example 11, except that the composition (E1) for luminescent-layer formation used in Example 11 was replaced with the composition (G1) for luminescent-layer formation used in Example 9.

### (Ascertainment of Luminescence of the Element)

The element obtained was caused to luminesce in the same manner as in Example 11. The results thereof are shown in Table 7.

### (EXAMPLE 14)

An organic electroluminescent element was produced in the same manner as in Example 11. except that the composition (E1) for luminescent-layer formation used in Example 11 was replaced with the composition (H1) for luminescent-layer formation used in Example 10.

### (Ascertainment of Luminescence of the Element)

The element obtained was caused to luminesce in the same manner as in Example 11. The results thereof are shown in Table 7.

**[Table 7]**

| | Example 11 | Example 12 | Example 13 | Example 14 |
|---|---|---|---|---|
| CIEx | 0.322 | 0.300 | 0.277 | 0.239 |
| CIEy | 0.307 | 0.339 | 0.320 | 0.388 |
| Blue Component | 1.00 (pk) | 1.00 (pk) | 1.00 (pk) | 0.88 (st) |
| Green Component | 0.63 (sh) | 0.78 (sh) | 0.70 (sh) | 1.00 (pk) |
| Red Component | 0.80 (st) | 0.65 (st) | 0.41 (st) | - |
| Blue Component [nm] | 474 | 475 | 471 | 475 |
| Green Component [nm] | 509 | 509 | 509 | 504 |
| Red Component [nm] | 621 | 619 | 622 | 620 |

| | | | | |
|---|---|---|---|---|
| Symbols in the parentheses in the rows Blue, Green, and Red Components are as follows. pk: maximum-intensity component st: intense component not having maximum intensity sh: shoulder component | | | | |

In each of the elements of Examples 11 to 14, the wavelengths of the respective components were regarded as the following wavelengths, with the exception of clear maximum components.
Blue component: 475 nm
Green component: 509 nm
Red component: 620 nm

It was ascertained from Table 7 that in each element, all of the green luminescent material, red luminescent material (no red component was used in Example 14), and blue luminescent material in the luminescent layer luminesced.

Application of the compound of the invention to a second luminescent layer greatly improved the solubility of this layer, and a poor solvent for the first luminescent layer was hence rendered usable as a coating-fluid solvent for the second luminescent layer. As a result, an organic electroluminescent element which included two kinds of luminescent layers superposed through coating fluid application was obtained.

### (EXAMPLE 15)

### (Production of Organic Electrophosphorescent Element)

### (Formation of Substrate, Hole Injection Layer, and Hole Transport Layer)

A hole injection layer and a hole transport layer were formed on a substrate in the same manner as in Example 6.

### (Formation of Luminescent Layer)

One hundred parts by weight of the compound CzPhDNPhCz obtained in Synthesis Example 7 and 1.0 part by weight of the green luminescent compound represented by the following formula (301) were dissolved in cyclohexylbenzene as a solvent so as to result in a concentration of 3.0 wt%. Thus, a composition for luminescent-layer formation was prepared.

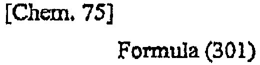

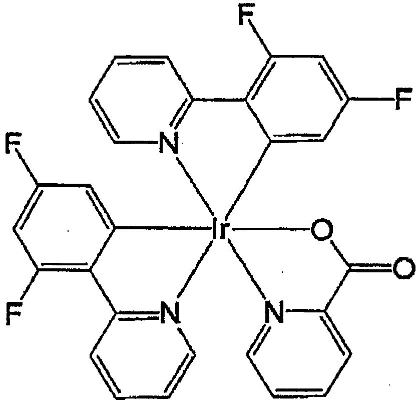

The composition for luminescent-layer formation obtained was applied on the hole transport layer by spin coating. The spin coating was conducted at a rotation speed of 1,500 rpm for a rotation period of 120 seconds. After the spin coating, the unnecessary part on the electrode was wiped off, and the residual coating film was heated and dried at 160°C for 1 hour on a hot plate under vacuum in order to eliminate the elimination group from the cyclohexadiene ring. Thus, a luminescent layer having a thickness of 49 nm was formed.

### (Formation of Hole Blocking Layer and Electron Transport Layer)

A hole blocking layer and an electron transport layer were formed in the same manner as in Example 6.

### (Formation of Cathode and Sealing)

A cathode was formed and sealing was conducted, in the same manner as in Example 6.

### (Ascertainment of Luminescence of the Element)

A voltage was applied to the element obtained, and blue luminescence was ascertained.

### (COMPARATIVE EXAMPLE 2)

An organic electroluminescent element was formed in the same manner as in Example 6, except that the polymer represented by formula (103) constituting the hole transport layer was replaced with a polymer (weight-average molecular weight, 45,000) having the repeating unit represented by the following formula. A voltage was applied thereto in the same manner as in Example 6. As a result, no luminescence was observed.

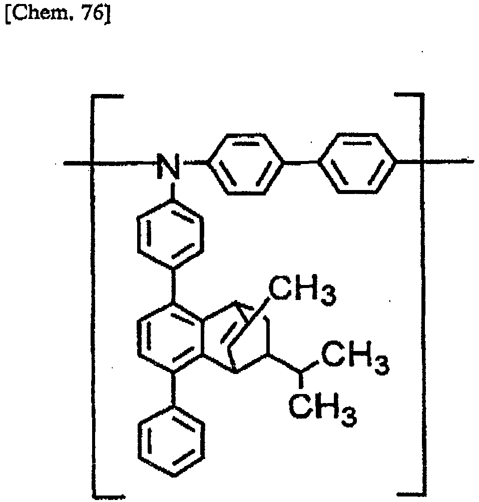

### (COMPARATIVE EXAMPLE 3)

### (Production of Organic Electroluminescent Element)

An organic electroluminescent element was produced in the same manner as in Example 2, except that the formation of a luminescent layer was changed to the following. One hundred parts by weight of the compound represented by the following formula (QQ1) and 10 parts by weight of the compound represented by the following formula (QQ2) were dissolved in toluene to prepare a 0.8-wt% solution. This solution was filtered through a 0.2-µm PTFE filter to prepare a coating fluid (QD1) for luminescent-layer formation. This coating fluid (QD1) for luminescent-layer formation was prepared in a gloved nitrogen box having an oxygen concentration of 1.0 ppm and a moisture concentration of 1.0 ppm .

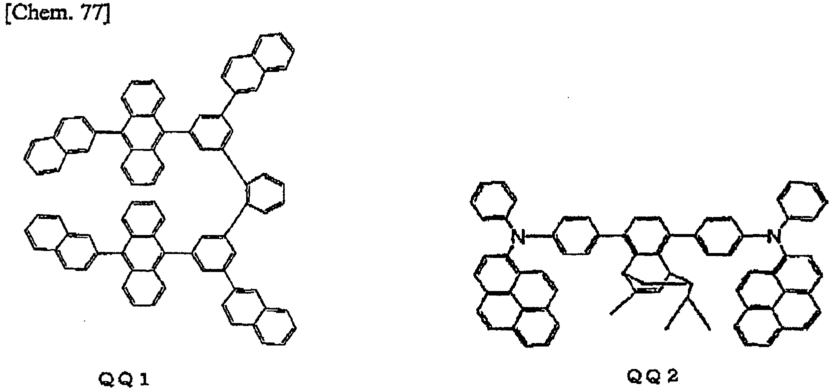

### (Formation of Luminescent Layer)

Using the coating fluid (QD1) for luminescent-layer formation, a luminescent layer was formed on the hole transport layer by spin coating. The spin coating was conducted in a gloved nitrogen box having an oxygen concentration of 1.0 ppm and a moisture concentration of 1.0 ppm, under the conditions of a rotation speed of 1,500 rpm and a rotation period of 30 seconds. After the application, the unnecessary part on the electrode was wiped off, and the residual coating film was heated at 180°C for 1 hour on a hot plate. Thus, a luminescent layer having a thickness of 65 nm was formed.

### (Ascertainment of Luminescence of the Element)

A voltage was applied to the organic electroluminescent element obtained. However, blue luminescence from the blue fluorescent compound (QQ3) obtained by elimination of the elimination group from the QQ2 was unable to be observed.

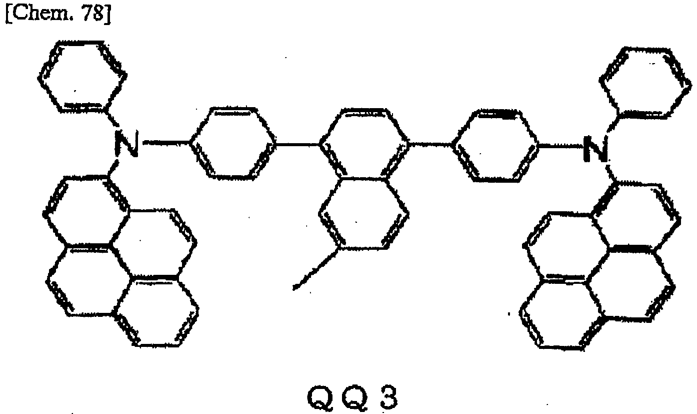

### (Reference Example 100)

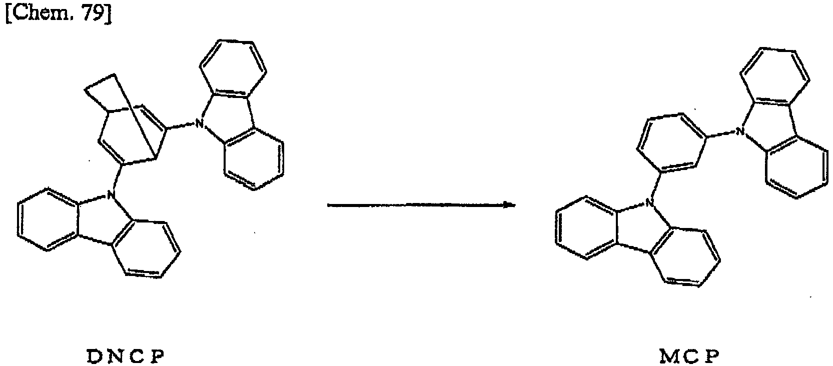

Toluene (70 mg) was added at room temperature to 1,3-bis(N-carbazoyl)benzene (MCP) (8 mg) manufactured by Luminescence Technology Corp., which was formed by elimination of the elimination group from DNCP. This mixture was stirred for 5 minutes and then allowed to stand for 1 hour. It was able to be ascertained that the MCP had completely dissolved in the toluene.

### Description of the Reference Numerals

- 1: Substrate
- 2: Anode
- 3: Hole injection layer
- 4: Hole transport layer
- 5: Luminescent layer
- 6: Hole blocking layer
- 7: Electron transport layer
- 8: Electron injection layer
- 9: Cathode
- 10: Hole extraction layer
- 11: Photoelectric conversion layer
- 12: Electron extraction layer

## Claims

1. A compound which is represented by the following general formula (V) and has a molecular weight of 400 or higher: 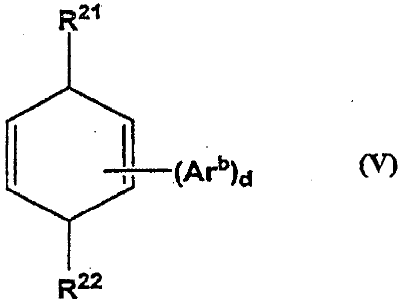 wherein
R²¹ and R²² are bonded to each other to represent a group represented by -CH=CH- or -CH₂-CH₂-;
Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
d represents an integer of 1 to 4;
when a plurality of Ar^{b}s are contained in the molecule, the Ar^{b}s may be the same or different; and
with the proviso that the compound which is represented by the general formula (V) is not

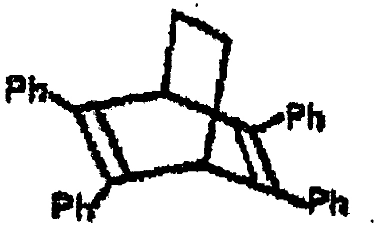

2. The compound according to claim 1, wherein d in the general formula (V) is an integer of 1 to 3.

3. An electronic-device material which comprises a compound which is represented by the following general formula (V) and has a molecular weight of 400 or higher: 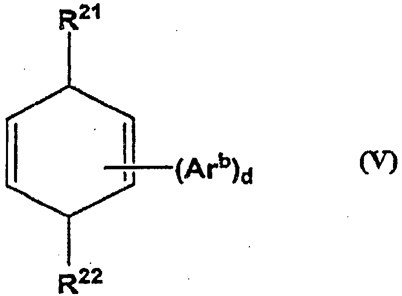 wherein
R²¹ and R²² are bonded to each other to represent a group represented by -CH=CH- or -CH₂-CH₂-;
Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
d represents an integer of 1 to 4 or 1 to 3;
when a plurality of Ar^{b}s are contained in the molecule, the Ar^{b}s may be the same or different.

4. A polymer which comprises a monovalent or divalent group represented by the following general formula (III): 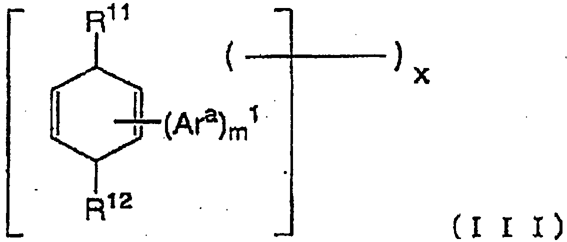 wherein
R¹¹ and R¹² are bonded to each other to represent a group represented by -CH=CH- or -CH₂-CH₂-;
Ar^{a} represents an aromatic hydrocarbon ring group which may have a substituent, wherein the substituent of Ar^{a} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{a} have 1 to 3 carbon atoms;
m¹ represents an integer of 1 to 4;
x represents 1 or 2;
when a plurality of Ar^{a}s are contained in the molecule, the Ar^{a}s may be the same or different.

5. The polymer according to claim 4, which comprises a repeating unit represented by the following formula (IV): 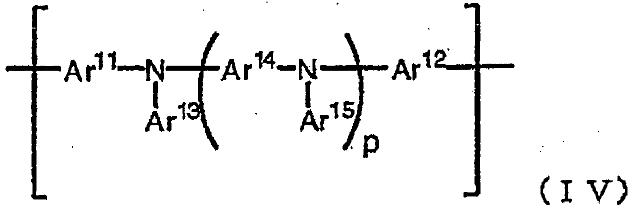 wherein
p represents an integer of 0 to 3,
Ar¹¹ and Ar¹² each independently represent a direct bond or an aromatic ring group which may have a substituent, and
Ar¹³ to Ar¹⁵ each independently represent an aromatic ring group which may have a substituent,
both of Ar¹¹ and Ar¹² being not a direct bond.

6. An electronic-device material which comprises the polymer according to claim 4 or 5.

7. A composition for electronic device which comprises the electronic-device material according to claim 3 or 6 and a solvent.

8. An organic electroluminescent element which comprises an anode, a cathode, and an organic layer interposed between the anode and the cathode, wherein the organic layer comprises a compound which is represented by the following general formula (V) and has a molecular weight of 400 or higher: 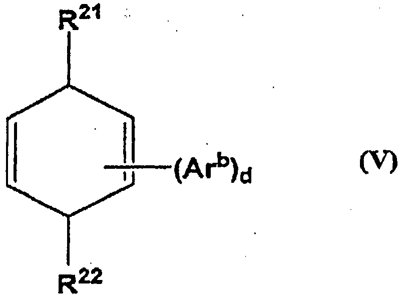 wherein
R²¹ and R²² are bonded to each other to represent a group represented by -CH=CH- or -CH₂-CH₂-;
Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
d represents an integer of 1 to 4 or 1 to 3;
when a plurality of Ar^{b}s are contained in the molecule, the Ar^{b}s may be the same or different; or the polymer according to claim 4 or 5 and wherein the organic layer is a layer formed by a wet film formation method using the composition for electronic devices according to claim 7.

9. The organic electroluminescent element according to claim 8, wherein the organic layer is a luminescent layer.

10. An organic solar cell element which comprises an anode, a cathode, and an organic layer interposed between the anode and the cathode, wherein the organic layer comprises a compound
which is represented by the following general formula (V) and has a molecular weight of 400 or higher: 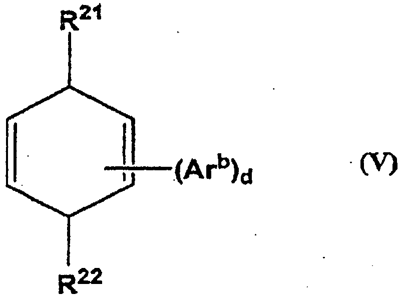 wherein
R²¹ and R²² are bonded to each other to represent a group represented by -CH=CH- or -CH₂-CH₂-;
Ar^{b} represents an aromatic hydrocarbon ring group which may have a substituent, wherein the substituent of Ar^{b} is selected from monovalent aromatic hydrocarbon ring groups derived from 5- or 6-membered monocycles or di- to pentacyclic fused rings; monovalent aromatic heterocyclic groups derived from 5- or 6-membered monocycles or di- to tetracyclic fused rings; and arylamino groups having an aromatic hydrocarbon ring group with 6 to 12 carbon atoms, wherein the hydrocarbon chain portions in the substituent of Ar^{b} have 1 to 3 carbon atoms;
d represents an integer of 1 to 4 or 1 to 3;
when a plurality of Ar^{b}s are contained in the molecule, the Ar^{b}s may be the same or different; or the polymer according to claim 4 or 5 and wherein the organic layer is a layer formed by a wet film formation method, using the composition for electronic devices according to claim 7.

11. A display which comprises the organic electroluminescent element according to claim 8 or 9.

12. A lighting which comprises the organic electroluminescent element according to claim 8 or 9.

## Patentansprüche

1. Eine Verbindung, die durch die folgende allgemeine Formel (V) dargestellt ist und ein Molekulargewicht von 400 oder höher hat: 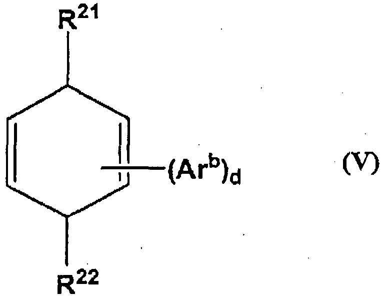 wobei
R²¹ und R²² miteinander verbunden sind, um eine Gruppe, dargestellt durch -CH=CH- oder -CH₂-CH₂-, darzustellen;
Ar^{b} eine aromatische Kohlenwasserstoffringgruppe, die einen Substituenten haben kann, darstellt, wobei der Substituent von Ar^{b} aus einwertigen aromatischen Kohlenwasserstoffringgruppen, abgeleitet von 5- oder 6-gliedrigen Monocyclen oder dibis pentacyclischen kondensierten Ringen; einwertigen aromatischen heterocyclischen Gruppen, abgeleitet von 5- oder 6-gliedrigen Monocyclen oder di- bis tetracyclischen kondensierten Ringen; und Arylaminogruppen, die eine aromatische Kohlenwasserstoffringgruppe mit 6 bis 12 Kohlenstoffatomen haben, ausgewählt ist, wobei die Kohlenwasserstoffkettenabschnitte in dem Substituenten von Ar^{b} 1 bis 3 Kohlenstoffatome haben;
d eine ganze Zahl von 1 bis 4 darstellt;
wenn eine Vielzahl von Ar^{b}s in dem Molekül enthalten ist, können die Ar^{b}s gleich oder verschieden sein; und
mit der Maßgabe, dass die Verbindung, die durch die allgemeine Formel (V) dargestellt ist, nicht

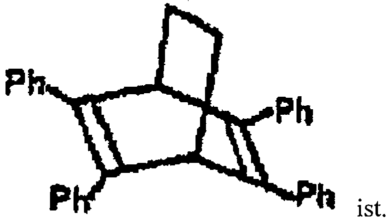

2. Die Verbindung gemäß Anspruch 1, wobei d in der allgemeinen Formel (V) eine ganze Zahl von 1 bis 3 ist.

3. Ein Material für eine elektronische Vorrichtung, das eine Verbindung umfasst, die durch die folgende allgemeine Formel (V) dargestellt ist und ein Molekulargewicht von 400 oder höher hat: 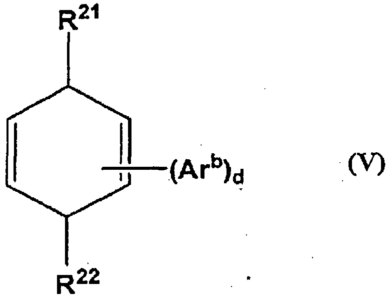 wobei
R²¹ und R²² miteinander verbunden sind, um eine Gruppe, dargestellt durch -CH=CH- oder -CH₂-CH₂-, darzustellen;
Ar^{b} eine aromatische Kohlenwasserstoffringgruppe, die einen Substituenten haben kann, darstellt, wobei der Substituent von Ar^{b} aus einwertigen aromatischen Kohlenwasserstoffringgruppen, abgeleitet von 5- oder 6-gliedrigen Monocyclen oder dibis pentacyclischen kondensierten Ringen; einwertigen aromatischen heterocyclischen Gruppen, abgeleitet von 5- oder 6-gliedrigen Monocyclen oder di- bis tetracyclischen kondensierten Ringen; und Arylaminogruppen, die eine aromatische Kohlenwasserstoffringgruppe mit 6 bis 12 Kohlenstoffatomen haben, ausgewählt ist, wobei die Kohlenwasserstoffkettenabschnitte in dem Substituenten von Ar^{b} 1 bis 3 Kohlenstoffatome haben;
d eine ganze Zahl von 1 bis 4 oder 1 bis 3 darstellt;
wenn eine Vielzahl von Ar^{b}s in dem Molekül enthalten ist, können die Ar^{b}s gleich oder verschieden sein.

4. Ein Polymer, das eine einwertige oder zweiwertige Gruppe umfasst, dargestellt durch die folgende allgemeine Formel (III): 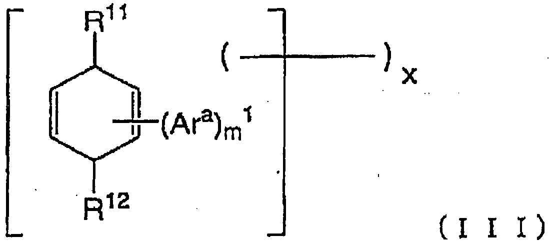 wobei
R¹¹ und R¹² miteinander verbunden sind, um eine Gruppe, dargestellt durch -CH=CH- oder -CH₂-CH₂-, darzustellen;
Ar^{a} eine aromatische Kohlenwasserstoffringgruppe, die einen Substituenten haben kann, darstellt, wobei der Substituent von Ar^{a} aus einwertigen aromatischen Kohlenwasserstoffringgruppen, abgeleitet von 5- oder 6-gliedrigen Monocyclen oder dibis pentacyclischen kondensierten Ringen; einwertigen aromatischen heterocyclischen Gruppen, abgeleitet von 5- oder 6-gliedrigen Monocyclen oder di- bis tetracyclischen kondensierten Ringen; und Arylaminogruppen, die eine aromatische Kohlenwasserstoffringgruppe mit 6 bis 12 Kohlenstoffatomen haben, ausgewählt ist, wobei die Kohlenwasserstoffkettenabschnitte in dem Substituenten von Ar^{a} 1 bis 3 Kohlenstoffatome haben;
m¹ eine ganze Zahl von 1 bis 4 darstellt;
x gleich 1 oder 2 darstellt;
wenn eine Vielzahl von Ar^{a}s in dem Molekül enthalten ist, können die Ar^{a}s gleich oder verschieden sein.

5. Das Polymer gemäß Anspruch 4, das eine wiederholende Einheit umfasst, dargestellt durch die folgenden Formel (IV): 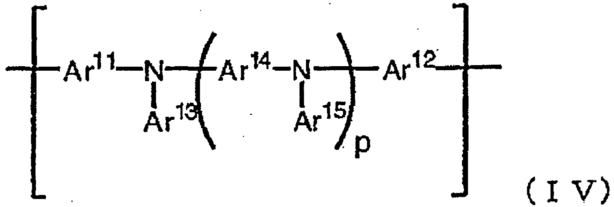 wobei
p eine ganze Zahl von 0 bis 3 darstellt,
Ar¹¹ und Ar¹² jeweils unabhängig eine direkte Bindung oder eine aromatische Ringgruppe, die einen Substituenten haben kann, darstellen und
Ar¹³ bis Ar¹⁵ jeweils unabhängig eine aromatische Ringgruppe, die einen Substituenten haben kann, darstellen,
wobei beide Ar¹¹ und Ar¹² keine direkte Bindung sind.

6. Ein Material für eine elektronische Vorrichtung, das das Polymer gemäß Anspruch 4 oder 5 umfasst.

7. Eine Zusammensetzung für elektronische Vorrichtung, die das Material für eine elektronische Vorrichtung gemäß Anspruch 3 oder 6 und ein Lösungsmittel umfasst.

8. Ein organisches elektrolumineszierendes Element, das eine Anode, eine Kathode und eine organische Schicht, eingeschoben zwischen die Anode und die Kathode, umfasst, wobei die organische Schicht eine Verbindung umfasst, die durch die folgende allgemeine Formel (V) dargestellt ist und ein Molekulargewicht von 400 oder höher hat: 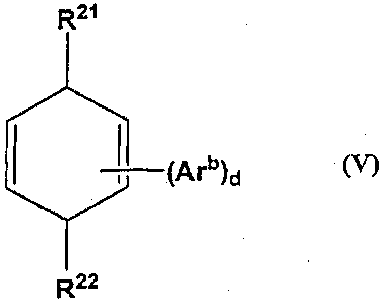 wobei
R²¹ und R²² miteinander verbunden sind, um eine Gruppe, dargestellt durch -CH=CH- oder -CH₂-CH₂-, darzustellen;
Ar^{b} eine aromatische Kohlenwasserstoffringgruppe, die einen Substituenten haben kann, darstellt, wobei der Substituent von Ar^{b} aus einwertigen aromatischen Kohlenwasserstoffringgruppen, abgeleitet von 5- oder 6-gliedrigen Monocyclen oder dibis pentacyclischen kondensierten Ringen; einwertigen aromatischen heterocyclischen Gruppen, abgeleitet von 5- oder 6-gliedrigen Monocyclen oder di- bis tetracyclischen kondensierten Ringen; und Arylaminogruppen, die eine aromatische Kohlenwasserstoffringgruppe mit 6 bis 12 Kohlenstoffatomen haben, ausgewählt ist, wobei die Kohlenwasserstoffkettenabschnitte in dem Substituenten von Ar^{b} 1 bis 3 Kohlenstoffatome haben;
d eine ganze Zahl von 1 bis 4 oder 1 bis 3 darstellt;
wenn eine Vielzahl von Ar^{b}s in dem Molekül enthalten ist, können die Ar^{b}s gleich oder verschieden sein;
oder das Polymer gemäß Anspruch 4 oder 5 und wobei die organische Schicht eine Schicht ist, gebildet durch ein Nassfilmbildungsverfahren, wobei die Zusammensetzung für die elektronische Vorrichtungen gemäß Anspruch 7 verwendet wird.

9. Das organische elektrolumineszierende Element gemäß Anspruch 8, wobei die organische Schicht eine Lumineszenzschicht ist.

10. Ein organisches Solarzellenelement, das eine Anode, eine Kathode und eine organische Schicht, eingeschoben zwischen die Anode und die Kathode, umfasst, wobei die organische Schicht eine Verbindung umfasst, die durch die folgende allgemeine Formel (V) dargestellt ist und ein Molekulargewicht von 400 oder höher hat: 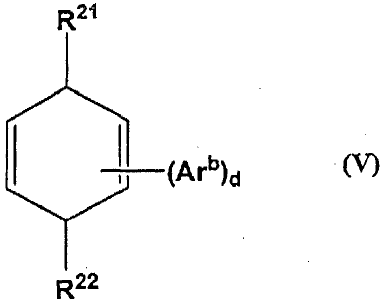 wobei
R²¹ und R²² miteinander verbunden sind, um eine Gruppe, dargestellt durch -CH=CH- oder -CH₂-CH₂-, darzustellen;
Ar^{b} eine aromatische Kohlenwasserstoffringgruppe, die einen Substituenten haben kann, darstellt, wobei der Substituent von Ar^{b} aus einwertigen aromatischen Kohlenwasserstoffringgruppen, abgeleitet von 5- oder 6-gliedrigen Monocyclen oder dibis pentacyclischen kondensierten Ringen; einwertigen aromatischen heterocyclischen Gruppen, abgeleitet von 5- oder 6-gliedrigen Monocyclen oder di- bis tetracyclischen kondensierten Ringen; und Arylaminogruppen, die eine aromatische Kohlenwasserstoffringgruppe mit 6 bis 12 Kohlenstoffatomen haben, ausgewählt ist, wobei die Kohlenwasserstoffkettenabschnitte in dem Substituenten von Ar^{b} 1 bis 3 Kohlenstoffatome haben;
d eine ganze Zahl von 1 bis 4 oder 1 bis 3 darstellt;
wenn eine Vielzahl von Ar^{b}s in dem Molekül enthalten ist, können die Ar^{b}s gleich oder verschieden sein;
oder das Polymer gemäß Anspruch 4 oder 5 und wobei die organische Schicht eine Schicht ist, gebildet durch ein Nassfilmbildungsverfahren, wobei die Zusammensetzung für die elektronische Vorrichtungen gemäß Anspruch 7 verwendet wird.

11. Eine Anzeige, die das organische elektrolumineszierende Element gemäß Anspruch 8 oder 9 umfasst.

12. Eine Beleuchtung, die das organische elektrolumineszierende Element gemäß Anspruch 8 oder 9 umfasst.

## Revendications

1. Composé qui est représenté par la formule générale (V) suivante et qui possède un poids moléculaire supérieur ou égal à 400 : 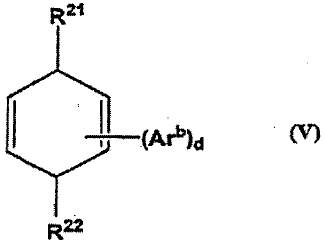 dans laquelle
R²¹ et R²² sont liés l'un à l'autre pour représenter un groupe représenté par -CH=CH- ou -CH₂-CH₂- ;
Ar^{b} représente un groupe cyclique hydrocarboné aromatique qui peut avoir un substituant, dans lequel le substituant de Ar^{b} est sélectionné parmi les groupes cycliques hydrocarbonés aromatiques monovalents dérivés de monocycles ou de cycles condensés di- à penta-cycliques à 5 ou 6 chaînons ; les groupes hétérocycliques aromatiques monovalents dérivés de monocycles ou de cycles condensés di- à tétra-cycliques à 5 ou 6 chaînons ; et les groupes arylamino ayant un groupe cyclique hydrocarboné aromatique ayant 6 à 12 atomes de carbone, dans lequel les parties de chaîne hydrocarbonée dans le substituant de Ar^{b} possèdent 1 à 3 atomes de carbone ;
d représente un nombre entier de 1 à 4 ;
quand une pluralité de Ar^{b} est contenue dans la molécule, les Ar^{b} peuvent être identiques ou différents ; et
à condition que le composé qui est représenté par la formule générale (V) ne soit pas

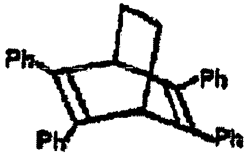

2. Composé selon la revendication 1, dans lequel d dans la formule générale (V) est un nombre entier de 1 à 3.

3. Matériau de dispositif électronique, qui comprend un composé qui est représenté par la formule générale (V) suivante et qui possède un poids moléculaire supérieur ou égal à 400 : 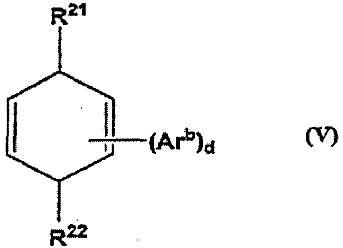 dans laquelle
R²¹ et R²² sont liés l'un à l'autre pour représenter un groupe représenté par -CH=CH- ou -CH₂-CH₂- ;
Ar^{b} représente un groupe cyclique hydrocarboné aromatique qui peut avoir un substituant, dans lequel le substituant de Ar^{b} est sélectionné parmi les groupes cycliques hydrocarbonés aromatiques monovalents dérivés de monocycles ou de cycles condensés di- à penta-cycliques à 5 ou 6 chaînons ; les groupes hétérocycliques aromatiques monovalents dérivés de monocycles ou de cycles condensés di- à tétra-cycliques à 5 ou 6 chaînons ; et les groupes arylamino ayant un groupe cyclique hydrocarboné aromatique ayant 6 à 12 atomes de carbone, dans lequel les parties de chaîne hydrocarbonée dans le substituant de Ar^{b} possèdent 1 à 3 atomes de carbone ;
d représente un nombre entier de 1 à 4 ou de 1 à 3 ; quand une pluralité de Ar^{b} est contenue dans la molécule, les Ar^{b} peuvent être identiques ou différents.

4. Polymère qui comprend un groupe monovalent ou divalent représenté par la formule générale (III) suivante : 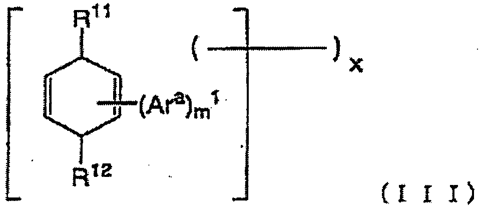 dans laquelle
R¹¹ et R¹² sont liés l' un à l'autre pour représenter un groupe représenté par -CH=CH- ou -CH₂-CH₂- ;
Ar^{a} représente un groupe cyclique hydrocarboné aromatique qui peut avoir un substituant, dans lequel le substituant de Ar^{a} est sélectionné parmi les groupes cycliques hydrocarbonés aromatiques monovalents dérivés de monocycles ou de cycles condensés di- à penta-cycliques à 5 ou 6 chaînons ;
les groupes hétérocycliques aromatiques monovalents dérivés de monocycles ou de cycles condensés di- à tétra-cycliques à 5 ou 6 chaînons ; et les groupes arylamino ayant un groupe cyclique hydrocarboné aromatique ayant 6 à 12 atomes de carbone, dans lequel les parties de chaîne hydrocarbonée dans le substituant de Ar^{a} possèdent 1 à 3 atomes de carbone ;
m¹ représente un nombre entier de 1 à 4 ;
x représente 1 ou 2 ;
quand une pluralité de Ar^{a} est contenue dans la molécule, les Ar^{a} peuvent être identiques ou différents.

5. Polymère selon la revendication 4, qui comprend un motif répétitif représenté par la formule (IV) suivante : 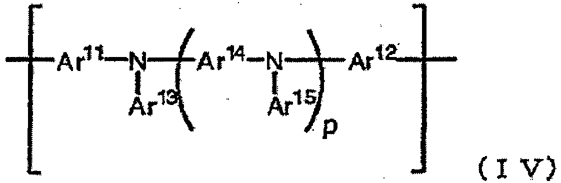 dans laquelle
p représente un nombre entier de 0 à 3,
Ar¹¹ et Ar¹² représentent chacun indépendamment une liaison directe ou un groupe cyclique aromatique qui peut avoir un substituant, et
Ar¹³ à Ar¹⁵ représentent chacun indépendamment un groupe cyclique aromatique qui peut avoir un substituant,
Ar¹¹ et Ar¹² ne pouvant pas être à la fois une liaison directe.

6. Matériau de dispositif électronique, qui comprend le polymère selon la revendication 4 ou 5.

7. Composition pour dispositif électronique, qui comprend le matériau de dispositif électronique selon la revendication 3 ou 6 et un solvant.

8. Élément électroluminescent organique qui comprend une anode, une cathode et une couche organique intercalée entre l'anode et la cathode, dans lequel la couche organique comprend un composé qui est représenté par la formule générale (V) suivante et qui possède un poids moléculaire supérieur ou égal à 400 : 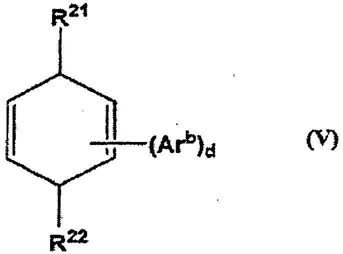 dans laquelle
R²¹ et R²² sont liés l'un à l'autre pour représenter un groupe représenté par -CH=CH- ou -CH₂-CH₂- ;
Ar^{b} représente un groupe cyclique hydrocarboné aromatique qui peut avoir un substituant, dans lequel le substituant de Ar^{b} est sélectionné parmi les groupes cycliques hydrocarbonés aromatiques monovalents dérivés de monocycles ou de cycles condensés di- à penta-cycliques à 5 ou 6 chaînons ; les groupes hétérocycliques aromatiques monovalents dérivés de monocycles ou de cycles condensés di- à tétra-cycliques à 5 ou 6 chaînons ; et les groupes arylamino ayant un groupe cyclique hydrocarboné aromatique ayant 6 à 12 atomes de carbone, dans lequel les parties de chaîne hydrocarbonée dans le substituant de Ar^{b} possèdent 1 à 3 atomes de carbone ;
d représente un nombre entier de 1 à 4 ou de 1 à 3 ; quand une pluralité de Ar^{b} est contenue dans la molécule, les Ar^{b} peuvent être identiques ou différents ; ou le polymère selon la revendication 4 ou 5 et dans lequel la couche organique est une couche formée par une méthode de formation de film humide au moyen de la composition pour dispositifs électroniques selon la revendication 7.

9. Élément électroluminescent organique selon la revendication 8, dans lequel la couche organique est une couche luminescente.

10. Élément de cellules solaires organiques qui comprend une anode, une cathode et une couche organique intercalée entre l'anode et la cathode, dans lequel la couche organique comprend un composé qui est représenté par la formule générale (V) suivante et qui possède un poids moléculaire supérieur ou égal à 400 : dans laquelle 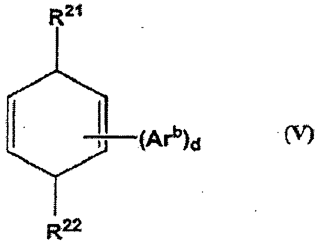
R²¹ et R²² sont liés l'un à l'autre pour représenter un groupe représenté par -CH=CH- ou -CH₂-CH₂- ;
Ar^{b} représente un groupe cyclique hydrocarboné aromatique qui peut avoir un substituant, dans lequel le substituant de Ar^{b} est sélectionné parmi les groupes cycliques hydrocarbonés aromatiques monovalents dérivés de monocycles ou de cycles condensés di- à penta-cycliques à 5 ou 6 chaînons ; les groupes hétérocycliques aromatiques monovalents dérivés de monocycles ou de cycles condensés di- à tétra-cycliques à 5 ou 6 chaînons ; et les groupes arylamino ayant un groupe cyclique hydrocarboné aromatique ayant 6 à 12 atomes de carbone, dans lequel les parties de chaîne hydrocarbonée dans le substituant de Ar^{b} possèdent 1 à 3 atomes de carbone ;
d représente un nombre entier de 1 à 4 ou de 1 à 3 ; quand une pluralité de Ar^{b} est contenue dans la molécule, les Ar^{b} peuvent être identiques ou différents ; ou le polymère selon la revendication 4 ou 5 et dans lequel la couche organique est une couche formée par une méthode de formation de film humide au moyen de la composition pour dispositifs électroniques selon la revendication 7.

11. Affichage qui comprend l'élément électro-luminescent organique selon la revendication 8 ou 9.

12. Éclairage qui comprend l'élément électro-luminescent organique selon la revendication 8 ou 9.
